# EUROPEAN PATENT APPLICATION

(11) **EP 4 847 522 A1**
(43) Date of publication of application: **02.12.2026**
(21) Application number: 25744697.1
(22) Date of filing: 23.01.2025
(51) Int. Cl.: C07K 14/415, C12N 15/29, C12N 15/82, A01H 5/10, A01H 6/46, A61Q 19/08

(54) **MICROPEPTIDE AND USE THEREOF**

(30) Priority: 25.01.2024 CN 202410103198; 25.01.2024 CN 202410102817; 25.01.2024 CN 202410103155; 25.03.2024 CN 202410341982
(71) Applicant: Huazhong Agricultural University, Wuhan, Hubei 430070 (CN); Hubei Hongshan Laboratory, Wuhan, Hubei 430070 (CN); Changzhou Xinmi Biotechnology Co., Ltd, Changzhou, Jiangsu 213000 (CN)
(72) Inventor: YAN, Jianbing, Wuhan, Hubei 430070 (CN); YU, Yanhui, Wuhan, Hubei 430070 (CN); LIU, Yuanfang, Wuhan, Hubei 430070 (CN); LI, Wenqiang, Wuhan, Hubei 430070 (CN); XU, Jieting, Wuhan, Hubei 430070 (CN); ZHUO, Lin, Wuhan, Hubei 430070 (CN); CHEN, Zhenxia, Wuhan, Hubei 430070 (CN); GUO, Min, Wuhan, Hubei 430070 (CN); HU, Xuehai, Wuhan, Hubei 430070 (CN)
(74) Representative: Gille Hrabal Partnerschaftsgesellschaft mbB Patentanwälte
(86) International application number: PCT/CN2025/074456
(87) International publication number: WO 2025/157234

(57) **Abstract**

Provided are a molecular element controlling the dehydration rate and kernel water content of maize kernels, and a use of the molecular element. The molecular element regulates the dehydration rate and kernel water content of maize kernels, and the molecular element can be used to cultivate maize materials having different maize kernel dehydration rates and kernel water contents. The provided micropeptide can also delay the ripening of plant fruits and increase the water content of kernels, and can delay aging, extend life, or reduce active oxygen generation for animal or human subjects.

## Description

The present application claims priority to the following four applications: the prior application with the application No. 202410103198.X and entitled "MICROPEPTIDE AND USE THEREOF" (filed on January 25, 2024), the prior application with the application No. 202410102817.3 and entitled "PROMOTER FOR CONTROLLING DEHYDRATION OF MAIZE KERNELS, ETHYLENE SIGNALING PATHWAY GENE, AND USE THEREOF" (filed on January 25, 2024), the prior application with the application No. 202410103155.1 and entitled "QTL FOR CONTROLLING MAIZE KERNEL DEHYDRATION RATE AND USE THEREOF" (filed on January 25, 2024), and the prior application with the application No. 202410341982.4 and entitled "GENE CONTROLLING MAIZE KERNEL DEHYDRATION RATE AND KERNEL WATER CONTENT AND USE THEREOF" (filed on March 25, 2024), which are incorporated herein by reference in their entirety.

### TECHNICAL FIELD

The present disclosure relates to a molecular element controlling the maize kernel dehydration rate and the kernel water content and use thereof, which pertain to the field of genetic engineering.

### BACKGROUND

Kernel water content is a critical factor that affects the mechanical harvesting quality, safe storage, and economic benefits of maize. The kernel water content at harvest has a great influence on the harvesting, drying, storage, transportation, and processing of maize. Excessive water content often causes economic losses to maize growers and operators, reduces economic benefits, and also easily causes kernels to become mildewed, which affects the quality of maize. In addition, the most critical aspect during the mechanical harvesting of maize kernels is that the maize kernel water content at harvest cannot reach the standard water content of ≤ 25% required for mechanical harvesting (Wang Z, Wang X, Zhang L, Liu X, Di H, Li T, Jin X. QTL underlying field grain drying rate after physiological maturity in maize (Zea Mays L.)[J]. Euphytica, 2012, 185(3): 521-528.). Therefore, it is very important for the selection and breeding of maize varieties with low kernel water content at harvest.

In another aspect, the appropriate harvest water content varies depending on different harvesting purposes. For example, maize kernels intended for silage require a high water content at harvest, which is generally controlled to be not less than 30%; while for fresh maize, to preserve relatively good taste and nutritional value, the kernel water content can be as high as 75%. Therefore, QTLs or functional genes capable of controlling the water content and dehydration rate of maize kernels are of great industrial value.

### SUMMARY

Specific aspects of the present disclosure include:
The present disclosure first provides a micropeptide, wherein the micropeptide is a micropeptide having an amino acid sequence set forth in any one of SEQ ID NO. 4 or SEQ ID NO. 11-SEQ ID NO. 18 or an amino acid sequence having not less than 90% sequence identity to the sequences described above.

The present disclosure further provides a nucleotide sequence encoding the micropeptide.

The present disclosure further provides a vector or construct expressing the micropeptide, comprising an operably linked promoter and a nucleotide sequence encoding the micropeptide, wherein preferably, the nucleotide sequence encoding the micropeptide is SEQ ID NO. 3.

The present disclosure further provides a method for synthesizing the micropeptide described above, wherein the method is a chemical method or a biological method; the chemical method comprises a step of liquid-phase synthesis or solid-phase synthesis, and the biological method comprises the following steps: 1) constructing a vector comprising an operably linked promoter and a nucleotide sequence encoding the micropeptide; 2) transfecting a host cell and screening for positive clones; and 3) culturing the host cell and isolating a micropeptide product.

The present disclosure further provides a formulation comprising the micropeptide described above or a nucleotide encoding the micropeptide at a concentration of 1 µM-50 µM, wherein optionally, the formulation further comprises: water, a bacterial solution, a cell culture medium, a plant culture medium, normal saline, a food excipient, a cosmetic excipient, or a pharmaceutically acceptable excipient.

The present disclosure further provides an anti-aging method, comprising preparing an anti-aging product, or a product associated with the treatment of an aging-related disease, or an antioxidant product by using the micropeptide described above or a nucleotide encoding the micropeptide, wherein preferably, the product comprises a formulation, a probe, a reagent, a kit, a health care product, a drug, or a pharmaceutical combination.

The present disclosure further provides a method for delaying aging, prolonging lifespan, or reducing generation of reactive oxygen species in an animal or human subject, comprising providing the micropeptide described above or a nucleotide encoding the micropeptide to the animal or human subject, preferably, by means of topical, oral, or intravenous injection.

The present disclosure further provides a method for regulating the plant kernel water content or delaying fruit ripening, comprising overexpressing a nucleotide sequence encoding the micropeptide in a plant; or administering to a plant a reagent comprising the micropeptide or a nucleotide encoding the micropeptide, wherein preferably, the reagent further comprises water, a bacterial solution, a cell culture medium, a plant culture medium, or normal saline; preferably, the administration comprises spraying *in vitro* or adding to the soil; preferably, the plant is a plant of the family *Poaceae*; preferably, the plant is rice, *Zea luxurians*, and maize.

The present disclosure first provides a method for cultivating a maize material having the trait of low kernel water content or fast dehydration rate, comprising the following steps:
1) providing a maize material to be improved; and
2) performing genetic modification or hybridization on the material to be improved in step 1), wherein
3) selecting or selecting from the progeny a maize material exhibiting the trait of low kernel water content or fast dehydration rate, wherein
step 2) is selected from at least one of the following steps:
i) reducing the expression level of a microRPG1 micropeptide set forth in SEQ ID NO: 4 in the material to be improved;
ii) increasing the expression levels of the ZmEIL1 gene set forth in SEQ ID NO. 19 and the ZmEIL3 gene set forth in SEQ ID NO. 20 in the material to be improved;
iii) increasing the expression levels of ZmMYBST1 and ZmMYBR43 transcription factors having the amino acid sequences set forth in SEQ ID NO. 35 and SEQ ID NO. 36, respectively, in the material to be improved, wherein preferably, the nucleotide sequences coding sequences of the transcription factors are set forth in SEQ ID NO. 33 and SEQ ID NO. 34, respectively; and
iv) selecting a maize material exhibiting the trait of low kernel water content or fast dehydration rate based on at least one of the following molecular markers and hybridizing the maize material with the material to be improved in step 1), wherein the molecular markers comprise:
   Indel6181: a 6181-bp insertion at positions 722-779 based on the sequence set forth in SEQ ID NO. 50;
   Indel50: a 50-bp deletion at positions 729-778 based on the sequence set forth in SEQ ID NO. 50; Indel234: a 234-bp insertion at positions 1131-1377 based on the sequence set forth in SEQ ID NO. 50; or
   an SNP mutation at position 67, or position 70, or position 473, or position 513, or position 597, or position 599, or position 605 based on the sequence set forth in SEQ ID NO. 50.

In a specific embodiment of the present disclosure, in step i), the coding sequence of microRPG1 is knocked out by means of gene editing, or the expression of the coding sequence of microRPG1 is reduced through RNA interference.

In a specific embodiment of the present disclosure, a nucleotide sequence encoding microRPG1 is SEQ ID NO. 3.

In a specific embodiment of the present disclosure, increasing the expression in step ii) or iii) comprises gene overexpression or strong promoter replacement.

In a specific embodiment of the present disclosure, in step 3), the selection is performed by detecting the molecular marker; preferably, the material with a fast dehydration rate is a haplotype that does not comprise Indel234, Indel50, or Indel6181.

The present disclosure further provides a method for cultivating a maize material having the trait of high kernel water content or slow dehydration rate, comprising the following steps:
1) providing a maize material to be improved; and
2) performing genetic modification or hybridization; and
3) selecting or selecting from the progeny a maize material exhibiting the trait of high kernel water content or slow dehydration rate, wherein
step 2) is selected from at least one of the following steps:
i) increasing the expression level of a microRPG1 micropeptide set forth in SEQ ID NO. 4 in the material to be improved;
ii) reducing the expression levels of the ZmEIL1 gene set forth in SEQ ID NO. 19 and the ZmEIL3 gene set forth in SEQ ID NO. 20 in the material to be improved;
iii) reducing the expression levels of ZmMYBST1 and ZmMYBR43 transcription factors having the amino acid sequences set forth in SEQ ID NO. 35 and SEQ ID NO. 36, respectively in the material to be improved, wherein preferably, the nucleotide sequence coding sequences of the transcription factors are set forth in SEQ ID NO. 33 and SEQ ID NO. 34, respectively;
iv) deleting any fragment between positions 1049-1417 based on SEQ ID NO. 50 in the material to be improved; and
v) selecting a maize material exhibiting the trait of high kernel water content or slow dehydration rate based on at least one of the following molecular markers and hybridizing the maize material with the material to be improved in step 1), wherein the molecular markers comprise:
   Indel6181: a 6181-bp insertion at positions 722-779 based on the sequence set forth in SEQ ID NO. 50;
   Indel50: a 50-bp deletion at positions 729-778 based on the sequence set forth in SEQ ID NO. 50; Indel234: a 234-bp insertion at positions 1131-1377 based on the sequence set forth in SEQ ID NO. 50; or
   an SNP mutation at position 67, or position 70, or position 473, or position 513, or position 597, or position 599, or position 605 based on the sequence set forth in SEQ ID NO. 50.

In a specific embodiment of the present disclosure, increasing the expression in step i) comprises introducing an expression cassette, wherein the expression cassette comprises a promoter, the *ZmRPG* gene set forth in SEQ ID NO. 1 or 2 or a coding sequence of microRPG1 set forth in SEQ ID NO. 3, and a terminator; preferably, the promoter is a high-activity promoter, such as a maize ubiquitin promoter.

In a specific embodiment of the present disclosure, reducing the expression in step ii) or iii) comprises reducing by means of gene editing or RNA interference.

In a specific embodiment of the present disclosure, the deletion in step iv) is performed using a gene editing method.

In a specific embodiment of the present disclosure, in step 3), the selection is performed by detecting the molecular marker; preferably, the material with a slow dehydration rate is a haplotype comprising Indel234 and Indel50, or a haplotype that does not comprise Indel234 and Indel50 but comprises Indel6181.

### BRIEF DESCRIPTION OF THE DRAWINGS

FIG. 1 shows the fine mapping and cloning of *qKDR1*, where A: a graph of the initial mapping results of QTL linkage analysis (curves of different colors represent AUDDC indices of BLUP at different degrees); B: the genotype of an HIF material segment; C: the fine mapping of *qKDR1*, where the phenotype is represented by the AUDDC value between two measurements of moisture; D: NIL material plants and ears.
FIG. 2 shows the distribution of 17 differentially expressed genes in 4 NIL populations (R1-R4). A: genes with downregulated expression; B: genes with upregulated expression. The numbers in the circle represent the number of intersecting genes.
FIG. 3 shows that *qKDR1* regulates the expression of the ZmRPG gene. A: the genotype schematic diagram of 4 NIL populations; B: the expression levels of ZmRPG genes in 4 NIL populations.
FIG. 4 shows the distribution of 12 open reading frames (ORFs) of the ZmRPG gene. Among them, ORF1 encodes the micropeptide microRPG1.
FIG. 5 shows that the overexpression of ZmRPG slows down the maize kernel dehydration rate. A: the structure of the expression cassette overexpressing ZmRPG; B: the expression levels of ZmRPG in two transgenic overexpression lines; C: the kernel dehydration rate of ZmRPG overexpression lines at the Hainan trial site in 2021 (expressed as the AUDDC value between two measurements of moisture); D: the kernel dehydration rate of ZmRPG overexpression lines at the Jilin trial site in 2021 (expressed as the AUDDC value between two measurements of moisture).
FIG. 6 shows that the overexpression of microRPG1 slows down the maize kernel dehydration rate. A: the structure of the expression cassette overexpressing microRPG1; B: the expression levels of microRPG1 in two transgenic overexpression lines; C: the results from the Hainan trial site in 2021; D: the results from the Beijing trial site in 2022; E: the results from the Jilin trial site in 2022. The phenotype is represented by the AUDDC value between two measurements of moisture.
FIG. 7 shows that knocking out microRPG1 accelerates the maize kernel dehydration rate. A: the results from the Beijing trial site in 2020; B: the results from the Hainan trial site in 2021; C: the results from the Beijing trial site in 2022; D: the results from the Jilin trial site in 2022. The data are expressed as the AUDDC value between two measurements of moisture.
FIG. 8 shows that the microRPG1 micropeptide delays the ripening of *Arabidopsis thaliana* siliques. A: the plant phenotype after micropeptide treatment; B: the silique phenotype after micropeptide treatment; C: the seed phenotype after micropeptide treatment; D: the phenotype at the beginning of silique ripening; E: the phenotype at the 50% silique ripening stage; F: the seed water content; G: the performance at anthesis.
FIG. 9 shows the effect of *in vitro* application of the microRPG1 micropeptide at different concentrations on the silique ripening. A: the treated phenotype at the beginning of silique ripening; B: the treated phenotype at the 50% silique ripening stage. n represents the number of plants.
FIG. 10 shows that the microRPG1 micropeptide can be absorbed by the root of *Arabidopsis thaliana*. FAM-ORF1: the image under fluorescence; Bright-field: the image under bright field; Merge: the overlay of images under fluorescence and bright field.
FIG. 11 shows that the microRPG1 micropeptide can improve the human cell viability (left) and reduce the production of reactive oxygen species at the cellular level (right).
FIG. 12 shows the nematode intestinal particulate matter in nematodes after feeding with ORF1 (N2/OP50 + ORF1) and the control (N2/OP50). Day 15/18/21/24 represents the days after feeding.
FIG. 13 shows the statistical analysis results of the survival days of nematodes after feeding with ORF1 (N2 + ORF1) and the control (N2).
FIG. 14 shows that the microRPG1 micropeptide inhibits the expression of EIL genes. A: the expression level of ZmEIL1 increases in the knockout strains and decreases in the overexpression strains, where ORF1-KO represents the microRPG1 knockout, and ORF1-OE represents the microRPG1 overexpression; B: the expression level of ZmEIL3 increases in the knockout strains and decreases in the overexpression strains, where ORF1-KO represents the microRPG1 knockout, and ORF1-OE represents the microRPG1 overexpression.
FIG. 15 shows that the dehydration rate slows down after knockout of ZmEIL1 and ZmEIL3. A: ZmEIL1, where KO1 and KO2 represent different knockout strains; B: ZmEIL3, where KO1, KO2, and KO3 represent different knockout strains. WT: recipient control; n: number of samples. The kernel water content is measured using AUDDC (the water content variation index); for the evaluation method, reference is made to: Yang J, Carena M, and Uphaus J. Area under the dry down curve (AUDDC): a method to evaluate rate of dry down in maize[J]. Crop Sci., 2010, 50(6): 2347-2354.
FIG. 16 shows the kernel water content in the materials treated with ethylene. A: F₁ of SK and KN5585 treated with ethylene; B: F₁ of SK and Zheng 58 treated with ethylene; C: KN5585 treated with ethylene. Pre-tre: before treatment; Post-tre: after treatment; Control: treated with an equal amount of water; Ethylene: treated with ethylene; NS.: no significant difference; the number of *: the degree of significant difference.
FIG. 17 shows the PCR validation of the expression of the ZmRPG gene in kernels, where *: P < 0.05, NS.: the difference is not significant.
FIG. 18 shows the dry weight, fresh weight, and kernel water content of kernels in the near-isogenic lines (NILs). MC: kernel water content; FW: fresh weight; DW: dry weight.
FIG. 19 shows the expression of *ZmRPG*, *ZmEIL1*, and *ZmEIL3* genes in different tissues of maize at different development stages. From top to bottom, the expression of *ZmRPG*, *ZmEIL1*, and *ZmEIL3* genes is shown in sequence. The horizontal axis represents different tissues, and the vertical axis represents the expression level.
FIG. 20 shows the expression patterns of ZmMYBST1, ZmMYBR43, and RPG genes in tissues of maize at different growth stages. The horizontal axis represents different tissue samples, and the vertical axis represents the expression level.
FIG. 21 shows the schematic diagram of the structures of effectors and reporters in the LUC experiment.
FIG. 22 shows the inhibitory effect of ZmMYBST1 or ZmMYBR43 on the LUC reporter genes.
FIG. 23 shows the dehydration rate of maize kernels in ZmMYBST1 and ZmMYBR43 knockout lines. KO1, KO2, and KO3 represent different knockout lines. WT: recipient control; n: number of samples. The kernel water content is measured using AUDDC (the water content variation index); for the evaluation method, reference is made to: Yang J, Carena M, and Uphaus J. Area under the dry down curve (AUDDC): a method to evaluate rate of dry down in maize[J]. Crop Sci., 2010, 50(6): 2347-2354.
FIG. 24 shows the kernel water content of ZmMYBST1 and ZmMYBR43 knockout lines at harvest. KO1, KO2, and KO3 represent different knockout lines. WT: recipient control; n: number of samples.
FIG. 25 shows the fine mapping and cloning of qKDR1. A: a graph of the initial mapping results of QTL linkage analysis (curves of different colors represent AUDDC indices of BLUP at different degrees); B: the position of the qKDR1 segment on the chromosome; C: the fine mapping of qKDR1, where the phenotype is represented by the AUDDC value between two measurements of moisture; D: the insertion of 6181 bp in NIL^{DAN340} relative to NIL^{K22}.
FIG. 26 shows the association analysis of the qKDR1 segment. Arrows indicate Indel6181, Indel50, and Indel234 markers, and the horizontal line represents the threshold (p = 0.01).
FIG. 27 shows the haplotype analysis of the qKDR1 segment. The combination of the three markers, Indel6181, Indel50, and Indel234, is used to screen for genotypes with different dehydration rates, where Hap1-Hap5 represent different haplotypes.
FIG. 28 shows that editing qKDR1 alters the dehydration rate of maize kernels. A: the target positions and sequences for qKDR1 editing in the maize inbred line B104; B: the target positions and sequences for qKDR1 editing in the maize inbred line Zheng 58; C: the phenotypic results from the Beijing trial site; D: the phenotypic results from the Jilin trial site. Z58-KO1 and B104-KO2 represent knockout lines, and Z58-WT1 and B104-WT2 represent wild types. Light-colored fonts represent target sequences.
FIG. 29 shows the activity assay of different qKDR1 fragments. A: the schematic diagram of the position and size of each fragment; B: the schematic diagram of the GUS reporter gene vector expression cassette; C: GUS activity, where different letters indicate significant differences.
FIG. 30 shows the regulatory network of the molecular element of the present disclosure.
FIG. 31 shows a graph of the number of laid eggs of wild-type *Caenorhabditis elegans* (treated or untreated with the micropeptide) at 4 days (day 1, day 2, day 3, and day 4) after adulthood.
FIG. 32 shows a graph of the total number of laid eggs of wild-type *Caenorhabditis elegans* (treated or untreated with the micropeptide) at 4 days after adulthood.
FIG. 33 shows that a 293 cell aging model is constructed using hydrogen peroxide, and the reactive oxygen species (ROS) are assayed by photographing after treatment with the microRPG1 micropeptide.
FIG. 34 shows that a 293 cell aging model is constructed using hydrogen peroxide, and the ROS signal intensity is assayed using a flow cytometer after treatment with the microRPG1 micropeptide and a randomized-sequence small peptide.
FIG. 35 shows that the microRPG1 micropeptide, the randomized-sequence small peptide, and the positive control NAC (acetyl cysteine) are first used to treat cells at a concentration of 50 µm for 24 h, then hydrogen peroxide is used to construct a 293 cell aging model, and finally, the ROS signal intensity is assayed using a flow cytometer.

### DETAILED DESCRIPTION

The present disclosure first identifies, through various genetic and molecular biological methods, a gene ZmRPG controlling the water content and dehydration rate traits of maize kernels, and identifies a micropeptide microRPG1 encoded by the ZmRPG gene and similar micropeptides in *Zea luxurians*. The ZmRPG gene or the microRPG1 micropeptide can be used to cultivate maize varieties with different water contents and dehydration rates as needed. The present disclosure provides the technical solutions of the first aspect as follows:
The present disclosure provides a micropeptide, wherein the micropeptide has an amino acid sequence set forth in any one of SEQ ID NO. 4 or SEQ ID NO. 11-SEQ ID NO. 18.

The present disclosure further provides a formulation. The micropeptide is dissolved in a specific reagent at a concentration of 1 µM-50 µM. In some embodiments, the reagent described above comprises: water, a bacterial solution, a cell culture medium, a plant culture medium, or normal saline. The present disclosure further provides use of the micropeptide or formulation described above, comprising any one of the following:
1) regulating the plant kernel water content; 2) delaying fruit ripening; 3) delaying aging; 4) prolonging lifespan; and 5) reducing generation of reactive oxygen species.

The present disclosure further provides use of a nucleic acid molecule in improving the water content or dehydration rate of maize kernels, wherein the nucleic acid has a nucleotide sequence or reverse complementary sequence set forth in any one of SEQ ID NO. 1 to SEQ ID NO. 3.

The present disclosure further provides a method for reducing the maize kernel water content or increasing the dehydration rate, comprising inhibiting the expression and/or activity of a micropeptide having the sequence set forth in SEQ ID NO. 4 or a nucleic acid molecule having the sequence set forth in any one of SEQ ID NO. 1 to SEQ ID NO. 3 in maize, and selecting a plant with reduced maize kernel water content or increased dehydration rate.

In some embodiments, the method for inhibiting the expression and/or activity of a protein or nucleic acid molecule comprises gene editing or RNA interference.

In some embodiments, the target DNA sequence for the gene editing is set forth in SEQ ID NO. 5.

The present disclosure further provides a kit for reducing the maize kernel water content or increasing the dehydration rate, comprising any one of the following:
(1) an RNA molecule capable of recognizing the target sequence described above, wherein in some embodiments, the sequence of the RNA molecule is set forth in SEQ ID NO. 6;
(2) a DNA molecule encoding the RNA of (1); and
(3) a vector expressing the RNA of (1).

The present disclosure further provides a mutant gene, wherein the mutant gene has a nucleic acid sequence set forth in any one of SEQ ID NO. 7 to SEQ ID NO. 9.

The present disclosure further provides a method for increasing the water content or reducing the dehydration rate of maize kernels, comprising improving, in a maize material to be improved, the expression and/or activity of a micropeptide having the sequence set forth in SEQ ID NO. 4 or a nucleic acid molecule having the sequence set forth in any one of SEQ ID NO. 1 to SEQ ID NO. 3 in maize, and selecting a plant with increased maize kernel water content or reduced dehydration rate.

In some embodiments, the method for increasing the expression comprises using a high-activity promoter to drive expression of a nucleic acid molecule.

In some embodiments, the high-activity promoter is a maize ubiquitin promoter.

In some embodiments, the sequence of the maize ubiquitin promoter is set forth in SEQ ID NO. 10. The present disclosure further provides use of the kit, the mutant gene, or the method described above in improving the water content or dehydration rate trait of maize kernels.

The present disclosure further finds that the expression levels of ZmEIL1 and ZmEIL3 genes in maize are inhibited by the microRPG1 micropeptide, thereby providing the technical solutions of the second aspect as follows:
The present disclosure provides use of a maize gene with an LOC number of Zm00001d047563 or Zm00001d028974 in the B73 reference genome in improving the water content or dehydration rate of maize kernels.

In some embodiments, the nucleotide sequence of the maize gene described above is set forth in SEQ ID NO. 19 or SEQ ID NO. 20.

The present disclosure further provides a method for reducing the maize kernel water content or increasing the dehydration rate, comprising using ethylene to treat maize kernels during the later stage of maize kernel development.

The present disclosure further provides a method for increasing the maize kernel water content or reducing the dehydration rate, comprising inhibiting the gene described above in maize, and selecting a plant with increased maize kernel water content or reduced dehydration rate.

In some embodiments, the target sequence for the gene editing is set forth in any one of the following: TCGCCTGGTTCGCCAGTCCA (SEQ ID NO. 102), CGCAGTGACTACAGCTACGG (SEQ ID NO. 103), GAGCTGCAGGACACCACACT (SEQ ID NO. 104), and
CCCTACAAGAAGCCCCATGA (SEQ ID NO. 105).

In some embodiments, the method for inhibiting the gene expression and/or activity described above comprises gene editing or RNA interference.

The present disclosure further provides a kit for increasing the maize kernel water content or reducing the dehydration rate, comprising a Cas9 protein and any one of the following RNA molecules:
(1) an RNA molecule capable of recognizing the target sequence described above, wherein optionally, the sequence of the RNA molecule is set forth in any one of SEQ ID NOs. 21-24;
(2) a DNA molecule encoding the RNA of (1); and
(3) a vector expressing the RNA of (1).

The present disclosure further provides a mutant gene, wherein the mutant gene has a nucleic acid sequence set forth in any one of SEQ ID NO. 25 to SEQ ID NO. 29.

The present disclosure further provides a promoter, wherein the promoter has a nucleotide sequence set forth in any one of SEQ ID NO. 30 to SEQ ID NO. 32.

The present disclosure further provides an expression cassette comprising the promoter described above.

The present disclosure further provides an expression vector comprising the expression cassette described above.

The present disclosure further provides a host cell comprising the expression vector described above. In some embodiments, the host cell described above is a prokaryotic cell or a non-renewable plant cell.

In some embodiments, the prokaryotic cell described above is an *Escherichia coli* cell or an *Agrobacterium* cell.

The present disclosure further provides use of the method, the kit, the mutant gene, the promoter, the expression cassette, the expression vector, or the host cell described above in improving the water content or dehydration rate trait of maize kernels.

The present disclosure obtains genes *ZmMYBST1* and *ZmMYBR43* controlling the maize kernel dehydration rate and the kernel water content by analyzing and screening for transcription factors binding to the *qKDR1* segment, and further discloses a method for manipulating *ZmMYBST1* and *ZmMYBR43* by genetic engineering means to alter the water content or dehydration rate of maize kernels.

In a third aspect, the present disclosure provides as follows:
The present disclosure provides use of a maize gene with an LOC number of *Zm00001d000184* or *Zm00001d029875* in the B73 reference genome in improving the water content or dehydration rate of maize kernels.

In some embodiments, the nucleotide sequence of the maize gene described above is set forth in any one of SEQ ID NO. 33 to SEQ ID NO. 36.

The present disclosure further provides use of a protein in improving the water content or dehydration rate of maize kernels, wherein the protein has an amino acid sequence set forth in any one of SEQ ID NO. 37 or SEQ ID NO. 38.

The present disclosure further provides a method for increasing the maize kernel water content or reducing the dehydration rate, comprising inhibiting the expression of the gene described above in maize and/or knocking out the gene, and selecting a plant with increased maize kernel water content or reduced dehydration rate.

In some embodiments, the method for inhibiting the gene expression and/or knocking out the gene described above comprises gene editing or RNA interference.

In some embodiments, the target sequence for the gene editing described above is set forth in SEQ ID NO. 39 and SEQ ID NO. 40.

The present disclosure further provides a kit for increasing the maize kernel water content or reducing the dehydration rate, comprising a Cas9 protein and any one of the following RNA molecules:
(1) an RNA molecule capable of recognizing the target sequence described above, wherein in some embodiments, the sequence of the RNA molecule described above is set forth in SEQ ID NO. 41 and SEQ ID NO. 42;
(2) a DNA molecule encoding the RNA of (1); and
(3) a vector expressing the RNA of (1).

The present disclosure further provides a mutant gene, wherein the mutant gene has a nucleic acid sequence set forth in any one of SEQ ID NO. 43 to SEQ ID NO. 48.

In the present disclosure, a major QTL, qKDR1, controlling the water content and dehydration rate traits of maize kernels is mapped through map-based cloning, and 10 molecular markers are identified within the segment. The qKDR1 segment and molecular markers can be used to screen the water content or dehydration rate trait of maize kernels, and qKDR1 can also be manipulated by genetic engineering means to alter the water content or dehydration rate of maize kernels.

In a fourth aspect, the present disclosure provides the following technical solutions:
The present disclosure provides use of a maize genomic fragment in improving the water content or dehydration rate trait of maize kernels, wherein the position of the fragment corresponds to Chromosome 1: 20,007,756 to 20,009,147 in the B73 genome V4 version.

In some embodiments, the fragment described above has a nucleotide sequence or reverse complementary sequence set forth in any one of SEQ ID NO. 49 or SEQ ID NO. 50.

The present disclosure further provides a molecular marker, wherein the marker corresponds to position 67, or position 70, or position 473, or position 513, or position 597, or position 599, or position 605, or positions 722-779, or positions 729-778, or positions 1131-1377 of the sequence set forth in SEQ ID NO. 50.

The present disclosure further provides a method for identifying or assisting in the identification of the water content or dehydration rate trait of maize kernels, comprising the following steps: (1) detecting the molecular marker described above in a material to be tested; and (2) if the detection result shows that the marker described above is contained, the material to be tested will exhibit the trait of low kernel water content or fast dehydration rate; and if the detection result shows that the marker described above is not contained, the material to be tested will exhibit the trait of high kernel water content or slow dehydration rate.

The present disclosure further provides a method for cultivating a maize material having the trait of low kernel water content or fast dehydration rate, comprising introducing a nucleic acid molecule having the sequence set forth in SEQ ID NO. 50 into a maize material to be improved, detecting the molecular marker described above in the material to be tested according to the method described above, and screening for a material comprising the molecular marker described above.

The present disclosure further provides a method for increasing the maize kernel water content or reducing the dehydration rate, comprising deleting genomic fragments corresponding to the bases between positions 501-1272 or between positions 1049-1417 of SEQ ID NO. 50 in a maize material to be improved, and selecting a plant with increased maize kernel water content or reduced dehydration rate.

In some embodiments, the method for deleting the fragments described above uses a gene editing method.

In some embodiments, the target DNA sequence for the gene editing described above is set forth in SEQ ID NO. 51 and SEQ ID NO. 52.

The present disclosure further provides a kit for increasing the maize kernel water content or reducing the dehydration rate, comprising any one of the following:
(1) an RNA molecule capable of recognizing the target sequence described above, wherein in some embodiments, the sequence of the RNA molecule described above is set forth in SEQ ID NO. 53 and SEQ ID NO. 54;
(2) a DNA molecule encoding the RNA of (1); and
(3) a vector expressing the RNA of (1).

The present disclosure further provides a mutant gene, wherein the mutant gene has a nucleic acid sequence set forth in SEQ ID NO. 55 or SEQ ID NO. 56.

The present disclosure further provides use of the molecular marker, the method, the kit, or the mutant gene described above in improving the water content or dehydration rate trait of maize kernels.

### Definitions of Terms

The following definitions and methods are provided to better define the present application and to guide those of ordinary skill in the art in the practice of the present application. Unless otherwise stated, terms are to be understood according to conventional usage by those of ordinary skill in the relevant art. All patent documents, academic papers, industry standards, other publications, and the like cited herein are incorporated by reference in their entirety.

As used herein, "maize" refers to any maize plant and includes all plant varieties that can be bred with maize, including whole plants, plant cells, plant organs, plant protoplasts, plant cell tissue cultures from which plants can regenerate, plant calli, and intact plant cells in plants or plant parts, wherein the plant parts are, for example, embryos, pollen, ovules, seeds, leaves, flowers, branches, fruits, stems, roots, root tips, anthers, and the like. Unless otherwise indicated, the nucleic acid is written from left to right in the orientation of 5' to 3', and the amino acid sequence is written from left to right in the orientation of amino to carboxyl. The amino acid herein may be represented by its commonly known three-letter symbol or by the one-letter symbol recommended by the IUPAC-IUB Biochemical Nomenclature Committee. Likewise, the nucleotide may be represented by the generally accepted one-letter code. The numeric range is inclusive of the numbers defining the range. As used herein, "nucleic acid" includes a deoxyribonucleotide or ribonucleotide polymer in single- or doublestranded forms, and unless otherwise limited, includes known analogs (e.g., peptide nucleic acids) that have basic properties of the natural nucleotide and hybridize with single-stranded nucleic acids in a manner similar to naturally occurring nucleotides. As used herein, the term "encoding" or "encoded" as used in the context of a specific nucleic acid means that the nucleic acid includes essential information to guide the translation of the nucleotide sequence into a specific protein. A codon is used to indicate information encoding a protein. As used herein, the "full-length sequence" involving a specific polynucleotide or a protein encoded thereby refers to the entire nucleic acid sequence or the entire amino acid sequence having a native (non-synthetic) endogenous sequence. The full-length polynucleotide encodes the full-length and catalytically active forms of the specific protein. The terms "peptide", "polypeptide", and "protein" herein are used interchangeably to refer to a polymer of amino acid residues. The term is used for an amino acid polymer in which one or more amino acid residues are artificial chemical analogs of corresponding naturally occurring amino acids. The term is also used for a naturally occurring amino acid polymer. The terms "residue", "amino acid residue", and "amino acid" herein are used interchangeably to refer to an amino acid incorporated into a protein, polypeptide, or peptide (collectively referred to as "protein"). The amino acid may be a naturally occurring amino acid, and unless otherwise limited, may include known analogs of natural amino acids that may function in a manner similar to naturally occurring amino acids.

The term "trait" refers to a physiological, morphological, biochemical, or physical characteristic of a plant or specific plant material or cell. In some cases, this characteristic is visible to the human eye, such as seeds or plant size, or can be measured by biochemical techniques, such as detecting the protein, starch, or oil content of seeds or leaves, or by observing metabolic or physiological processes, e.g., by measuring tolerance to water deprivation or specific salt or sugar or nitrogen concentrations, or by observing the expression level of one or more genes, or by agronomic observations such as tolerance to osmotic stress or yield.

"Plant" includes the indexing of whole plants, plant organs, plant tissues, seeds, and plant cells, as well as their progeny. The plant cells include, but are not limited to, cells from seeds, suspension cultures, embryos, meristematic regions, calli, leaves, roots, seedlings, gametophytes, sporophytes, pollen, and microspores. "Progeny" includes any subsequent generation of a plant.

In the present application, the word "comprise", "include", or variations thereof should be construed as including other elements, numbers, or steps in addition to the elements, numbers, or steps described. "Test plant" or "test plant cell" refers to a plant or plant cell in which genetic modification has taken effect, or a progeny cell of the plant or cell modified in this way, wherein the progeny cell comprises the modification. "Control" or "control plant" or "control plant cell" provides a reference point for measuring changes in the phenotype of the test plant or plant cell.

A negative or control plant may include, for example: (a) a wild-type plant or cell, i.e., a plant or cell with the same genotype as a genetically modified starting material, wherein the genetic modification produces a test plant or cell; (b) a plant or plant cell with the same genotype as the starting material but transformed with an empty construct (i.e., a construct that has no known effect on the target trait, such as a construct comprising a marker gene); (c) a plant or plant cell that is a non-transformed segregant of the test plant or plant cell; (d) a plant or plant cell that is genetically identical to the subject plant or plant cell but not exposed to conditions or stimuli that would induce the expression of the target gene; or (e) the test plant or plant cell itself, which is under a condition in which the target gene is not expressed.

Those skilled in the art will readily agree that advances in the field of molecular biology, such as site-specific mutagenesis and random mutagenesis, polymerase chain reaction methods, and protein engineering techniques, provide a wide range of appropriate tools and procedures for modifying or engineering the amino acid sequences and underlying gene sequences of proteins of agricultural interest.

In some embodiments, the nucleotide sequence of the present application may be altered for the conservative amino acid replacement. The principles and examples of the conservative amino acid replacement are further described below. In certain embodiments, the nucleotide sequence of the present application may be replaced without altering the amino acid sequence according to the disclosed monocotyledonous codon preference. For example, codons encoding the same amino acid sequence may be replaced with monocotyledonous plant-preferred codons without altering the amino acid sequence encoded by the nucleotide sequence. In some embodiments, a portion of the nucleotide sequence in the present application is replaced with different codons encoding the same amino acid sequence, thereby altering the nucleotide sequence without altering the amino acid sequence encoded thereby. Conservative variants include those sequences that, because of genetic codon degeneracy, encode the amino acid sequence of one of the proteins of the embodiments. In some embodiments, a portion of the nucleotide sequence in the present application is replaced based on monocotyledonous plant-preferred codons. Those skilled in the art may recognize that amino acid additions and/or substitutions are generally based on the relative similarity of amino acid side chain substituents, e.g., the hydrophobicity, charge, size, or the like of the substituents. Exemplary amino acid substituents with various of the aforementioned properties considered are well known to those skilled in the art, and include arginine and lysine; glutamic acid and aspartic acid; serine and threonine; glutamine and asparagine; and valine, leucine, and isoleucine. Guidelines for appropriate amino acid substitutions that do not affect the biological activity of the target protein can be found in the model of Dayhoff et al. (1978), Atlas of Protein Sequence and Structure (Natl. Biomed. Res. Found., Washington, D. C) (incorporated herein by reference). Conservative substitutions, such as exchanging one amino acid for another amino acid having similar properties, can be made. The identification of sequence identity includes hybridization techniques. For example, all or part of a known nucleotide sequence is used as a probe for selective hybridization with other corresponding nucleotide sequences, wherein the other corresponding nucleotide sequences are present in a population of cloned genomic DNA fragments or cDNA fragments (i.e., genomic libraries or cDNA libraries) from a selected organism. The hybridization probe may be a genomic DNA fragment, a cDNA fragment, an RNA fragment, or other oligonucleotides, and may be labeled with a detectable group such as 32P or other detectable markers. Therefore, for example, the hybridization probe can be prepared by labeling synthetic oligonucleotides based on the sequences of the embodiments. The methods for preparing hybridization probes and for constructing cDNA and genomic libraries are generally known in the art. The hybridization of the sequences can be performed under a stringent condition. As used herein, the term "stringent condition" or "stringent hybridization condition" refers to a condition under which a probe will hybridize with its target sequence to a greater detectable extent (e.g., at least 2-fold, 5-fold, or 10-fold over background) relative to hybridization with other sequences. The stringent condition is sequence-dependent and varies across different environments. By controlling the stringency of hybridization and/or controlling a wash condition, a target sequence that is 100% complementary to the probe can be identified (homologous probe method). Optionally, the stringent condition can be adjusted to allow some sequence mismatches, thereby facilitating the detection of lower similarity (heterologous probe method). Generally, a probe is less than about 1000 or 500 nucleotides in length. Generally, the stringent condition is as follows, i.e., under the condition, the salt concentration is less than about 1.5 M Na ions, generally about 0.01 M to 1.0 M Na ions (or other salts) at pH 7.0 to 8.3, and the temperature condition is: at least about 30 °C when used for a short probe (e.g., 10 to 50 nucleotides); and at least about 60 °C when used for a long probe (e.g., greater than 50 nucleotides). The stringent condition may also be achieved by the addition of a destabilizer such as formamide. Exemplary low-stringent conditions include hybridization using 30% to 35% formamide buffer, 1 M NaCl, and 1% SDS (sodium dodecyl sulfate) at 37 °C, and a wash in 1× to 2× SSC (20× SSC = 3.0 M NaCl/0.3 M trisodium citrate) at 50 °C to 55 °C. Exemplary moderate-stringent conditions include hybridization in 40% to 45% formamide, 1.0 M NaCl, and 1% SDS at 37 °C, and a wash in 0.5× to 1× SSC at 55 °C to 60 °C. Exemplary high-stringent conditions include hybridization in 50% formamide, 1 M NaCl, and 1% SDS at 37 °C, and a final wash in 0.1× SSC at 60 °C to 65 °C for at least about 20 min. Optionally, the wash buffer may comprise about 0.1% to about 1% SDS. The duration of hybridization is generally less than about 24 h, generally about 4 h to about 12 h. The specificity generally depends on the wash after hybridization, and the critical factors are the ionic strength and temperature of the final wash solution. Tm (thermodynamic melting point) of a DNA-DNA hybrid can be approximated from the formula of Meinkoth and Wahl (1984), Anal. Biochem., 138: 267-284: Tm = 81.5 °C + 16.6 (logM) + 0.41 (% GC) - 0.61 (% formamide) - 500/L, where M is the gram molar concentration of monovalent cations, % GC is the percentage of guanosine and cytosine nucleotides in DNA, "formamide %" is the percentage of formamide in a hybridization solution, and L is the base pair length of the hybrid. Tm is the temperature (under the determined ionic strength and pH) at which 50% of a complementary target sequence hybridizes with an exactly matched probe. Generally, the wash is performed at least until equilibrium is reached and a low level of hybridization background is achieved, such as for 2 h, 1 h, or 30 min. Each 1% of mismatch corresponds to a decrease in Tm by about 1 °C; thus, the Tm, hybridization, and/or wash conditions can be adjusted to achieve hybridization with a sequence with desired identity. For example, if a sequence with ≥ 90% identity is desired, the Tm can be reduced by 10 °C. Generally, the stringent condition is selected to be about 5 °C lower than the Tm for the specific sequence and its complementary sequence under the determined ionic strength and pH. However, under extremely stringent conditions, the hybridization and/or wash can be performed at 4 °C lower than the Tm; under moderate-stringent conditions, the hybridization and/or wash can be performed at 6 °C lower than the Tm; under low-stringent conditions, the hybridization and/or wash can be performed at 11 °C lower than the Tm.

In some embodiments, fragments of a nucleotide sequence and an amino acid sequence encoded thereby are also included. As used herein, the term "fragment" refers to a portion of a nucleotide sequence of a polynucleotide or a portion of an amino acid sequence of a polypeptide of the embodiments. The fragment of the nucleotide sequence may encode a protein fragment, wherein the protein fragment retains the biological activity of a native or corresponding full-length protein, and thus has protein activity. A mutant protein includes a bioactive fragment of a native protein comprising contiguous amino acid residues that retain the biological activity of the native protein.

Some embodiments also include a transformed plant cell or a transgenic plant comprising at least one of the nucleotide sequences of the embodiments. In some embodiments, an expression vector is used to transform a plant, wherein the expression vector comprises at least one of the nucleotide sequences of the embodiments and a promoter that is operably linked thereto and drives expression in a plant cell. The transformed plant cell and the transgenic plant refer to a plant cell or a plant comprising a heterologous polynucleotide within its genome. Generally, the heterologous polynucleotide is stably integrated within the genome of the transformed plant cell or transgenic plant so as to transmit the polynucleotide to the progeny. The heterologous polynucleotide can be integrated into the genome alone or as part of the expression vector. In some embodiments, the plants involved in the present application include plant cells, plant protoplasts, plant cell tissue cultures from which plants can regenerate, plant calli, plant clumps, and plant cells that are intact plants or parts of plants such as embryos, pollen, ovules, seeds, leaves, flowers, branches, fruits, kernels, ears, cobs, husks, stalks, roots, root tips, anthers, and the like. The present application also includes plant cells, a protoplast, tissues, calli, embryos, flowers, stems, fruits, leaves, and roots derived from the transgenic plant of the present application or progeny thereof, and thus at least partially comprising the nucleotide sequence of the present application.

The term "amplification" in the case of nucleic acid amplification is any process in which additional copies of a selected nucleic acid (or its transcribed form) are produced. Typical amplification methods include replication methods based on multiple polymerases, including the polymerase chain reaction (PCR), ligase-mediated methods such as ligase chain reaction (LCR), and the RNA polymerase-based amplification (e.g., through transcription) method.

When an allele is linked to a trait, and when the presence of the allele is an indication that the desired trait or trait form may occur in a plant comprising the allele, the allele is "associated with" the trait.

The term "quantitative trait locus" or "QTL" used herein refers to a polymorphic locus having at least one allele associated with differential expression of a phenotypic trait in at least one genetic background (e.g., in at least one breeding population or progeny). QTLs can function through single-gene or multi-gene mechanisms.

The term "QTL mapping" used herein refers to the mapping of QTLs on a genetic map using a method similar to single-gene mapping, determining the distance between the QTL and a genetic marker (expressed as a recombination rate). According to the number of markers, methods can be classified into single-marker, double-marker, and multi-marker methods. According to different statistical analysis methods, they can be classified into a variance and mean analysis method, a regression and correlation analysis method, a moment estimation method, a maximum likelihood method, and the like. According to the number of labeled intervals, they can be classified into zero-interval mapping, single-interval mapping, and multi-interval mapping. In addition, there are comprehensive analysis methods that combine different methods, such as QTL composite interval mapping (CIM), multi-interval mapping (MIM), multi-QTL mapping, multi-trait mapping (MTM), and the like.

The term "molecular marker" used herein refers to a specific DNA fragment capable of reflecting certain differences in genomes among individual organisms or populations.

The term "major gene" used herein refers to a single gene that individually determines a certain trait. The term "minor gene" herein refers to each of several non-allelic genes that exerts only a partial effect on the phenotype of the same trait, and the several genes are collectively referred to as additive genes or polygenes. Among the additive genes, each gene exhibits only a relatively small part of the phenotypic effects and thus is also referred to as a minor gene.

The term "inbred line" used herein refers to a line with relatively uniform agronomic traits and a relatively simple genetic basis that is obtained by continuously eliminating undesirable ear rows and selecting individual plants with relatively good agronomic traits for selfing over several generations in the case of artificially controlled self-pollination.

The term "backcross" used herein refers to a method in which a first filial generation is crossed with either of two parents.

The term "hybridization" or "hybrid" used herein refers to the fusion of gametes (e.g., cells, seeds, or plants) resulting in progeny via pollination. The term includes sexual hybridization (the pollination of one plant by another plant) and selfing (self-pollination, e.g., when the pollen and ovules are from the same plant). The term "hybridization" refers to the fusion of gametes resulting in progeny via pollination.

The term "backcross" used herein refers to a process in which a hybrid progeny is repeatedly backcrossed with one of the parents. In a backcrossing scheme, a "donor" parent refers to a parent plant that possesses the desired gene or locus to be introduced. A "recipient" parent (used one or more times) or "recurrent" parent (used two or more times) refers to a parent plant into which a gene or locus is introduced. The initial hybridization produces the F₁ generation; then, the term "BC₁" refers to the second use of the recurrent parent, "BC₂" refers to the third use of the recurrent parent, and so on.

The term "genotype" is the genetic composition of an individual (or individual group) at one or more loci, as contrasted with an observable trait (phenotype). The genotype is defined by the alleles of one or more known loci, and an individual has inherited the loci from its parents. The term "genotype" may be used to refer to the genetic composition of an individual at a single locus, or the genetic composition of an individual at multiple loci, or more generally, the term "genotype" may be used to refer to the genetic composition of all the genes in an individual's genome.

"Germplasm" refers to a genetic material of or obtained from an individual (e.g., a plant), a group of individuals (e.g., a plant line, variety, or family), or a clone derived from a line, variety, species, or culture. The germplasm may be part of an organism or cell, or may be isolated from an organism or cell. The germplasm generally provides the genetic material and the specific molecular composition that provides the physical basis for some or all of the genetic traits of an organism or cell culture. As used herein, the germplasm includes a cell, seed, or tissue from which a new plant may be grown, or a plant part such as leaves, stems, pollen, or cells, which can be cultured into an entire plant.

"Recombinant expression vector" comprises the polynucleotide encoding the micropeptide described above. The polynucleotide is operably linked to a control sequence capable of directing the expression of the micropeptide in a suitable host. The control sequence may be derived from the host organism or from another organism. The expression vector may also comprise a marker gene for the selection of a transformant, or the selection marker may be introduced into the host by co-transformation in another vector construct.

"Construct" refers to a host cell comprising the gene encoding the micropeptide or the recombinant expression vector, and the host cell is a bacterium, a fungus, a plant cell, or an animal cell.

As used herein, the term "identity" is used to refer to the matching of sequences between two polypeptides or between two nucleic acids. When a position in two compared sequences is occupied by the same base or amino acid monomer subunit (e.g., a position in each of two DNA molecules is occupied by adenine, or a position in each of two polypeptides is occupied by lysine), the molecules are identical at the position. The "percent identity" between two sequences is a function of the number of matching positions shared by the two sequences divided by the number of positions for comparison × 100. For example, if 6 out of 10 positions in two sequences match, the two sequences have 60% identity. For example, DNA sequences, CTGACT and CAGGTT, share 50% identity (3 out of a total of 6 positions match). Generally, the comparison is made when the two sequences are aligned to produce the maximum identity. Such alignment can be conveniently performed by using, for example, a computer program such as the Align program (DNAstar, Inc.). The percent identity between two amino acid sequences can also be determined using the E. Meyers and W. Miller algorithm (Comput. ApplBiosci., 4: 11-17 (1988)) that has been integrated into the ALIGN program (version 2.0), with a PAM120 weight residue table, a gap length penalty of 12, and a gap penalty of 4. In addition, the percent identity between two amino acid sequences can be determined using the Needleman and Wunsch algorithm that has been integrated into the GAP program of the GCG software package (available at www.gcg.com), with a Blosum 62 matrix or a PAM250 matrix, a gap weight of 16, 14, 12, 10, 8, 6, or 4, and a length weight of 1, 2, 3, 4, 5, or 6.

The micropeptide provided in the present disclosure comprises a micropeptide having not less than 90%, or not less than 95%, or not less than 99% sequence identity to any one of SEQ ID NO. 4 or SEQ ID NO. 11 to SEQ ID NO. 18.

In certain aspects of the present disclosure, the micropeptide comprises variants with conservative amino acid replacement. The term "conservative replacement" refers to amino acid replacement that does not adversely affect or alter the expected properties of a protein/polypeptide comprising an amino acid sequence. For example, the conservative replacement can be introduced by standard techniques known in the art, e.g., site-directed mutagenesis and PCR-mediated mutagenesis. The conservative amino acid replacement includes a replacement in which an amino acid residue is replaced with an amino acid residue having a similar side chain, e.g., replacement with a residue that is physically or functionally similar to the corresponding amino acid residue (e.g., possessing similar size, shape, charge, and chemical properties, including the ability to form a covalent bond or a hydrogen bond, or the like). Families of amino acid residues having similar side chains have been defined in the art. These families include amino acids having basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine). Therefore, it is preferred to replace the corresponding amino acid residue with another amino acid residue from the same side chain family. Methods for identifying conservative amino acid replacement are well known in the art.

As used herein, an "individual" or "subject" in need refers to a human or non-human animal. Without being limited by any theory, the microRPG1 micropeptide provided in the present disclosure, after being administered to an individual via topical, oral, injection, or other routes, can significantly slow the aging process of the individual, improve the appearance changes associated with aging, improve the physical function of the aged individual, prolong the lifespan of the individual, and prevent or treat aging-related diseases.

Herein, the aging-related diseases include aging-related cardiovascular diseases, neurodegenerative diseases, skeletal diseases, and chronic metabolic diseases; the aging-related cardiovascular diseases include hypertension and atherosclerosis; the neurodegenerative diseases include Parkinson's disease and Alzheimer's disease; the skeletal diseases include osteoporosis, osteoarthritis, and skeletal deformities; the chronic metabolic diseases include type II diabetes, non-alcoholic fatty liver disease, and hepatic fibrosis. The aging-related diseases also include symptoms of aging, such as memory decline, cognitive decline, behavioral degeneration, age-dependent weight gain, mitochondrial function decline, hair loss, subcutaneous fat loss, skin wrinkles, and the like.

Herein, the anti-aging treatment includes (but is not limited to) a treatment that results in the prevention, amelioration, or alleviation of the effects of aging, reduction in or delay of biological age gain, and delay of aging; treatment, prevention, amelioration, or alleviation of frailty or the effects of aging-related diseases and conditions or decline, slowing the progression of such decline, conditions, or diseases, prolongation of healthspan or lifespan, rejuvenation, enhancement of stress resistance or resilience, increase in recovery rates after surgery, radiotherapy, disease, and/or any other stress, or other enhancements; prevention and/or treatment of climacteric syndrome, restoration of reproductive function, elimination or reduction of the spread of senescent cells, reduction in all or multiple mortality risks, or all-cause or multi-cause mortality risk associated with at least one or at least two age-related diseases or conditions, or delay of the increase in such risks; and reduction in the risk of disease onset. A treatment that adjusts at least one biomarker of aging to a younger state or slows its transition to an "older" state is also considered an anti-aging treatment, including but not limited to, a biomarker of aging that shows signs of aging, such as wrinkles, gray hair, and the like.

Herein, the non-human animal includes companion animals, e.g., dogs or cats; in some other embodiments, the non-human animal is a test animal, such as nematodes, fruit flies, mice, or rats.

Herein, anti-aging includes delaying aging, prolonging longevity, and improving the appearance of aging-related changes; maintaining homeostasis includes maintaining redox homeostasis, glucose and lipid homeostasis, and endocrine homeostasis.

As used herein, "topical administration" refers to a pharmaceutical or cosmetic treatment of the skin, which is primarily understood as a pharmaceutical (or dermatological) treatment in pathological skin conditions. In contrast, the cosmetic treatment and/or prevention of undesired oxidative damage of the skin is primarily directed to healthy skin. Without being limited by any theory, the microRPG1 provided in the present disclosure provides, at an effective dose, an improvement in pathological skin or protection for healthy skin by ameliorating and alleviating oxidative stress.

Herein, ameliorating and alleviating oxidative stress includes increasing antioxidant factor reserves and removing oxidative substances *in vivo*; the antioxidant factors include superoxide dismutase (SOD), catalase (CAT), and glutathione peroxidase (GSH-px); the oxidative substances *in vivo* include reactive oxygen species (ROS), superoxide anion O²⁻, hydrogen peroxide H₂O₂, and hydroxyl radical OH⁻.

The micropeptide described herein may be formulated for topical, oral, or injection administration. The micropeptide may be combined with one or more pharmaceutically acceptable carriers and/or excipients, wherein the carriers and/or excipients are considered safe and effective and can be administered to an individual without causing undesired biological side effects or unwanted interactions. A carrier refers to all components in a pharmaceutical formulation other than one or more active ingredients.

Suitable oral dosage forms herein include tablets, capsules, solution formulations, suspension agents, syrups, and lozenges. Tablets may be prepared using compression or molding techniques well known in the art. Gelatin or non-gelatin capsules can be prepared as hard or soft capsule shells that can encapsulate liquid, solid, and semi-solid filling materials using techniques well known in the art.

Suitable injection formulations herein may be, for example, solutions or suspensions; solid forms suitable for preparation of solutions or suspensions following the addition of reconstitution media prior to injection; emulsions, such as water-in-oil (w/o) emulsions, oil-in-water (o/w) emulsions, and microemulsions, liposomes, or emulsions thereof. The carrier may be a solvent or dispersion medium comprising, for example, water, ethanol, one or more polyols (e.g., glycerol, propylene glycol, and liquid polyethylene glycol), oils such as vegetable oils (e.g., peanut oil, corn oil, sesame oil, and the like), and combinations thereof. The proper flowability can be maintained, for example, by the use of a coating such as lecithin, by the maintenance of the required particle size in the case of dispersion and/or by the use of surfactants. In many cases, it is preferred to include isotonic agents, e.g., sugars or sodium chloride.

Suitable cosmetic or dermatological formulations herein contain, with respect to the total weight of the formulation, 0.000001 wt% to 10 wt%, particularly 0.0001 wt% to 3 wt%, especially 0.001 wt% to 1 wt% of one or more micropeptides used according to the present disclosure. The cosmetic or dermatological formulations according to the present disclosure may be present in different forms. Therefore, the cosmetic or dermatological formulations may be solutions, anhydrous formulations, emulsions or microemulsions of the water-in-oil (W/O) type or the oil-in-water (O/W) type, e.g., multiple emulsions of the water-in-oil-in-water (W/O/W) type, gels, solid sticks, ointments, or aerosols. The micropeptide may also be incorporated into aqueous systems or surfactant formulations used for cleansing skin and hair.

The micropeptide of the present disclosure may also be formulated as a composition for animal food, a dietary supplement, or human food.

As used herein, "therapeutically effective" or "effective amount" means that the amount of the composition used is an amount sufficient to ameliorate one or more causes or symptoms of a disease or condition. Such amelioration only requires reduction or alteration, not necessarily elimination. As used herein, the terms "therapeutically effective amount", "therapeutic amount", and "pharmaceutically effective amount" are synonymous. Those skilled in the art can readily determine the suitable therapeutic amount.

The following examples are intended to illustrate the present disclosure, but not to limit the scope of the present disclosure. Modifications or substitutions to the methods, procedures, or conditions of the present disclosure are made without departing from the spirit and essence of the present disclosure, all of which fall within the scope of the present application. Unless otherwise specified, the examples follow conventional experimental conditions, such as those described in Molecular cloning: a laboratory manual (Sambrook J & Russell D W, 2001), or follow the conditions recommended by the manufacturer's instructions. Unless otherwise specified, all chemical reagents used in the examples are conventional commercially available reagents, and the technical means used in the examples are conventional means well known to those skilled in the art.

### Example 1. MicroRPG1 Micropeptide

### Example 1.1. Identification of QTL qKDR1 for Maize Kernel Water Content

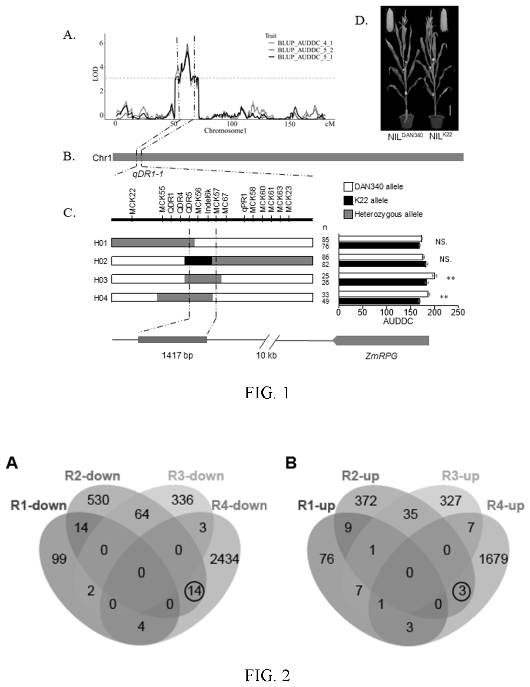

A population of 201 recombinant inbred lines derived from the hybridization of the maize inbred line DAN340 with K22 was used to detect QTL loci for the maize kernel dehydration rate. The population of DAN340/K22 recombinant inbred lines was planted in five geographic locations in China in a randomized block design: in 2013, Hainan (Sanya; 109.19° E, 18.38° N); in 2014, Hubei (Wuhan; 114.32° E, 30.58° N), Henan (Xinxiang; 113.81° E, 35.20° N), Liaoning (Shenyang; 123.47° E, 41.68° N), and Jilin (Gongzhuling; 124.83° E, 43.51° N). The water content of maize kernels was measured at 5 consecutive stages: 34, 40, 46, 52, and 58 days after pollination, and the area under the dry down curve (AUDDC) was calculated to measure the kernel dehydration rate. A mixed linear model was used to calculate the best linear unbiased prediction (BLUP) values to eliminate environmental impacts. BLUP values for each line were used as phenotypes for QTL mapping. One major QTL, i.e., *qKDR1*, was ultimately detected on the left arm of chromosome 1, which could explain 9.81% of the phenotypic variation. During the stage of fine mapping of *qKDR1*, heterozygous individuals were planted to screen for new recombination events, new recombinants were identified using flanking markers in the *qKDR1* region, and new molecular markers were developed to determine the breakpoint of the identified recombinant. For the new recombinants, the dehydration rates of NIL^{DAN340} and NIL^{K22} homozygous individuals of the progeny were compared using the Student's t-test. By integrating the QTL mapping information of all recombinants, the QTL was narrowed down to a 1417-bp non-coding region (FIG. 1). This segment corresponded to Chromosome 1: 20,007,756-20,009,147 in the B73 V4 genome. The markers and sequences for fine mapping of *qKDR1* are shown in Table 1.

**Table 1. Sequences of markers for fine mapping**

| Name | Name_F^{a} | Forward primer (5'-3') | Name_R^{b} | Reverse primer (5'-3') |
|---|---|---|---|---|
| MCK22 | MCK22_F1 SEQ ID NO. 59 | GAAGGTGACCAAGTT CATGCTTCCAGCGAG TGCAGGCCCTG | MCK22_R SEQ ID NO. 86 | GATTTGCAGTTCG AATCACC |
| | MCK22_F2 SEQ ID NO. 60 | GAAGGTCGGAGTCAA CGGATTTCCAGCGAG TGCAGGCCCTT | | |
| MCK55 | MCK55_F1 SEQ ID NO. 61 | GAAGGTGACCAAGTT CATGCTTGCATGTGA TGAGACGTTAG | MCK55_R SEQ ID NO. 87 | CCCAACAAGCTA GAATCAAAGG |
| | MCK55_F2 SEQ ID NO. 62 | GAAGGTCGGAGTCAA CGGATTTGCATGTGA TGAGACGTTAA | | |
| QDR1 | QDR1_F SEQ ID NO. 63 | TATTTGCCACCTCCA ACCTC | QDR1_R SEQ ID NO. 88 | GCAGCACATTTCC TGTCAGA |
| QDR4 | QDR4_F1 SEQ ID NO. 64 | GAAGGTGACCAAGTT CATGCTCGAGGCAGG AATGTTGCGT | QDR4_R SEQ ID NO. 89 | CGGACAGTCTAA GTGTCTTCG |
| | QDR4_F2 SEQ ID NO. 65 | GAAGGTCGGAGTCAA CGGATTCGAGGCAGG AATGTTGCGC | | |
| QDR5 | QDR5_F1 SEQ ID NO. 66 | GAAGGTGACCAAGTT CATGCTATCATAGTT TGCGTCCTAAC | QDR5_R SEQ ID NO. 90 | AATGATGACCAA CGGGTAGC |
| | QDR5_F2 SEQ ID NO. 67 | GAAGGTCGGAGTCAA CGGATTATCATAGTT TGCGTCCTAAT | | |
| MCK56 | MCK56_F1 SEQ ID NO. 68 | GAAGGTGACCAAGTT CATGCTACTCTCTGC GCCGGTTTCCC | MCK56_R SEQ ID NO. 91 | CTCCTCGATCAGG AAGAGTGA |
| | MCK56_F2 SEQ ID NO. 69 | GAAGGTCGGAGTCAA CGGATTACTCTCTGC GCCGGTTTCCG | | |
| Indel6k | Inde16k_F SEQ ID NO. 70 | GTGGTGCATGCATTG TTCAT | Indel6k_R SEQ ID NO. 92 | GTCTACAGCCACG AACACGA |
| MCK57 | MCK57_F1 SEQ ID NO. 71 | GAAGGTGACCAAGTT CATGCTGCGTCGTGC GCCGACACCAC | MCK57_R SEQ ID NO. 92 | GTCTACAGCCACG AACACGA |
| | MCK57_F2 SEQ ID NO. 72 | GAAGGTCGGAGTCAA CGGATTGCGTCGTGC GCCGACACCAG | | |
| MC67 | MC67_F SEQ ID NO. 73 | AGTACGAAACCGACA GCTGG | MC67_R SEQ ID NO. 94 | GAAGCCAGCACTT ACAAGCG |
| qPR1 | qPR1_F1 SEQ ID NO. 74 | GAAGGTGACCAAGTT CATGCTAGCATCTAG TTTCGTCCCTT | qPR1_R SEQ ID NO. 95 | TCATACGCACGAC GCTGTAG |
| | qPR1_F2 SEQ ID NO. 75 | GAAGGTCGGAGTCAA CGGATTGCATCTAGT TTCGTCCCTGC | | |
| MCK58 | MCK58_F1 SEQ ID NO. 76 | GAAGGTGACCAAGTT CATGCTGTGAGCCAA AGAATGCCTGG | MCK58_R SEQ ID NO. 96 | CGAGAACGTTGA GATGACTGA |
| | MCK58_F2 SEQ ID NO. 77 | GAAGGTCGGAGTCAA CGGATTGTGAGCCAA AGAATGCCTGT | | |
| MCK60 | MCK60_F1 SEQ ID NO. 78 | GAAGGTGACCAAGTT CATGCTGATTAATCA ACCAAAATCTA | MCK60_R SEQ ID NO. 97 | AAACATGTTGAAT CTTGCAC |
| | MCK60_F2 SEQ ID NO. 79 | GAAGGTCGGAGTCAA CGGATTGATTAATCA ACCAAAATCTC | | |
| MCK61 | MCK61_F1 SEQ ID NO. 80 | GAAGGTGACCAAGTT CATGCTAGCCAAAAA TGGTAAATTGG | MCK61_R SEQ ID NO. 98 | AAAGGTGGCCAA CATCTCAC |
| | MCK61_F2 SEQ ID NO. 81 | GAAGGTCGGAGTCAA CGGATTAGCCAAAAA TGGTAAATTGA | | |
| MCK63 | MCK63_F1 SEQ ID NO. 82 | GAAGGTGACCAAGTT CATGCTGGTTCTAGT CCTCAGTAGGG | MCK63_R SEQ ID NO. 99 | GGACTCGAATGA ATCGTGGA |
| | MCK63_F2 SEQ ID NO. 83 | GAAGGTCGGAGTCAA CGGATTGGTTCTAGT CCTCAGTAGGT | | |
| MCK23 | MCK23_F1 SEQ ID NO. 84 | GAAGGTGACCAAGTT CATGCTTAAGGCCTG CATGTTTTAAT | MCK23_R SEQ ID NO. 100 | TGCAGTTTCTCTA ACTGTTCCA |
| | MCK23_F2 SEQ ID NO. 85 | GAAGGTCGGAGTCAA CGGATTTAAGGCCTG CATGTTTTAAG | | |

Name_F and Name_R represent the names of a forward primer and a reverse primer, respectively.

Since this segment was located in an intergenic region where it was neither transcribed nor translated, this segment may affect changes in the expression levels of other genes and thus affect changes in the kernel water content.

### Example 1.2. Identification of ZmRPG Gene

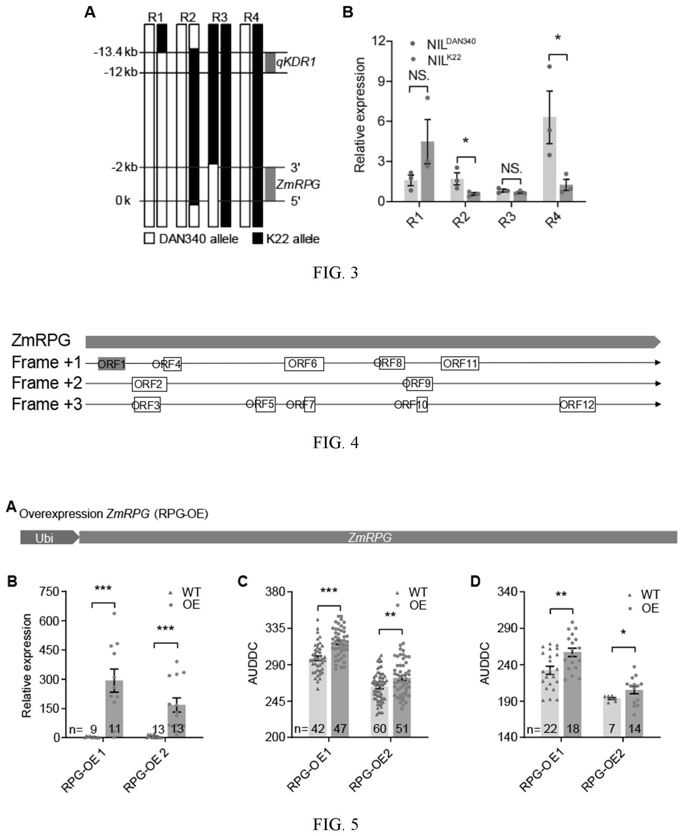

The inventors further used two pairs of NIL materials (designated as R1 to R4, with the genotype information shown in FIG. 3A), one pair with segregation and the other without segregation in the target segment, and selected kernels at the late stage of ripening for RNAseq analysis to identify differentially expressed genes. Through the joint analysis of multiple sets of RNAseq data, a total of 17 differentially expressed genes were identified (FIG. 2). Among them, R1 and R3 did not segregate in the *qKDR1* segment, meaning that the differentially expressed genes derived therefrom should not be the target gene; R2 and R4 segregated in the *qKDR1* segment, meaning that the differentially expressed genes derived therefrom should include the gene regulated by *qKDR1*. Therefore, the differentially expressed gene *ZmRPG* was identified in this way, located about 10 kb downstream of *qKDR1* (FIG. 1). The expression level of *ZmRPG* in the R1-R4 populations was further verified using a qPCR method. It was found that in the R2 and R4 populations, the expression level of *ZmRPG* in the NIL^{DAN340} genotype material was higher than that in the NIL^{K22} genotype material. This indicates that the *qKDR1* fragment may affect the expression of the *ZmRPG* gene (FIG. 3). Since NIL^{K22} had a faster kernel dehydration rate than NIL^{DAN340}, the higher the expression level of *ZmRPG*, the slower the kernel dehydration rate; the lower the expression level, the faster the kernel dehydration rate. By comparing the sequences of near-isogenic lines, it was found that the *ZmRPG* gene in NIL^{DAN340} had a full length of 2010 bp (SEQ ID NO. 1), and the *ZmRPG* gene in NIL^{K22} had a full length of 2013 bp (SEQ ID NO. 2). There were only a few base variations between these two sequences, and these base variations did not affect the function of the gene.

The *ZmRPG* gene was located at Chromosome 1: 20,019,106-20,021,118 in the B73 V4 genome, with a full length of 2013 bp. The *ZmRPG* gene was not annotated in the B73-V4.0 reference genome, but transcription signals were present. The inventors further performed sRNA-seq and Ribo-seq on kernels at the late stage of ripening and found that this segment was not covered by small RNA. By combining ORF annotations of unknown sequences, it was found that ZmRPG could be annotated into 12 ORFs, all of which were small peptides with only dozens of amino acids (FIG. 4). Furthermore, it was found that the positions of ORF1, ORF2, and ORF3 were covered by Ribo-seq reads, indicating that they can be translated into proteins. Through further analysis and validation in associated populations, it was found that only ORF1 (designated as microRPG1) was a small peptide protein that affects the maize kernel dehydration rate. The nucleotide sequence encoding microRPG1 was 96 bp in length (SEQ ID NO. 3), and the amino acid sequence was 31 amino acids in length (SEQ ID NO. 4).

### Example 1.3. Manipulation of ZmRPG to Alter Maize Kernel Dehydration Rate

Since different expression levels of ZmRPG can regulate different maize kernel dehydration rates, the inventors further tried to overexpress ZmRPG (SEQ ID NO. 1 and SEQ ID NO. 2) in the maize inbred line B104 using a maize *ubiquitin* promoter to verify its effect. The terminator used was a commonly used nos terminator. A total of two overexpression strains, RPG-OE1 and RPG-OE2, were generated. Compared with wild-type controls, both RPG-OE1 and RPG-OE2 exhibited slower dehydration rates (FIG. 5), indicating that increasing ZmRPG expression can reduce the dehydration rate of maize kernels.

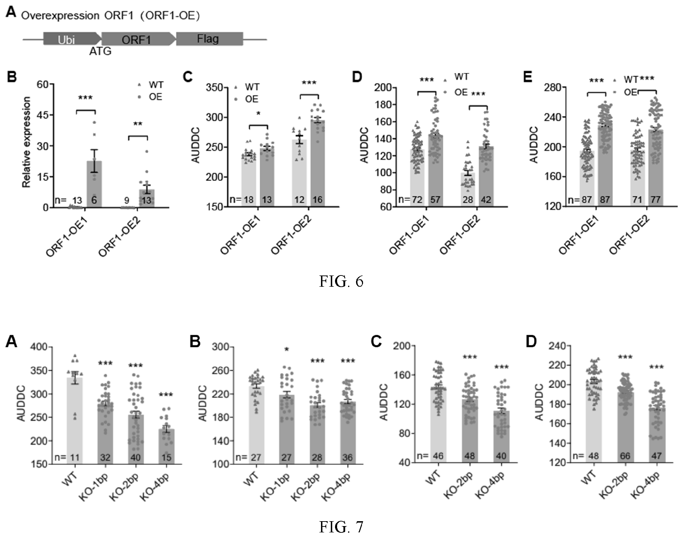

Further overexpression of microRPG1 in the maize inbred line B104 using a similar method (using the same promoter and terminator as in the previous paragraph) also resulted in two overexpression strains, ORF1-OE1 and ORF1-OE2. Compared with wild-type controls, ORF1-OE1 and ORF1-OE2 also exhibited slower dehydration rates (FIG. 6), indicating that increasing microRPG1 can also reduce the dehydration rate of maize kernels.

The present disclosure further used CRISPR-Cas9 technology to knock out microRPG1 in the maize inbred line B104. The designed target sequence is set forth in SEQ ID NO. 5, and the gRNA sequence for target recognition is set forth in SEQ ID NO. 6. For the remaining maize gene editing operations, reference can be made to the content of Example 2 in CN112646013A.

After gene editing, 3 mutation types, KO-1bp (SEQ ID NO. 7), KO-2bp (SEQ ID NO. 8), and KO-4bp (SEQ ID NO. 9), were generated. Compared with the wild types, KO-1bp, KO-2bp, and KO-4bp knockouts all exhibited faster dehydration rates (FIG. 7), indicating that the dehydration rate of maize kernels can be increased by knocking out microRPG1.

### Example 1.4. Effect of microRPG1 Micropeptide in Other Plant Species

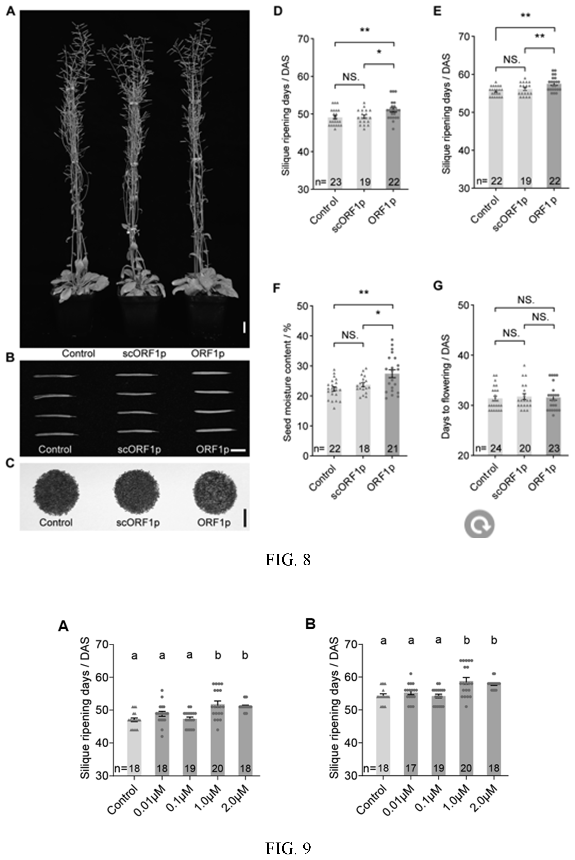

To further investigate the function of microRPG1, microRPG1 (designated as ORF1p) was chemically synthesized based on the amino acid sequence (SEQ ID NO. 4) of the micropeptide and prepared as a 2 µM aqueous solution. The model plant *Arabidopsis thaliana* was treated by *in vitro* spraying or direct addition to the soil, with a treatment time set at the beginning of silique ripening and at the 50% silique ripening stage. A randomized sequence with the same amino acid composition as the microRPG1 micropeptide (designated as scORF1p) was used as a control peptide for treatment. The results showed that regardless of treatment at the beginning of silique ripening or at the 50% silique ripening stage, ORF1p could delay silique ripening (but the anthesis remained unchanged), and the water content of seeds was significantly increased. However, when treatment was performed using scORF1p and clean water, the silique ripening and water content did not change (FIG. 8).

The present disclosure also set up a concentration gradient experiment (from 0.01 µM to 2 µM) to test the effect of ORF1p on the ripening of *Arabidopsis thaliana* silique, and reference was made to the previous paragraph for the treatment method. The results showed that the ORF1p micropeptide at 1 µM and 2 µM could delay the ripening of *Arabidopsis thaliana* silique (FIG. 9).

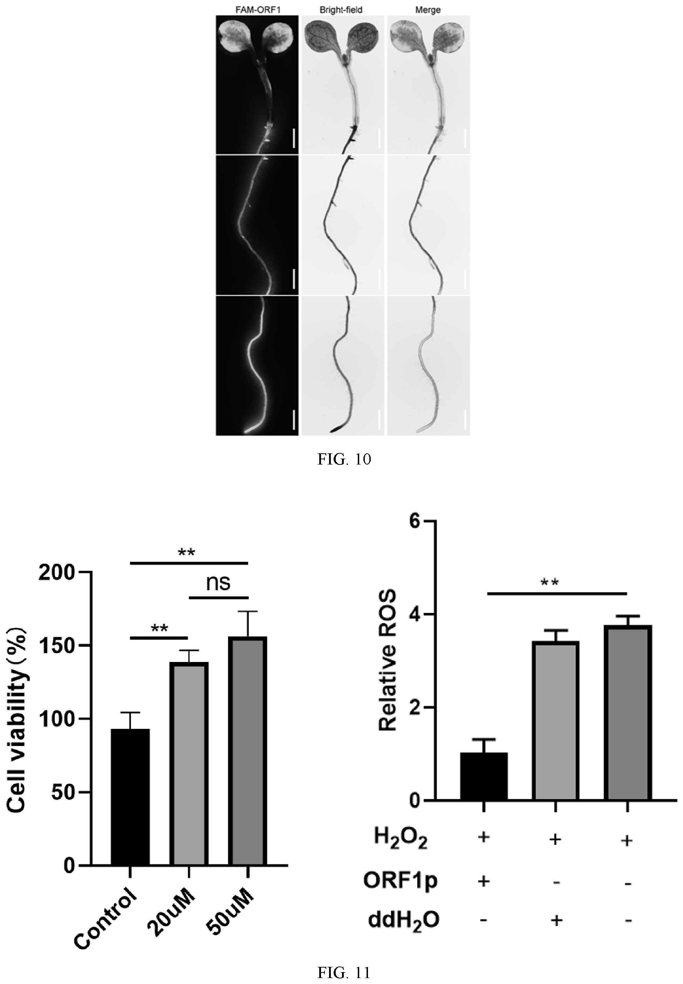

In addition, the present disclosure further synthesized a fluorescent-labeled microRPG1 micropeptide (designated as FAM-ORF1p). FAM-ORF1p was applied *in vitro* to *Arabidopsis thaliana* at a concentration of 2 µM. It was found through observation with a fluorescence microscope that FAM-ORF1p could be absorbed by the root of *Arabidopsis thaliana* and transported into the aerial cotyledons (FIG. 10). This indicates that the microRPG1 micropeptide can be absorbed by plant roots to affect the growth and development of plants, and can enter plant cells.

The above results indicate that the microRPG1 micropeptide can affect the growth, development, and ripening of plants across species. Therefore, the manipulation of microRPG1 or the *in vitro* application of the microRPG1 micropeptide can alter the processes of growth and development, particularly delay ripening.

### Example 1.5. Anti-Aging Effect of microRPG1 Micropeptide in Other Species

The present disclosure further assayed the anti-aging effect of the microRPG1 micropeptide on other non-plant species.

Chemically synthesized micropeptide ORF1p was co-cultured with 293T cells (a cell line derived from human embryonic kidney cells) at concentrations of 20 µM and 50 µM in a cell culture medium, and the cell viability was determined using a CCK-8 kit (BCCK1000). It was found that ORF1p could significantly improve 293T cell viability. Then, the 293T cells were treated with H₂O₂ (hydrogen peroxide) at a concentration of 200 µM and ORF1p at a concentration of 50 µM. The specific process was as follows: 293T cells were stimulated with H₂O₂ at a concentration of 200 µM for 4 h and then cultured for 24 h to construct a cell oxidative stress model. Then, in the experimental group, ORF1p was added to the culture medium (at a final concentration of 50 µM), while in the control group, an equal amount of double-distilled water was added or nothing was added. Co-culture was performed for 24 h. The content of reactive oxygen species was then assayed using a reactive oxygen species (ROS) assay kit. It was found that compared with the control group, ORF1p had the potential function of reducing the production of reactive oxygen species at the cellular level and alleviating oxidative stress (FIG. 11). The results indicate that the microRPG1 micropeptide also has the function of delaying aging in human cells.

About 60%-80% of genes in *Caenorhabditis elegans* are homologous to human genes, including at least 42% of human disease-related genes (Sugi et al., 2016). These advantages enable *C. elegans* to be a widely used animal model, which is often used for studying the direction related to anti-aging.

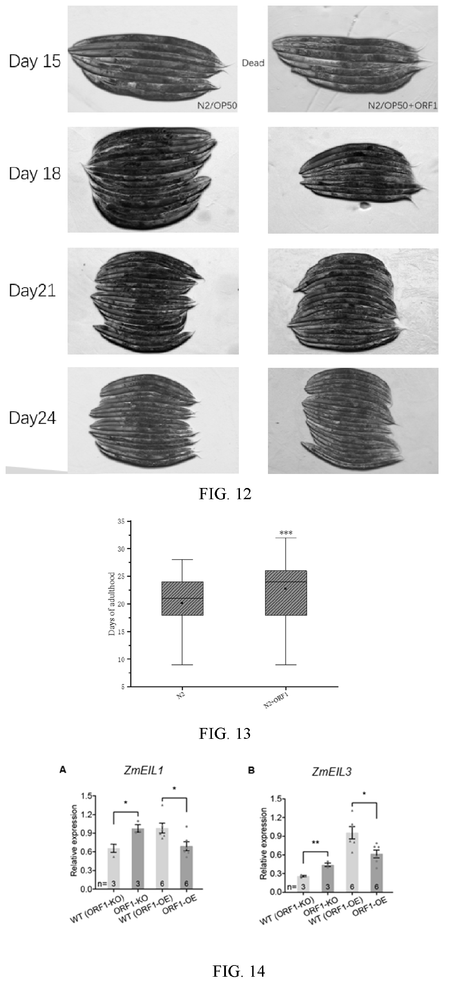

The model animal was hermaphroditic N2 wild-type *Caenorhabditis elegans* (*C*. *elegans*), which was further treated with ORF1p at a concentration of 50 µM. A treatment group and a control group were set, with 7 replicates in each group. For each replicate, 20 worms were treated. The treatment method was as follows: An *Escherichia coli* OP50 bacterial solution and the microRPG1 micropeptide were well mixed and then spread onto a culture dish, and bacterial shaking and bacterial spreading were performed weekly to ensure the effectiveness of the microRPG1 micropeptide. The bacteria were cultured at 20 °C for not less than 40 days. It was found through direct observation that intestinal particulate matter aggregation occurred later in nematodes fed with ORF1 than in the control group (FIG. 12; intestinal particulate matter aggregation is an important indicator associated with nematode aging). A survey on nematode lifespan showed that the average lifespan of nematodes in the ORF1p treatment group was increased by about 13.2% compared with that in the control group (FIG. 13).

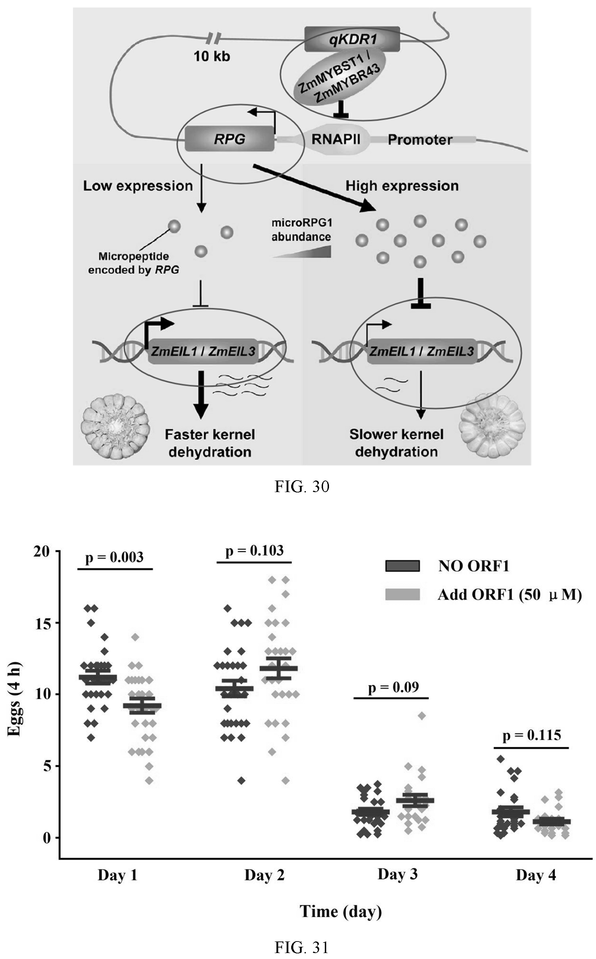

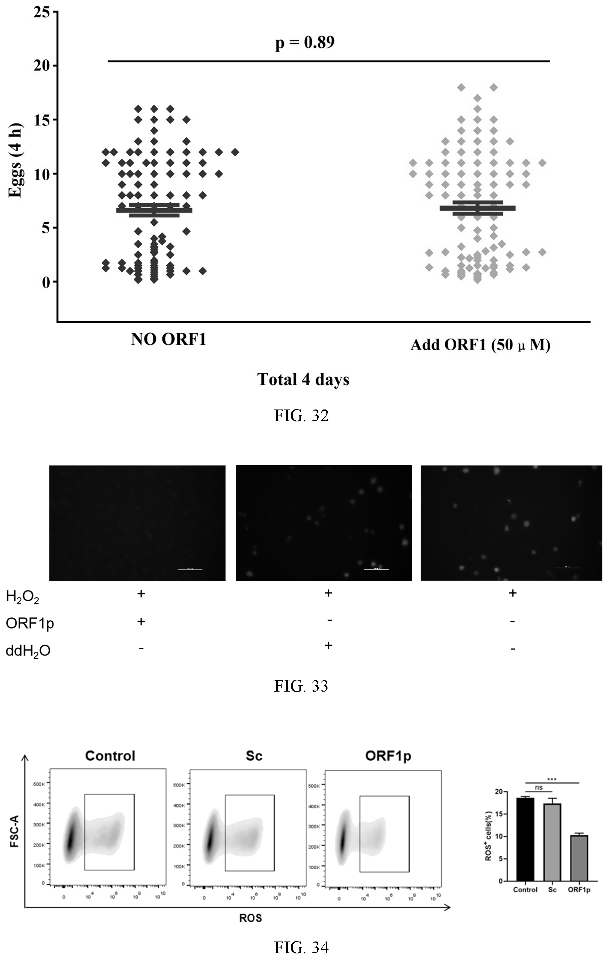

The effect of ORF1p treatment on the egg-laying of wild-type *Caenorhabditis elegans* was also studied. The number of laid eggs of the wild-type *Caenorhabditis elegans* at 4 days (day 1, day 2, day 3, and day 4) after adulthood was counted. The statistical time course was 4 h, and the number of laid eggs without ORF1p addition and with ORF1p addition was counted separately. The results showed that the addition of ORF1 reduced the egg-laying of nematodes on day 1. There was an increase in the egg-laying of nematodes on day 2 and day 3 (FIG. 31), while there was no significant difference in the total number of laid eggs within 4 days (FIG. 32). The above results indicate that the addition of ORF1 can delay the egg-laying of nematodes, but the total number of laid eggs remains unchanged, thereby indirectly demonstrating the delay of nematode aging.

Further, mice were injected intravenously with 100 µL of normal saline (0.9%) containing 0.2 µg-20 µg of ORF1p to observe the performance of the mice after injection of the micropeptide. It was found that ORF1p could also prolong the lifespan of the mice. In addition, fruit flies were fed with a culture medium supplemented with the micropeptide at 3 different concentrations (2 µM, 10 µM, and 50 µM), and it was found that the micropeptide could also prolong the lifespan of fruit flies. The culture medium for feeding fruit flies contained the following ingredients: 66.825 g of cornmeal, 9.18 g of soybean meal, 6 g of agar, 40 g of sucrose, 42.4 g of maltose, 25 g of yeast, 6.85 mL of propionic acid, 1 g of sodium benzoate, 0.25 g of methylparaben, and different contents of the micropeptide, and the mixture was finally brought to 1 L.

### Example 1.6. Identification of Homologous Proteins of microRPG1 in Zea luxurians

The inventors searched for homologous sequences of the microRPG1 micropeptide in *Zea luxurians* by sequence alignment, and 8 similar micropeptides (sequences set forth in SEQ ID NOs. 11-18) were identified in 8 different wild-type *Zea luxurians* species (*Zea luxurians*-RIL003, *Zea nicaraguensis-*PI615697, *Zea diploperennis*-Gigi, *Zea diploperennis*-Momo, *Zea perennis*, *Zea mays subspecies huehuetenangensis*-RIMHU001, *Zea mays subspecies parviglumis*, and *Zea mays subspecies mexicana*-TIL25), respectively.

The inventors further synthesized homologous micropeptides found in *Zea luxurians* and assayed their functions according to the methods of Example 4 and Example 5. It was found that the 8 micropeptides, like microRPG1 in maize, could also affect the kernel water content trait of plants and had the effect of delaying the aging of plants and animals.

### Example 1.7. Effect of microRPG1 Micropeptide on Cellular ROS Production

A cell aging model was induced by using hydrogen peroxide (H₂O₂). The cells were seeded into a 6-well plate at a density of 70%-80%. After cell adhesion, the cells were treated with hydrogen peroxide at a concentration of 200 µM for 4 h. Then, the culture medium was changed, and the cells were cultured for 24 h. Thus, the aging model was successfully constructed. Treatment groups with the respective addition of the small peptide and a randomized-sequence small peptide at a concentration of 50 µM were set up, and a blank control group with the addition of an equal amount of dd water was set. After 24 h of co-culture, the content of reactive oxygen species (ROS) was determined using a fluorescent probe DCFH-DA. Imaging was performed using an inverted fluorescence microscope, and detection was performed using a flow cytometer.

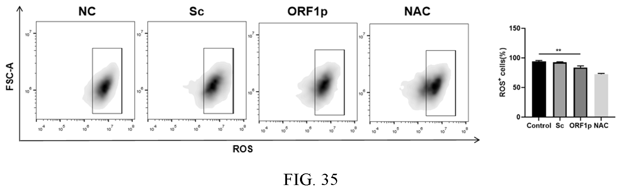

The experimental results showed that the microRPG1 micropeptide had the potential function of reducing the production of reactive oxygen species in senescent cells (FIGs. 33-35) and alleviating oxidative stress.

Therefore, these microRPG1 micropeptides can be developed into cosmetic raw materials, drugs, health care products, plant nutritional liquids, and other products for use in fields such as increasing the plant kernel water content, anti-aging, and the like.

### Example 2. Promoter for Controlling Dehydration of Maize Kernels and Ethylene Signaling Pathway Genes

### Example 2.1. Effect of microRPG1 Micropeptide on Expression Levels of EIL Genes

Through RNA-seq analysis of microRPG1 knockout, overexpression, and in vitro peptide treatment (using 2 µM chemically synthesized microRPG1, designated as ORF1p), a total of 505 differentially expressed genes were identified. Among the 505 differentially expressed genes, the genes co-expressed with the *ZmRPG* gene in the kernel were screened, and 11 genes were found to be co-expressed with the *ZmRPG* gene, among which Zm00001d047563 had the highest expression level. Therefore, the microRPG1 micropeptide may regulate the expression of Zm00001d047563 (designated as ZmEIL1). Zm00001d047563 is a key gene in the ethylene signaling pathway. There are a total of 4 homologous genes in maize, among which ZmEIL1 (Zm00001d047563) and ZmEIL3 (Zm00001d028974) are highly expressed in kernels. The two genes were selected for further analysis. The nucleic acid sequences of ZmEIL1 and ZmEIL3 are set forth in SEQ ID NO. 19 and SEQ ID NO. 20.

The inventors further analyzed the expression levels of ZmEIL1 and ZmEIL3 genes in the maize materials with microRPG1 micropeptide knockout and overexpression, and found that the expression levels of ZmEIL1 and ZmEIL3 were increased in the knockout material and reduced in the overexpression material (FIG. 14), indicating that the expression levels of ZmEIL1 and ZmEIL3 genes in maize are inhibited by the microRPG1 micropeptide.

### Example 2.2. Cloning of Maize Kernel Water Content Gene

Since the expression levels of EIL genes were inhibited by the microRPG1 micropeptide, to clarify the specific functions of the ZmEIL1 and ZmEIL3 genes, the inventors knocked out ZmEIL1 and ZmEIL3 genes in maize using a CRISPR-Cas9 tool. The target sequences of ZmEIL1 were TCGCCTGGTTCGCCAGTCCA (SEQ ID NO. 102) and CGCAGTGACTACAGCTACGG (SEQ ID NO. 103), and the gRNA sequences were SEQ ID NO. 21 and SEQ ID NO. 22. The target sequences of ZmEIL3 were GAGCTGCAGGACACCACACT (SEQ ID NO. 104) and CCCTACAAGAAGCCCCATGA (SEQ ID NO. 105), and the gRNA sequences were SEQ ID NO. 23 and SEQ ID NO. 24. ZmEIL1-edited plants ZmEIL1-KO1 and ZmEIL1-KO2 were obtained, with sequences set forth in SEQ ID NOs. 25-26, respectively; ZmEIL3-edited plants ZmEIL3-KO1, ZmEIL3-KO2, and ZmEIL3-KO3 were obtained, with sequences set forth in SEQ ID NOs. 27-29, respectively.

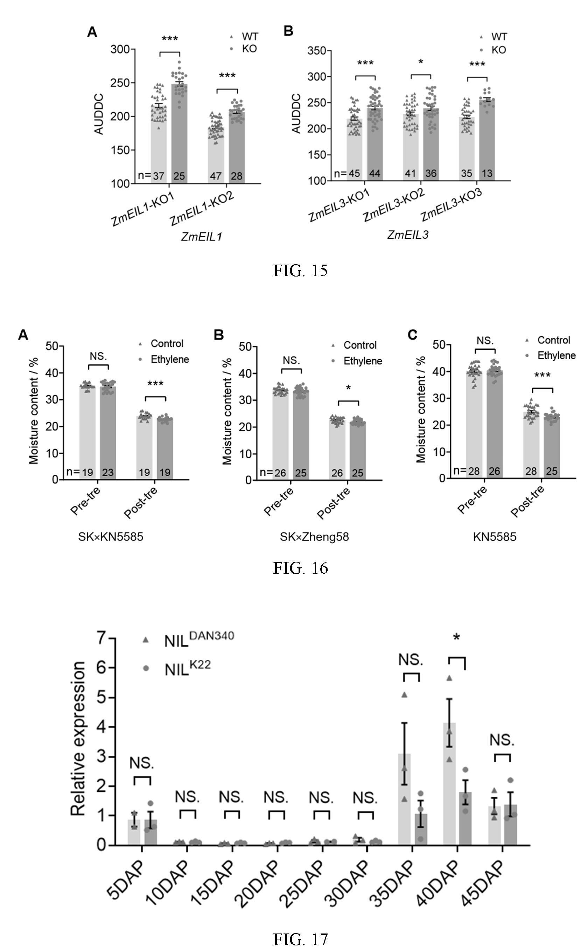

The phenotype of the maize material after the knockout of ZmEIL1 and ZmEIL3 genes was investigated, and the results showed that the dehydration rate of maize kernels slowed down after the knockout of ZmEIL1 and ZmEIL3 genes (FIG. 15), indicating that ZmEIL1 and ZmEIL3 control the dehydration rate of maize kernels. The dehydration of maize kernels could be slowed down by knocking out ZmEIL1 and ZmEIL3.

### Example 2.3. Effect of Ethylene Treatment on Dehydration of Maize Kernels

Since ZmEIL1 and ZmEIL3 were key genes in the ethylene signaling pathway, maize kernels were further treated through *in vitro* application of ethylene. Specifically, maize ears were directly sprayed with 277 mM ethephon (5-10 mL per ear) at the late stage of maize kernel development to investigate changes in the dehydration of maize kernels. It was found that in the maize materials with three different backgrounds, the ethylene treatment could accelerate the dehydration rate of maize kernels (FIG. 16).

### Example 2.4. Identification of Three Kernel-Specific Expression Promoters

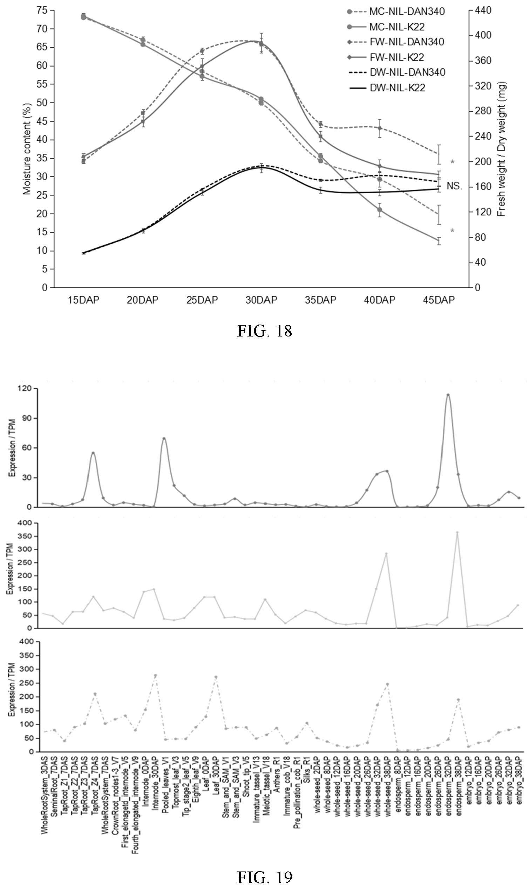

In the process of studying the three genes (*ZmRPG*, *ZmEIL1*, and *ZmEIL3*) described above, the inventors found that they all had the characteristics of high expression in kernels and specific high expression at the late stage of kernel development. In order to further define their expression patterns, the inventors assayed the expression of the genes in maize kernels 5 to 45 days after pollination by using the qPCR experiment, and found that Expression of the ZmRPG gene began 35 days after pollination (FIG. 17). To further study the effect of the ZmRPG gene on the kernel development, kernels of the maize near-isogenic lines NIL^{DAN340} and NIL^{K22} from 15 days after pollination (15DAP) to 45 days after pollination (45DAP) were analyzed for their fresh weight, dry weight, and water content indicators. The results showed that at harvest (45DAP), there was no difference in the dry weight between NIL^{DAN340} and NIL^{K22}; however, there was a significant difference in the water content (FIG. 18). Therefore, the knockout of the microRPG1 micropeptide does not affect the yield of kernels. This is because the ZmRPG gene begins to be expressed at the late stage of kernel development, at which time the accumulation of dry matter in the kernels has already been completed; thus, the knockout of the microRPG1 micropeptide does not affect the yield of kernels. This also indicates that the genes expressed at the late stage of kernel development and capable of controlling the maize kernel dehydration rate have great significance for improving the dehydration rate. The *ZmEIL1* and *ZmEIL3* genes also had the characteristics of high expression in kernels. The expression of the three genes in tissues at different development stages of maize is shown in FIG. 19.

The characteristics of *ZmRPG*, *ZmEIL1*, and *ZmEIL3* genes enable the knockout of the genes to accelerate the dehydration without affecting the anthesis, agronomic traits, and yield-related traits. Therefore, driving other genes that affect dehydration to be expressed at the late stage of kernel development by using the promoters of *ZmRPG*, *ZmEIL1*, and *ZmEIL3* genes has great significance for improving the dehydration rate. The inventors cloned and determined the sequences of promoters of the three genes described above, which are set forth in SEQ ID NO. 30-SEQ ID NO. 32, respectively.

### Example 3. Genes ZmMYBST1 and ZmMYBR43 Controlling Maize Kernel Dehydration Rate and Kernel Water Content

### Example 3.1. Screening for qKDR1-Binding Proteins

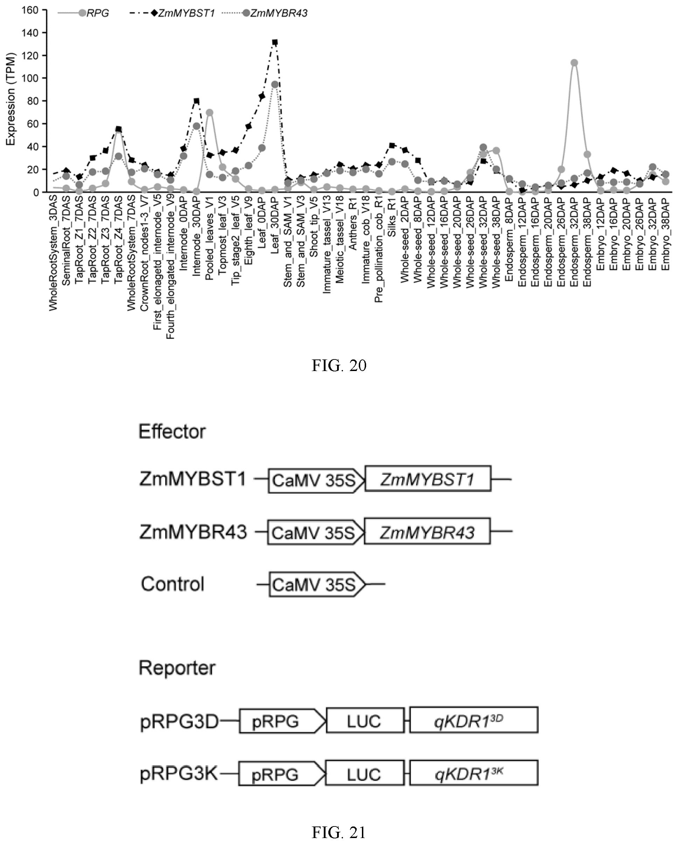

The inventors first analyzed disclosed chromatin immunoprecipitation sequencing data (see Tu X, Mejía-Guerra MK, Valdes Franco JA, et al., Reconstructing the maize leaf regulatory network using ChIP-seq data of 104 transcription factors. Nat Commun, 2020, 11: 5089) and found that 43 transcription factors could bind to the position of the *qKDR1* (see Example 4) fragment. The kernel expression levels of these transcription factors in B73 and the knockout material of another gene, *RPG*, regulated by the *qKDR1* fragment (see Example 1 for details) were further analyzed, and a total of 26 genes with low expression or no expression in the kernels were removed (Table 2). The remaining 17 genes were analyzed in depth for their expression levels in tissues throughout the entire growth stage of B73, and the expression patterns of the two genes, Zm00001d000184 and Zm00001d029875, in maize kernels, embryos, and endosperms were found to be similar to that of *RPG* (FIG. 20). The inventors designated the Zm00001d000184 and Zm00001d029875 genes as ZmMYBST1 and ZmMYBR43, respectively (the genome sequences are set forth in SEQ ID NO. 33 and SEQ ID NO. 34, respectively), to further verify their functions.

**Table 2. Transcription factors likely to bind to qKDR1**

| **Gene** | **KO-1** | **KO-2** | **KO-3** | **WT-1** | **WT-2** | **WT-3** | **Expression level in kernels** |
|---|---|---|---|---|---|---|---|
| *Zm00001d016861* | 36.72 | 41.70 | 40.22 | 36.06 | 36.87 | 33.02 | |
| *Zm00001d017176* | 1.31 | 1.56 | 1.60 | 3.18 | 2.96 | 3.60 | Low |
| *Zm00001d025770* | 0.00 | 0.00 | 0.00 | 0.00 | 0.00 | 0.00 | No |
| *Zm00001d015468* | 3.06 | 4.41 | 3.27 | 1.70 | 1.37 | 1.15 | Low |
| *Zm00001d016793* | 9.28 | 10.68 | 11.17 | 10.76 | 11.23 | 9.71 | |
| *Zm00001d020954* | 165.68 | 190.61 | 200.00 | 79.53 | 72.39 | 73.08 | |
| *Zm00001d024324* | 1.76 | 1.68 | 2.19 | 2.27 | 3.67 | 3.34 | Low |
| *Zm00001d020595* | 6.95 | 8.32 | 7.32 | 4.36 | 4.18 | 4.99 | Low |
| *Zm00001d043205* | 30.36 | 29.98 | 30.67 | 20.79 | 22.87 | 23.42 | |
| *Zm00001d044232* | 29.87 | 35.82 | 31.36 | 17.58 | 14.99 | 16.92 | |
| *Zm00001d030891* | 6.48 | 6.70 | 5.76 | 5.09 | 6.11 | 5.17 | Low |
| *Zm00001d026542* | 0.18 | 0.03 | 0.27 | 0.06 | 0.00 | 0.03 | No |
| *Zm00001d017726* | 1.67 | 2.13 | 1.76 | 1.56 | 1.92 | 1.87 | Low |
| *Zm00001d039260* | 0.38 | 0.35 | 0.38 | 0.24 | 0.60 | 0.51 | No |
| *Zm00001d010634* | 0.04 | 0.04 | 0.00 | 0.00 | 0.03 | 0.00 | No |
| *Zm00001d044785* | 0.04 | 0.00 | 0.00 | 0.00 | 0.00 | 0.00 | No |
| *Zm00001d006551* | 11.17 | 10.26 | 10.91 | 10.73 | 8.44 | 9.33 | |
| *Zm00001d002799* | 3.33 | 3.94 | 3.06 | 1.25 | 1.36 | 0.62 | Low |
| *Zm00001d015549* | 18.08 | 15.35 | 16.98 | 19.13 | 15.54 | 16.91 | |
| *Zm00001d009599* | 0.00 | 0.00 | 0.00 | 0.00 | 0.00 | 0.00 | No |
| *Zm00001d000184* | 12.07 | 13.07 | 12.36 | 9.28 | 10.59 | 11.07 | |
| *Zm00001d024644* | 28.39 | 29.55 | 31.82 | 27.34 | 25.94 | 27.98 | |
| *Zm00001d029875* | 12.96 | 13.58 | 14.89 | 13.19 | 11.91 | 12.26 | |
| *Zm00001d013777* | 34.51 | 34.00 | 36.52 | 45.51 | 46.45 | 45.38 | |
| *Zm00001d048623* | 0.49 | 0.15 | 0.23 | 0.96 | 0.96 | 0.62 | Low |
| *Zm00001d032024* | 60.01 | 63.74 | 60.47 | 19.38 | 16.64 | 17.18 | |
| *Zm00001d013399* | 0.00 | 0.00 | 0.00 | 0.00 | 0.00 | 0.00 | No |
| *Zm00001d039893* | 5.74 | 6.14 | 5.67 | 6.48 | 6.25 | 6.02 | Low |
| *Zm00001d044409* | 0.53 | 0.90 | 0.77 | 0.81 | 1.30 | 0.93 | Low |
| *Zm00001d029963* | 10.89 | 10.41 | 8.22 | 6.06 | 6.80 | 6.65 | Low |
| *Zm00001d006236* | 5.50 | 6.11 | 7.39 | 4.84 | 5.52 | 5.06 | Low |
| *Zm00001d041576* | 7.43 | 7.96 | 8.32 | 7.33 | 7.05 | 5.91 | Low |
| *Zm00001d042609* | 167.62 | 177.63 | 171.63 | 177.68 | 180.40 | 184.79 | |
| *Zm00001d012527* | 38.67 | 40.88 | 37.34 | 23.87 | 22.75 | 20.44 | |
| *Zm00001d020614* | 0.27 | 0.12 | 0.64 | 0.19 | 0.05 | 0.16 | No |
| *Zm00001d033050* | 1.15 | 1.50 | 2.04 | 1.52 | 1.71 | 3.27 | Low |
| *Zm00001d047017* | 4.55 | 5.26 | 6.14 | 3.43 | 3.49 | 4.35 | Low |
| *Zm00001d033267* | 5.60 | 8.07 | 8.12 | 2.30 | 2.38 | 2.45 | Low |
| *Zm00001d021019* | 25.82 | 29.47 | 25.05 | 22.97 | 22.11 | 21.71 | |
| *Zm00001d031717* | 0.00 | 0.00 | 0.00 | 0.00 | 0.00 | 0.00 | No |
| *Zm00001d034298* | 3.13 | 3.07 | 2.66 | 3.50 | 2.77 | 3.45 | Low |
| *Zm00001d015421* | 51.38 | 52.75 | 52.03 | 36.27 | 36.41 | 37.65 | |
| *Zm00001d020025* | 39.75 | 40.42 | 37.78 | 24.16 | 24.64 | 25.28 | |

KO-1 to KO-3 are the three replicates of expression data in the RPG gene knockout material, and WT-1 to WT-3 are the three replicates of expression data in the wild-type B73 material.

### Example 3.2. Validation of Binding Function of ZmMYBST1 and ZmMYBR43

The inventors designed a transient transcriptional activity assay based on an LUC (firefly luciferase) system to verify the binding of ZmMYBST1 and ZmMYBR43 proteins to the *qKDR1* fragment. The specific procedures were as follows:
The protein coding sequences of ZmMYBST1 and ZmMYBR43 (set forth in SEQ ID NO. 35 and SEQ ID NO. 36, respectively) driven by the CaMV 35S promoter were used as effectors, and pRPG3D and pRPG3K with a *qKDR1* fragment were used as reporters. The structures of the effectors and reporters are shown in (FIG. 21). The LUC promoter in the reporter used the promoter of the *RPG* gene (for the sequence, see SEQ ID NO. 30 in Example 2.4). ZmMYBST1 and ZmMYBR43 were respectively co-expressed with pRPG3D or pRPG3K as experimental groups. The control without the coding gene was co-expressed with pRPG3D (containing a qKDR1^{3D} fragment) or pRPG3K (containing a qKDR1^{3K} fragment) as control groups, and the sequences of the qKDR1^{3D} fragment and the qKDR1^{3K} fragment are shown in Example 4.2. Protoplasts were extracted from the leaves of etiolated seedlings of the maize inbred line B73 and transformed into plasmids. After the completion of transformation and culture, the cellular proteins were extracted, and the operation was performed according to the scheme provided in the kit; that is, the LUC and REN signals were collected using a dual luciferase reporter analysis system (Promega, Cat. No.: E1980). Finally, the relative LUC activity, i.e., the value of LUC/REN, was calculated.

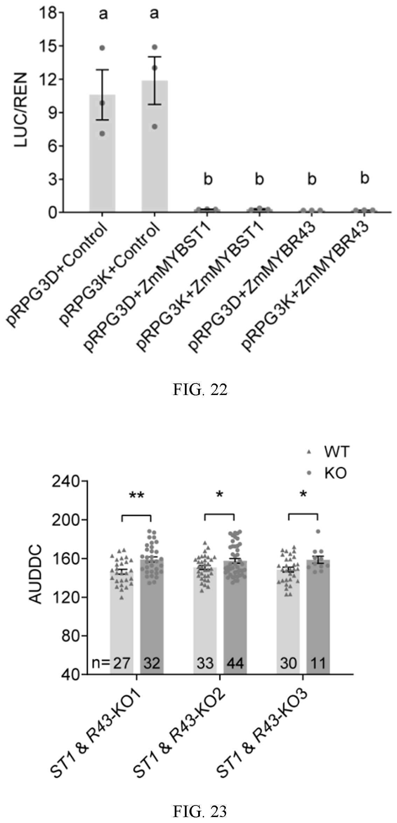

The experimental results showed that compared with the control groups, in the experimental groups, the co-expression with ZmMYBST1 or ZmMYBR43 resulted in strong inhibition of the LUC activity of the reporter genes of the two reporters (FIG. 22). The experimental results indicate that the two transcription factors, ZmMYBST1 and ZmMYBR43, both bind to the *qKDR1* region and inhibit the expression of the *RPG* gene.

### Example 3.3. Gene Knockout Experiment

The inventors further used a CRISPR-Cas9 tool to knock out the ZmMYBST1 and ZmMYBR43 genes in maize to verify the extent of their effect on the maize kernel dehydration trait.

Maize B104 was selected as the recipient. The designed target sequences are set forth in SEQ ID NO. 39 and SEQ ID NO. 40, and the gRNA sequences binding to the target are set forth in SEQ ID NO. 41 and SEQ ID NO. 42. For the remaining maize gene editing operations, reference can be made to the content of Example 2 in CN112646013A.

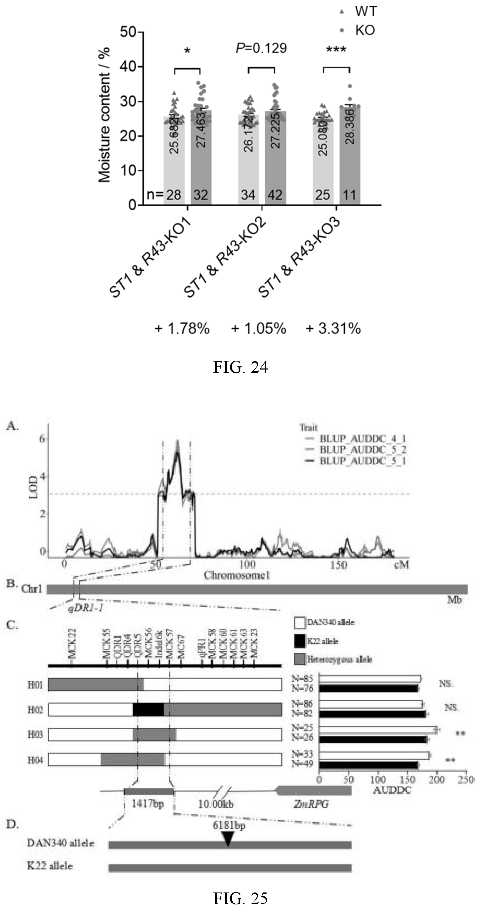

The results showed that 3 strains (designated as KO1, KO2, and KO3) were obtained after knocking out ZmMYBST1 and ZmMYBR43 genes using CRISPR-Cas9 technology. The field test results showed that compared with the non-knockout recipient control plants, the dehydration rate of maize kernels in KO1, KO2, and KO3 all significantly slowed down (FIG. 23). Water content at harvest was further evaluated, and it was found that the water content increased by 1.78% in KO1, the water content increased by 1.05% in KO2, and the water content increased by 3.31% in KO3 (FIG. 24). These results showed that the *ZmMYBST1* and *ZmMYBR43* genes and the proteins encoded thereby had the function of promoting the dehydration of maize kernels, and the inhibition or knockout of the two genes could reduce the dehydration rate of kernels and increase the kernel water content.

The analysis of the gene sequences of the edited ZmMYBST1 and ZmMYBR43 genes in KO1, KO2, and KO3 showed that the ZmMYBST1 and ZmMYBR43 genes in KO1 were mutated into (SEQ ID NO. 43 and SEQ ID NO. 44), respectively, the ZmMYBST1 and ZmMYBR43 genes in KO2 were mutated into (SEQ ID NO. 45 and SEQ ID NO. 46), respectively, and the ZmMYBST1 and ZmMYBR43 genes in KO3 were mutated into (SEQ ID NO. 47 and SEQ ID NO. 48), respectively. Introducing these mutant genes into other maize varieties by means of backcross breeding can improve the kernel dehydration and water content traits of the maize, thereby obtaining a maize variety with a slower dehydration rate and higher water content.

### Example 4. QTL Controlling Maize Kernel Dehydration Rate

### Example 4.1. Identification of QTL for Maize Kernel Water Content

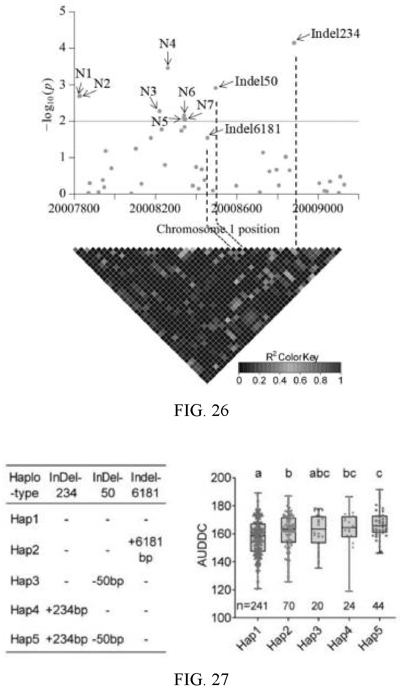

The QTL was mapped through several quarters of fine mapping to a 1417-bp segment on maize chromosome 1. This segment corresponded to Chromosome 1: 20,007,756-20,009,147 in the B73 V4 genome, and was a non-coding region between genes without any functional annotations. The results of sequence analysis of the segment in the near-isogenic line showed that NIL^{DAN340} had a length of 7592 bp (set forth in SEQ ID NO. 49), NIL^{K22} had a length of 1417 bp (set forth in SEQ ID NO. 50), and NIL^{DAN340} had an additional 6181-bp insertion fragment between positions 722-779 of SEQ ID NO. 50 as compared to NIL^{K22} (FIG. 25). Compared with NIL^{DAN340}, NIL^{K22} had a faster kernel dehydration rate, but there was substantially no difference between the two NILs in other agronomic traits (Table 3). This indicates that qKDR1 does not affect yield and other agronomic traits while controlling the dehydration rate. Further, 399 population materials derived from different genetic backgrounds were analyzed by sequencing and PCR detection methods, and 9 loci significantly associated with the dehydration rate of kernels were identified, which were located at positions Chr1: 20007822 (N1), Chr1: 20007825 (N2), Chr1: 20008218 (N3), Chr1: 20008258 (N4), Chr1: 20008336 (N5), Chr1: 20008338 (N6), Chr1: 20008344 (N7), Chr1: 20008466-20008515 (Indel50), and Chr1: 20008872-20009107 (Indel234), respectively (FIG. 26). The markers corresponded to position 67, position 70, position 473, position 513, position 597, position 599, position 605, positions 729-778, and positions 1131-1377 of the sequence set forth in SEQ ID NO. 50, respectively.

**Table 3. Agronomic and yield-related traits of NIL^{DAN340} and NIL^{K22}**

| **Trait** | **Experimental environment** | **NIL^{DAN340}** | | **NIL^{K22}** | | **P value** |
|---|---|---|---|---|---|---|
| | | **(Mean ± SD)** | **N** | **(Mean ± SD)** | **N** | |
| Silking stage | 18HN | 54.866±2.039 | 97 | 54.559±1.914 | 93 | 0.2867 |
| Tasseling stage | 18HN | 51.247±1.339 | 97 | 50.685±0.806 | 89 | 0.0007 |
| Anthesis | 18HN | 55.899±1.555 | 99 | 55.510±1.673 | 96 | 0.0945 |
| Plant height (cm) | 18HN | 211.065±8.096 | 94 | 212.957±6.718 | 90 | 0.0870 |
| Ear height (cm) | 18HN | 86.255±7.122 | 95 | 85.609±8.009 | 92 | 0.5604 |
| Leaf length (cm) | 18HN | 79.242±2.584 | 89 | 79.201±3.341 | 87 | 0.9281 |
| Leaf width (cm) | 18HN | 10.053±0.593 | 85 | 10.011±0.661 | 79 | 0.6720 |
| Ear length (cm) | 18HN | 14.027±0.692 | 62 | 13.936±0.754 | 63 | 0.4582 |
| Ear diameter (cm) | 18HN | 4.089±0.202 | 63 | 4.020±0.198 | 63 | 0.0534 |
| Number of ear rows | 18HN | 15.360±1.239 | 62 | 15.446±1.820 | 65 | 0.1968 |
| Number of kernels per row | 18HN | 24.054±4.366 | 63 | 24.021±4.085 | 64 | 0.9713 |
| Kernel length (mm) | 18HN | 10.352±0.437 | 60 | 10.323±0.452 | 62 | 0.7180 |
| Kernel width (mm) | 18HN | 8.246±0.426 | 63 | 8.271±0.365 | 61 | 0.7288 |
| Hundred-kernel weight (g) | 18HN | 20.909±2.378 | 60 | 21.376±2.373 | 63 | 0.2778 |

18HN represents the phenotypic experiment conducted in Hainan in 2018; N represents the number of samples; the P value is evaluated by the two-tailed T-test.

### Example 4.2. Cloning of Maize Kernel Water Content Gene

The 10 trait-associated loci described above can be used as detection targets to assist in the identification or screening of maize materials with different kernel water contents or dehydration rates.

In this example, the loci located at Chr1: 20008493 and Chr1: 20008878 (designated as Indel50 and Indel234, respectively) and the insertion fragment of NIL^{DAN340} relative to NIL^{K22} (designated as Indel6181) were selected as screening markers to design a detection method.

The primer pair MCK57_3F: GTGGTGCATGCATTGTTCAT (SEQ ID NO. 101) and MCK57_R: GTCTACAGCCACGAACACGA (SEQ ID NO. 92) was designed to detect the Indel6181 marker. With the NIL^{K22} genotype as a reference, genotypes with slow dehydration were screened out by detecting a band with a 6181-bp insertion, and genotypes with fast dehydration were screened out by detecting a band without the insertion. The primer pair QDR1_F: TCACACGGGACAATCTGTAG (SEQ ID NO. 106) and QDR1_R: AGGAAGCTTGTGCGAATTTG (SEQ ID NO. 107) was designed to detect the Indel50 marker. With the NIL^{K22} genotype as a reference, genotypes with slow dehydration were screened out by detecting a band with a 50-bp deletion (corresponding to bases at positions 729-778 of the sequence set forth in SEQ ID NO. 50), and genotypes with fast dehydration were screened out by detecting a band without the deletion. The primer pair MCK57_7F: CGCACTGCAGCTTCGTAGTC (SEQ ID NO. 93) and MCK57_R:
GTCTACAGCCACGAACACGA (SEQ ID NO. 92) was designed to detect the Indel234 marker. With the NIL^{K22} genotype as a reference, genotypes with slow dehydration were screened out by detecting a band with a 234-bp insertion, and genotypes with fast dehydration were screened out by detecting a band without the insertion. The sequence
"GATATATATAGCCGCGCGCAATACGCACATGCATCATGCATGGGGGACAGGTTGCTCT TATGATCTATCGGCAAGCTGGAGATGCTAGCTGCTGCTGCGTCCATGCGTTGTTCTTCT TTAGCCGTCAGGCATGGACCTGACTGGAACCAACCGAATTCCTGAGTTTCTTCTCTTTT TTTCCCCCTCGCGTACCTGCGCTGCGCTGCGCAGGGCATGTGACGCAGCCACATCTCGA ACTTAACGAGAG" (SEQ ID NO. 57) corresponding to Indel234 at positions 1131-1377 of the sequence set forth in SEQ ID NO. 50 was replaced with the sequence
"CATGGACCTGACTGGAAACCAACCGAATTCCTGAGTTTCTTCTCTTTTTTCCCCCTTCG CGTACGTACCTGCGCTGCGCAGGGCATGTGAGGCTACCCGCAGTGATATACGGTAAAG AAGACGCCACCAATATAAGGCTAGGTATAGAGTAAAATTTTACTGCAGTGGAGTACTC TATAGGTAGCTGTAAACTTATGGGGGGCAAACAAAACGCTATTTACAGCGTCGATGCT TTTTTACTCTATCAGTCCACACGTCCATGCAGCAGGTACGGTGCCGAGCGCCATCAGCC GGTGTGGCAGTAGGGGACAAAAAAAGATATATAATATATGTAGTGATTGATATAGAGT TGATGTATGGAGTAAATATGACTGCAGTAGATTATGATATAGGGTAGAGAATCTTGAT GATGAGGATAGAATATTTCTTTTAGAGTAGAAATTTAGAGTAGAATGACTGCAGATAG CCTGACGCAGCCCAT" (SEQ ID NO. 58, which was 234 bp longer than the original sequence SEQ ID NO. 57). The remaining 7 marker loci can also be used as screening markers by designing primers according to the related sequences of the genes. The combination of the three markers, Indel6181, Indel50, and Indel234, was used to screen for genotypes with different dehydration rates. The associated populations could be divided into 5 haplotypes. Among them, Hap1 was similar to the NIL^{K22} genotype and dehydrated the fastest; Hap2 was similar to the NIL^{DAN340} genotype and dehydrated relatively slowly; the Hap5 genotype dehydrated the slowest (FIG. 27).

The specific molecular marker detection procedures follow conventional steps; for example, reference could be made to the content of Example 3 in CN114989281A.

### Example 4.3. Editing qKDR1 Segment to Regulate Kernel Dehydration Rate

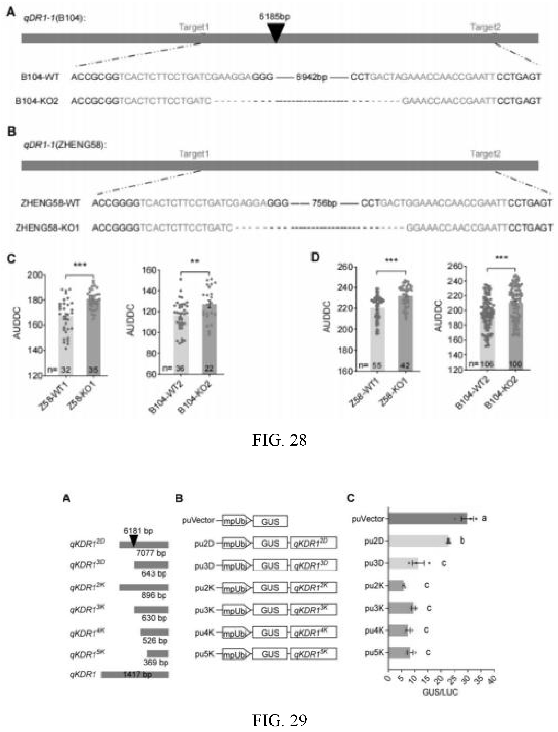

The present disclosure further tried to use CRISPR-Cas9 technology to knock out the qKDR1 segment in the maize inbred lines Zheng 58 and B104, so as to detect the technical effects of the technical solutions. The qKDR1 genotypes in Zheng 58 and B104 were the same as those in K22 and DAN340, respectively. The designed target positions and sequences are shown in FIG. 28.

In this example, gene editing produced two mutants, Z58-KO1 and B104-KO2, in Zheng 58 and B104. Compared with wild-type plants, both Z58-KO1 and B104-KO2 knockout lines exhibited slower dehydration rates (FIG. 28), indicating that the dehydration rate of maize kernels can be altered by manipulating qKDR1. In addition, the knockout of qKDR1 only slightly affected individual agronomic traits (Table 4), leaving most agronomic traits unaffected. Therefore, the dehydration rate of maize kernels can be improved by manipulating qKDR1, thereby constructing maize germplasms with different water contents and dehydration rates.

**Table 4. Agronomic and yield-related traits under qKDR1 knockout**

| Trait | Experimental environment | B104-WT2 | | B104-KO2 | | P value |
|---|---|---|---|---|---|---|
| | | (Mean ± SD) | N | (Mean ± SD) | N | |
| Tasseling stage | 22BJ | 57.194±1.211 | 108 | 56.894±1.102 | 94 | 0.0678 |
| Anthesis | 22BJ | 59.410±1.111 | 100 | 59.355±1.248 | 93 | 0.7457 |
| Plant height (cm) | 22BJ | 173.133±8.622 | 104 | 174.141±11.721 | 95 | 0.4877 |
| Ear height (cm) | 22BJ | 52.458±5.603 | 100 | 53.333±6.463 | 91 | 0.3177 |
| Leaf length (cm) | 22BJ | 68.256±5.514 | 105 | 67.695±6.982 | 95 | 0.5268 |
| Leaf width (cm) | 22BJ | 7.471±0.566 | 104 | 7.154±0.748 | 96 | 0.0008 |
| Tasseling stage | 22JL | 79.754±1.387 | 199 | 80.031±1.715 | 196 | 0.0783 |
| Anthesis | 22JL | 83.665±0.896 | 197 | 83.948±1.117 | 193 | 0.1345 |
| Plant height (cm) | 22JL | 185.686±7.327 | 140 | 187.059±7.869 | 136 | 0.0645 |
| Ear height (cm) | 22JL | 60.542±7.079 | 144 | 62.058±6.645 | 139 | 0.0645 |
| Leaf length (cm) | 22JL | 81.615±3.598 | 144 | 81.161±3.404 | 137 | 0.2787 |
| Leaf width (cm) | 22JL | 8.939±0.554 | 141 | 8.746±0.578 | 136 | 0.0012 |
| Ear length (cm) | 22JL | 13.314±1.176 | 66 | 12.661±1.287 | 67 | 0.0059 |
| Ear diameter (cm) | 22JL | 3.945±0.176 | 67 | 3.946±0.194 | 65 | 0.9669 |
| Number of ear rows | 22JL | 14.314±1.389 | 70 | 14.635±1.383 | 63 | 0.1852 |
| Number of kernels per row | 22JL | 24.652±3.776 | 66 | 22.831±3.375 | 65 | 0.0043 |
| Kernel length (mm) | 22JL | 9.536±0.340 | 78 | 9.705±0.282 | 71 | 0.0013 |
| Kernel width (mm) | 22JL | 7.027±0.261 | 80 | 6.934±0.229 | 72 | 0.0214 |
| Hundred-kernel weight (g) | 22JL | 19.029±1.847 | 78 | 19.142±1.568 | 72 | 0.6855 |

22BJ and 22JL represent the phenotypic experiments conducted in Beijing in 2022 and in Jilin in 2022, respectively; the P value is evaluated by the two-tailed T-test.

Further, in the present disclosure, qKDR1 was truncated into different fragments, which were ligated to the downstream of the GUS gene via a dual-reporter gene vector. The fragments of NIL^{DAN340} were qKDR1^{2D} and qKDR1^{3D}, and the fragments of NIL^{K22} were qKDR1^{2K}, qKDR1^{3K}, qKDR1^{4K}, and qKDR1^{5K} (FIG. 29). After ligation to the vector, the expression level of the reporter gene was detected, and it was found that qKDR1^{3D}, qKDR1^{2K}, qKDR1^{3K}, qKDR1^{4K}, and qKDR1^{5K} all could inhibit the expression of the GUS gene (FIG. 29), indicating that the core region for qKDR1 to exert its function is qKDR1^{5K}, i.e., a 369-bp region located at positions 1049-1417 of the sequence set forth in SEQ ID NO. 50. Therefore, deleting fragments between positions 1049-1417 can achieve the technical effects of reducing the dehydration rate and increasing the kernel water content.

By combining the examples described above, an interaction network of molecular elements that controls the maize kernel dehydration rate and the kernel water content can be obtained, as shown in FIG. 30.

Although the present disclosure has been described in detail herein above through general explanations and specific embodiments, it will be apparent to those skilled in the art that modifications or improvements can be made on the basis of the present disclosure. Accordingly, such modifications or improvements made without departing from the spirit of the present disclosure all fall within the protection scope of the present disclosure.

### SEQUENCE LISTING

### Example 1

> SEQ ID NO. 1 NILDAN3402010bp sequence of ZmRPG 
AGAACACAACCAAAGCATCTAGTTTCGTCCCTTCCTGCCTACAGCGTCGTGCGTATGAT
ACTTCTTCCTAATCATTCGCGTGCACCGACAAACAAGAGAGCAGGTCTCCAAAACTATT
GCCCCCAGAGATCTTGTATCAGGAGAGGGTAAATAAAGTTTTTGGGAAGCACTCTACA
TGGATGCTCGATCACTTCATTGCCGTCTTCAATCTCATTGCCATGGCTCATCTGTCGCCT
TGTCACGTGGACGTTTCATGTCGTCGGTAACAATGAGATGGTGTTAGGCGTCAAGTTGT
CTTCTCTACCCGATCGTCGGCAAGGAACAAACGTATAACTATCTCCCTTTCATCATGTA
TTAAATATTGTTTAATTTAATTTGTTTAGATCAGTGCAAGTTTTAATCTTAGTTATCTTT
GTAAAGACAATTCATTATGTAATAAATTTGTGCCATTGGATTCTATAAACTGAGTCATT
ATATGTGTGAAATCTAATTTTGACACACATATGAGATACATCTGGATTTTTCCTTAAAT
TCGTGTGTGACAATAGGACTGACAATTCTAGATCACTTCAACCGCAATAACAACTCAA
AGGATGTTTAACTCAATACTTTTGAAGGTTCATTCAAATCAAAGCGCAAGTACCCGATG
TACAAGATGACGTGGCCATCATAGCAACAATCAAAAGGTTTCATATGGGACAAATGCA
CATCACACTTTGCTCGAGAACCAACAAGCAACAATATAAGAACTTTATGAAGTCATGC
AAAAACTTCTGCAAGTCAAACATGGACCATAGAAAAATAGTCGAAGAAGAAGCTAAC
TTCTAAACTCAAGCCAAAAGTGGTAACAATTACAACCAATCCAAAACTAGCAACTTTG
AGTGAAGGCCTCTACCTTCGTATCACCAACTAAACCAAATAGACAATCAACTACTGGC
GAGAGCAACCACTAAGAGAGAACTCATTATAGTGAAACAACATTCAATAGAGGAGGG
ACAAACCACAAAGGACAGAGAGGAGGTAGAATTGGTGGGGGTGAAAGGGAAATGTG
CCTTTGGGCCATTTCTAAGTATTTTGGTGATTTAGTGTCCAACACAAGTGCCTAAGTGT
TGATCTATGCAAAGTGGTGGACAAAGAGCAAATCAAGTCAAAAGGTATGTTTCTAGAC
TTAGTACATTGTTTTATGGACTGATGTATTGTGTCTAAGTGCTGGAAACAGGAGAAATC
AAATTGAAAAAGAGATGGCTTTATTCAGCCAAAGTCAGCTCCGTCTGGGTGCACCGGA
CTGTCCGGTGGCGCACCGGACAGTGTCCGGTGCGCCAGGCTGGCTCAGGCGAACTTGC
TGCTCTCGGGAAGTAATTAACGGCGTACGGCTAAAATTCACCGGACTGTCCGGTATGC
ACCAGACTGTCCGGTGAGCCAACGGTCGGCCGCGCGATCCGCGCGAGACACGTGGCCG
AGCCAACGGTCAGAAGGGGGCACCGGACTGTCCGGTGTGCACCGGACAGTGTCCGGTG
CGCCAACGGCTCCAAAGTGCCAACGGTCGGCTTCGCTAAAGAAGGAAAGAAATCCGC
ACCGGACAGTGTCCGGTGGCGCACTGGACTGTCCGGTGCGCCAGGCGACAGAAGGCA
AGAATTGCCTTCCTGGAATGCTCTCAACGGCTCCTAGCTGCCTTGGGGCTATAAAGGG
ACCCCTAGGCGCATGGAAGAAAGCACCAAGCAACCTTTGAGCATTGTTGATCATTCAC
 
ACTTCACTCTTGCGCACTAATTCGACATTCTTAGTGATTTGAGCTCTGTTCTAGTGAGA
ACCTTGAGATATTGTTTTGAGCTCAAGTTTTGATCGTGTGCGCGTATTTGTTGTGGTT
TTGAGTGTGTTGCTTCCCTCCCTTACTCTTGTGCTTCATATTGATTCTTATTGTAAGGGC
GAGAGACTCCAAGTTGTGGAGATTCCTCGCAAACGGAAAAGAGCAAAGTAAAAAGA
ACACTGATGAGGACATCCTCCACTA
> SEQ ID NO. 2 NILK222013bp sequence of ZmRPG 
AGAACACAACCAAAGCATCTAGTTTCGTCCCTGCTTCCTGCCTACAGCGTCGTGCGTAT
GATACTTCTTCCTAATCATTCGCGTGCACCGACAAACAAGAGAGCAGGTCTCCAAAAC
TATTGCCCCCAGAGATCTTGTATCAGGAGAGGGTAAATAAAGTTTTTGGGAAGCACTC
TACATGGATGCTCGATCACTTCATTGCCGTCTTCAATCTCATTGCCATGGCTCATCTGTC
GCCTTGTCACGTGGACGTTTCATGTCGTCGGTAACAATGAGATGGTGTTAGGCGTCAAG
TTGTCTTCTCTACCCGATCGTCGGCAAGGAACAAACGTATAACTATCTCCCTTTCATCA
TGTATTAAATATTGTTTAATTTAATTTGTTTAGATCAGTGCAAGTTTTAATCTTAGTTAT
CTTTGTAAAGACAATTCATTATGTAATAAATTTGTGCCATTGGATTCTATAAACTGAGT
CATTATATGTGTGAAATCTAATTTTGACACACATATGAGATACATCTGGATTTTTCCTT
AAATTCGTGTGTGACAATAGGACTGACAATTCTAGATCACTTCAACCGCAATAACAAC
TCAAAGGATGTTTAACTCAATACTTTTGAAGGTTCATTCAAATCAAAGCGCAAGTACCC
GATGTACAAGATGACGTGGCCATCATAGCAACAATCAAAAGGTTTCATATGGACAAA
TGCACATCACACTTTGCTCGAGAACCAACAAGCAACAATATAAGAACTTTATGAAGTC
ATGCAAAAACTTCTGCAAGTCAAACATGGACCATAGAAAAATAGTCGAAGAAGAAGC
TAACTTCTAAACTCAAGCCAAAAGTGGTAACAATTACAACCAATCCAAAACTAGCAAC
TTTGAGTGAAGGCCTCTACCTTCGTATCACCAACTAAACCAAATAGACAATCAACTACT
GGCGAGAGCAACCACTAAGAGAGAACTCATTATAGTGAAACAACATTCAATAGAGGA
GGGACAAACCACAAAGGACAGAGAGGAGGTAGAATTGGTGGGGGTGAAAGGAAA
TGTGCCTTTGGGCCATTTCTAAGTATTTTGGTGATTTAGTGTCCAACACAAGTGCCTAA
GTGTTGATCTATGCAAAGTGGTGGACAAAGAGCAAATCAAGTCAAAAGGTATGTTTCT
AGACTTAGTACATTGTTTTATGGACTGATGTATTGTGTCTAAGTGCTGGAAACAGGAGA
AATCAAATTGAAAAAGAGATGGCTTTATTCAGCCAAAGTCAGCTCTGTCTGGGTGCAC
CGGACTGTCCGGTGGCGCACCGGACAGTGTCCGGTGCGCCAGGCTGGCTCAGGCGAAC
TTGCTGCTCTCGGGAAGTAATTAACGGCGTACGGCTAAAATTCACCGGACTGTCCGGT
ATGCACCGGACTGTCCGGTGAGCCAACGGTCGGCCGCGCGATCCGCGCGAGACACGTG
GCCGAGCCAACGGTCAGAAGGGGGCACCGGACTGTCCGGTGTGCACCGGACAGTGTCC
 
GGTGCGCCAACGGCTCCAAAGTGCCAACGGTCGGCTTCGCTAAAGAAGGAAAGAAAT
CCGCACCGGACAGTGTCCGGTGGCGCACTGGACTGTCCGGTGCGCCAGGCGACAGAAG
GCAAGAATTGCCTTCCTGGAATGCTCTCAACGGCTCCTAGCTGCCTTGGGGCTATAAAA
GGGACCCCTAGGCGCATGGAAGAAAGCACCAAGCAACCTTTGAGCATTGTTGATCATT
CACACTTCACTCTTGCGCACTAATTCGACATTCTTAGTGATTTGAGCTCTGTTCTAGTGA
GAACCTTGAGATATTGTTTTGAGCTCAAGTTTTGATCGTGTGTGCGCGTATTTGCTGTG
GTTTTGAGTGTGTTGCTTCCCTCCCTTACTCTTGTGCTTCATATTGATTCTTATTGTAAG
GGCGAGAGACTCCAAGTTGTGGAGATTCCTCGCAAACGGGAAAAGAGCAAAGTAAAA
AGAACACTGATGAGGACATCCTCCACTA
> SEQ ID NO. 3 96bp sequence of microRPG1 
ATGATACTTCTTCCTAATCATTCGCGTGCACCGACAAACAAGAGAGCAGGTCTCCAAA
ACTATTGCCCCCAGAGATCTTGTATCAGGAGAGGGTAA
> SEQ ID NO. 4 Protein sequence of microRPG1
   MILLPNHSRAPTNKRAGLQNYCPQRSCIRRG
> SEQ ID NO. 5 Target
   AATCATTCGCGTGCACCGAC
> SEQ ID NO. 6 gRNA 
GUCGGUGCACGCGAAUGAUUguuuuagagcuagaaauagcaaguuaaaauaaggcuaguccguuaucaacuugaa
aaaguggcaccgagucggugcuuuuuuu
>SEQ ID NO. 7 KO-1bp 
ATGATACTTCTTCCTAATATTCGCGTGCACCGACAAACAAGAGAGCAGGTCTCCAAAA
CTATTGCCCCCAGAGATCTTGTATCAGGAGAGGGTAA
>SEQ ID NO. 8 KO-2bp 
ATGATACTTCTTCCTAATTTCGCGTGCACCGACAAACAAGAGAGCAGGTCTCCAAAAC
TATTGCCCCCAGAGATCTTGTATCAGGAGAGGGTAA
>SEQ ID NO. 9 KO-4bp ATGATACTTCTTCCTAATCGCGTGCACCGACAAACAAGAGAGCAGGTCTCCAAAACTA TTGCCCCCAGAGATCTTGTATCAGGAGAGGGTAA
> SEQ ID NO. 10 Strong promoter ubiquitin sequence 
CTGCAGTGCAGCGTGACCCGGTCGTGCCCCTCTCTAGAGATAATGAGCATTGCATGTCT
AAGTTATAAAAAATTACCACATATTTTTTTGTCACACTTGTTTGAAGTGCAGTTTATCT
ATCTTTATACATATATTTAAACTTTACTCTACGAATAATATAATCTATAGTACTACAAT
AATATCAGTGTTTTAGAGAATCATATAAATGAACAGTTAGACATGGTCTAAAGGACAA
TTGAGTATTTTGACAACAGGACTCTACAGTTTTATCTTTTTAGTGTGCATGTGTTCTCCT
TTTTTTTTGCAAATAGCTTCACCTATATAATACTTCATCCATTTTATTAGTACATCCATT
TAGGGTTTAGGGTTAATGGTTTTTATAGACTAATTTTTTTAGTACATCTATTTTATTCTA
TTTTAGCCTCTAAATTAAGAAAACTAAAACTCTATTTTAGTTTTTTTATTTAATAATTTA
GATATAAAATAGAATAAAATAAAGTGACTAAAAATTAAACAAATACCCTTTAAGAAAT
TAAAAAAACTAAGGAAACATTTTTCTTGTTTCGAGTAGATAATGCCAGCCTGTTAAACG
CCGTCGACGAGTCTAACGGACACCAACCAGCGAACCAGCAGCGTCGCGTCGGGCCAA
GCGAAGCAGACGGCACGGCATCTCTGTCGCTGCCTCTGGACCCCTCTCGAGAGTTCCG
CTCCACCGTTGGACTTGCTCCGCTGTCGGCATCCAGAAATTGCGTGGCGGAGCGGCAG
ACGTGAGCCGGCACGGCAGGCGGCCTCCTCCTCCTCTCACGGCACCGGCAGCTACGGG
GGATTCCTTTCCCACCGCTCCTTCGCTTTCCCTTCCTCGCCCGCCGTAATAAATAGACAC
CCCCTCCACACCCTCTTTCCCCAACCTCGTGTTGTTCGGAGCGCACACACACACAACCA
GATCTCCCCCAAATCCACCCGTCGGCACCTCCGCTTCAAGGTACGCCGCTCGTCCTCCC
CCCCCCCCTCTCTACCTTCTCTAGATCGGCGTTCCGGTCCATGGTTAGGGCCCGGTAGT
TCTACTTCTGTTCATGTTTGTGTTAGATCCGTGTTTGTGTTAGATCCGTGCTGCTAGCGT
TCGTACACGGATGCGACCTGTACGTCAGACACGTTCTGATTGCTAACTTGCCAGTGTTT
CTCTTTGGGGAATCCTGGGATGGCTCTAGCCGTTCCGCAGACGGGATCGATTTCATGAT
TTTTTTTGTTTCGTTGCATAGGGTTTGGTTTGCCCTTTTCCTTTATTTCAATATATGCCGT
GCACTTGTTTGTCGGGTCATCTTTTCATGCTTTTTTTTGTCTTGGTTGTGATGATGTGGTC
TGGTTGGGCGGTCGTTCTAGATCGGAGTAGAATTCTGTTTCAAACTACCTGGTGGATTT
ATTAATTTTGGATCTGTATGTGTGTGCCATACATATTCATAGTTACGAATTGAAGATGA
TGGATGGAAATATCGATCTAGGATAGGTATACATGTTGATGCGGGTTTTACTGATGCAT
ATACAGAGATGCTTTTTGTTCGCTTGGTTGTGTTGATGTGGTGTGGTTGGGCGGTCGTT
CATTCGTTCTAGATCGGAGTAGAATACTGTTTCAAACTACCTGGTGTATTTATTAATTTT
GGAACTGTATGTGTGTGTCATACATCTTCATAGTTACGAGTTTAAGATGGATGGAAATA
TCGATCTAGGATAGGTATACATGTTGATGTGGGTTTTACTGATGCATATACATGATGGC
ATATGCAGCATCTATTCATATGCTCTAACCTTGAGTACCTATCTATTATAATAAACAAG
 
TATGTTTATAATTATTTGATCTTGATATACTTGGATGATGGCATATGCAGCAGCTATA
TGTGGATTTTTTAGCCCTGCCTTCATACGCTATTTATTTGCTTGGTACTGTTTCTTTTGT
CGATGCTCACCCTGTTGTTTGGTGTTACTTCTGCAG
> SEQ ID NO. 11 Homologous sequence of microRPG1 in *Zea luxurians*-RIL003
   MVLLPDHLCAPTNKRAGLQNYCPQRSCISRG
> SEQ ID NO. 12 Homologous sequence of microRPG1 in *Zea nicaraguensis*-PI615697
   MILLPDHPRAPTNKRAGCPQRSCIRRR
> SEQ ID NO. 13 Homologous sequence of microRPG1 in *Zea diploperennis*-Gigi
   MILLPDHPRAPTNKRAGLQNYCP
> SEQ ID NO. 14 Homologous sequence of microRPG1 in *Zea diploperennis*-Momo
   MILPDHPCAPTNKRAGLQNYCP
> SEQ ID NO. 15 Homologous sequence of microRPG1 in *Zea perennis*
   MILLPNHPRAPTNKRAGLQNYCP
> SEQ ID NO. 16 Homologous sequence of microRPG1 in *Zea mays subspecies huehuetenangensis-*RIMHU001
   MILLPNHPRAPTNKRASLQNYCPQRSCIRRG
> SEQ ID NO. 17 Homologous sequence of microRPG1 in *Zea mays subspecies parviglumis*
   MILLPNHPRAPTNKRASLQNYCPQRSCIRRG
> SEQ ID NO. 18 Homologous sequence of microRPG1 in *Zea mays subspecies mexicana*-TIL25
   MILLPNHSRAPTNKRAGLQNYCPQRSCIRRG

### Example 2

> SEQ ID NO. 19 ZmEIL1 sequence ATGATGGGAGGCGGGCTGTTGGTGGATCAGAGCGTGGTGTTCCCTGGCGTCCACAACT 
TCGTGGATCTCCTGCAGCAGAACGGCGACAAGAACCTGGGCTTCGGGTCTCTGATGCC
GCAGACGTCCTCTGGCGACCAGTGCGTGATGGGGAGGGCGATCTCGTGGACCCGCCT
CCGGATAGCTTCCCGGACGCCGGGGAGGACGACAGCGACGATGACGTCGAGGACATC
GAGGAGCTGGAGCGCCGCATGTGGCGCGACCGTATGAAGCTGAAGCGGCTCAGGGAA
CTGCAGCAGACCCGCGGCAAGGACTCGTTGGCTAGCGGTGCGGACTGGCTGATGGCT
CGTCCAAGCCAAGGCAGTCGCAGGAGCAGGCCCGGCGCAAGAAGATGTCGCGCGCGC
AGGACGGCATCCTCAAGTACATGCTCAAGATGATGGAGGTGTGCCGCGCGCAGGGGTT
TGTGTATGGGATCATTCCGGAGAAGGGCAAGCCAGTGAGTGGTGCCTCCGACAACCTC
CGTGCGTGGTGGAAGGAGAAGGTCCGCTTCGACCGCAACGGACCGGCCGCCATTGCCA
AGTACCAGGCCGACAACGCCGTCCCTGGCGCCGAGAACGAGCTCGCCTCGGGCGCTGC
CAGCCCCCATTCCTTGCAGGAGCTGCAGGACACTACGCTGGGCTCACTGCTCTCAGCGC
TTATGCAGCACTGCGAGCCCCCACAGCGGCGCTACCCGCTCGAGAAGGGCGTTCCTCC
ACCGTGGTGGCCTACCGGCGACGAGGAGTGGTGGCCGGAACTCGGCATTCCCAAGGAC
CAGGGCCCACCCCCGTACAAGAAGCCTCATGACCTTAAGAAGGCCTGGAAGGTGAGCG
TGCTCACCGCTGTCATCAAGCACATGTCACCGGACATAGAGAAGATCCGTCGCCTGGT
TCGCCAGTCCAAGTGCCTCCAGGACAAGATGACTGCCAAGGAGATCTCAACCTGGCTG
GCGGTCGTCAAGCAGGAAGAGGAGCTGTACCAGAAGCTGAACCCGGGCGCACGCCCA
CCGGCGTCTACTGGTGGCATCGCAAGTGCCATATCCTTCAACACCAGCTCGAGCGAGT
ATGATGTGGACATCATCGATGAGTGCAAGGGGGACGAGGCCGGTAACCAGAGGACGG
CAGTCACTGACCCAACCGCGTTCAACCTTGGTGCCGCTATCCTAAGCGACAAGTTCCTC
GTGCCGACGCCGATGAAGGAGGAGACCGCCGACGTGGAGTTCATCCAGAAGAGGAAC
GCCCCCGCTGCAGCCGAGCCAGAGCTGATGCTAAACAACCGATTGTACACCTGCAACA
ACGTCCAGTGCCCGCGCAGTGACTACAGCTACGGATTCCTGGACCGGAATGCCCGCAA
CAGCCACCAGTACACCTGCAAGCACAAGGATCCAACCCCTCAGAGCACCGAGAACAA
GCCGCCGTCAGCACCGCCACAGCCACAAGCCTTCCAGCCGGCCTTCAGCCAACCCAAC
CAGGCACTGAACAGTCTGGATTTCAGCCTGCCCATGGACGGGCAGAGGTCCATCGCCG
AGCTGATGAACATGTACGACAACAACTTCGTGCCGAACAAGAACCCGAGCAGCGACA
GCGTCGCCGTCATGGAGAGGCCAAACGCGATGCCCCAGCAGAGGATCCAGATGGACG
AGGGTTTCTTCGTACAGGGCAACGGAGCCTTCGACGACGTCAACAACAGCATGATGCA
GCAGCAGCAGCAGCAGGCGCCAGTGCAGCAGCAGCAGCAGTTCTTCATCCGCGACGAC
ACGCCATTCGTGAGCCAGATGGGCGACATCGCCGCCAGCGCGCCGGAGTTCAGGTTCG
GTCCTGGTTTCAACATGTCTAGCGGCGTCGACTACCCAGGCGCGGCACAGAGGAACGA
CGGGACCAATTGGTTCTACTGA
SEQ ID NO. 20 ZmEIL3 sequence 
ATGATGGAGGCGGGCTGATGATGGATCAGAGCGTGGTGTTCCCTGGCGTCCACAACT
TCGTGGATCTCCTGCAGCAGAACGGCGACAAGAACCTGGGCTTCGCCTCACTCATGCC
GCAGACGTCCTCCGGCGACCAGTGCGTGATGGGGGAGGGTGATCTCGTGGACCCACCG
CCAGACAGCTTCCCGGACGCCGTGGAGGACGACAGCGATGACGACGTTGAGGACATC
GAGGAGCTGGAGCGCCGCATGTGGCGCGACCGCATGAAGCTGAAGCGGCTCAGGGAG
CTGCAGCAGAGCCGCGGCAAGGATCCCATGGCTAGCGGTGGGGGCCTGGCCGACGGCT
CATCCAAGCCAAGGCAGTCGCAGGAGCAGGCCCGGCGCAAGAAGATGTCGCGCGCGC
AGGACGGCATCCTCAAGTACATGCTCAAGATGATGGAGGTGTGCCGCGCGCAGGGGTT
TGTGTACGGGATCATTCCGGAGAAGGGCAAGCCGGTGAGCGGCGCCTCCGACAACCTC
CGTGCCTGGTGGAAGGAGAAGGTCCGCTTCGACCGCAACGGCCCGGCCGCCATCGCCA
AGTATCAGGCCGACAACGCCGTCCCGGGCGCCGAGAATGAGCTCGCCTCGGGCGCTGC
CAGCCCTCATTCCTTGCAGGAGCTGCAGGACACCACACTCGGCTCGCTGCTCTCAGCAC
TCATGCAGCACTGCGACCCCCCACAGCGGCGCTACCCGCTGGAGAAGGGCGTTCCTCC
ACCGTGGTGGCCTACCGGCGACGAGGAGTGGTGGCCGGAGCTTGGCATTCCCAAGGAC
CAGGGCCCACCCCCCTACAAGAAGCCCCATGACCTTAAGAAGGCCTGGAAGGTGAGCG
TGCTCACCGCTGTCATCAAGCACATGTCACCAGACATAGAGAAGATCCGACGCCTCGT
TCGCCAGTCCAAGTGCCTCCAGGACAAGATGACTGCCAAGGAGATCTCGACCTGGCTG
GCGGTCGTCAAGCAGGAAGAGGAGCTGTACCTGAAGATGCACCCTGGCGCACGCCCGC
CGGCATCCACTGGTGGCATCGCCAGTGCCATATCTTTCAACACCACCTCAAGCGAGTAC
GACGTGGACATCGTTGATGAGTGCAAGGGGGATGAGGCTGGCAACCAGAAGACAGCA
GTCACTGACCCAACCTCCTTCAACCTTGGTGCGGCTATCCTAAGTGACAAGTTCCTCAT
GCCGACGCCGATGAAGGAGGAGACCGCTGACGTCGAGTTCATCCAGAAGAGGAACGC
CCCCGCTCCTGCTGAGCCAGAGCTAATGCTAAACAACCGGGTGTACACCTGCAACAAC
GTCCAGTGCCCGCACAGTGACTACAGCTATGGATTCCTTGACCGGAATGCCCGCAACA
GCCACCAGTACACCTGTAAGTACAACGATCCGATCACTCAGAGCGCCGAGAACAAGCC
TCTGCCAGCACCGCCGCAACCACAAGCCTTCCAGCCGGCCTTCAGCCAAGCCAATCAG
GCAGCGAACAATCTGGACTTCAGCCTGCCTATGGACGGGCAGAGGTCCATCGCCGAGC
TGATGAACATGTACGACACCAACTTCATGACGAGCAAGACCATGAGCAGTAGTGACAG
CGTCACCATCATGGAGAGGCCGAACGCGATGCCCCAGAGGATCCAGATGGACGAGGG
CTTCTTCGGACAGGGCAACGGAGTCTTCGACGACGTCAATAGCATGATGCAGCAACAA
CAGCAGCAGGCACCACCCGTGGTGCAGCAGCAGCAGCAGCAGTTCTTCATCCGCG
ACGACACGCCATTCATGAGCCAGATGGGCGACATCACCAGCACGGCGGAGTTCAGGTT
CGGCTCTGGTTTCAACATGTCTAGCACCGCCGCCGCCGCTGATTACCCAGGCGCGGCGC
 AGAAGAACGACGGACCAATTGGTTCTACTGA
>SEQ ID NO.21 ZmEIL1 gRNA1 
UGGACUGGCGAACCAGGCGAguuuuagagcuagaaauagcaaguuaaaauaaggcuaguccguuaucaacuugaa
aaaguggcaccgagucggugcuuuuuuu
>SEQ ID NO.22 ZmEIL1 gRNA2 
CCGUAGCUGUAGUCACUGCGguuuuagagcuagaaauagcaaguuaaaauaaggcuaguccguuaucaacuugaa
aaaguggcaccgagucggugcuuuuuuu
>SEQ ID NO.23 ZmEIL3 gRNA1 
GAGCUGCAGGACACCACACUguuuuagagcuagaaauagcaaguuaaaauaaggcuaguccguuaucaacuugaa
aaaguggcaccgagucggugcuuuuuuu
>SEQ ID NO.24 ZmEIL3 gRNA2 
UCAUGGGGCUUCUUGUAGGguuuuagagcuagaaauagcaaguuaaaauaaggcuaguccguuaucaacuuga
aaaaguggcaccgagucggugcuuuuuuu
>SEQ ID NO.25 ZmEIL1 KO1 
ATGATGGAGGCGGCTGTTGGTGGATCAGAGCGTGGTGTTCCCTGGCGTCCACAACT
TCGTGGATCTCCTGCAGCAGAACGGCGACAAGAACCTGGGCTTCGGGTCTCTGATGCC
GCAGACGTCCTCTGGCGACCAGTGCGTGATGGGGGAGGGCGATCTCGTGGACCCGCCT
CCGGATAGCTTCCCGGACGCCGGGGAGGACGACAGCGACGATGACGTCGAGGACATC
GAGGAGCTGGAGCGCCGCATGTGGCGCGACCGTATGAAGCTGAAGCGGCTCAGGGAA
CTGCAGCAGACCCGCGGCAAGGACTCGTTGGCTAGCGGTGCGGACTGGCTGATGGCT
CGTCCAAGCCAAGGCAGTCGCAGGAGCAGGCCCGGCGCAAGAAGATGTCGCGCGCGC
AGGACGGCATCCTCAAGTACATGCTCAAGATGATGGAGGTGTGCCGCGCGCAGGGGTT
TGTGTATGGGATCATTCCGGAGAAGGGCAAGCCAGTGAGTGGTGCCTCCGACAACCTC
CGTGCGTGGTGGAAGGAGAAGGTCCGCTTCGACCGCAACGGACCGGCCGCCATTGCCA
AGTACCAGGCCGACAACGCCGTCCCTGGCGCCGAGAACGAGCTCGCCTCGGGCGCTGC
CAGCCCCCATTCCTTGCAGGAGCTGCAGGACACTACGCTGGGCTCACTGCTCTCAGCGC
TTATGCAGCACTGCGAGCCCCCACAGCGGCGCTACCCGCTCGAGAAGGGCGTTCCTCC
ACCGTGGTGGCCTACCGGCGACGAGGAGTGGTGGCCGGAACTCGGCATTCCCAAGGAC
CAGGGCCCACCCCCGTACAAGAAGCCTCATGACCTTAAGAAGGCCTGGAAGGTGAGCG
 
TGCTCACCGCTGTCATCAAGCACATGTCACCGGACATAGAGAAGATCCGTCGAGTGAC
TACAGCTACGGATTCCTGGACCGGAATGCCCGCAACAGCCACCAGTACACCTGCAAGC
ACAAGGATCCAACCCCTCAGAGCACCGAGAACAAGCCGCCGTCAGCACCGCCACAGC
CACAAGCCTTCCAGCCGGCCTTCAGCCAACCCAACCAGGCACTGAACAGTCTGGATTT
CAGCCTGCCCATGGACGGGCAGAGGTCCATCGCCGAGCTGATGAACATGTACGACAAC
AACTTCGTGCCGAACAAGAACCCGAGCAGCGACAGCGTCGCCGTCATGGAGAGGCCA
AACGCGATGCCCCAGCAGAGGATCCAGATGGACGAGGGTTTCTTCGTACAGGGCAACG
GAGCCTTCGACGACGTCAACAACAGCATGATGCAGCAGCAGCAGCAGCAGGCGCCAG
TGCAGCAGCAGCAGCAGTTCTTCATCCGCGACGACACGCCATTCGTGAGCCAGATGGG
CGACATCGCCGCCAGCGCGCCGGAGTTCAGGTTCGGTCCTGGTTTCAACATGTCTAGCG
GCGTCGACTACCCAGGCGCGGCACAGAGGAACGACGGACCAATTGGTTCTACTGA
>SEQ ID NO.26 ZmEIL1 KO2 
ATGATGGGAGGCGGGCTGTTGGTGGATCAGAGCGTGGTGTTCCCTGGCGTCCACAACT
TCGTGGATCTCCTGCAGCAGAACGGCGACAAGAACCTGGGCTTCGGGTCTCTGATGCC
GCAGACGTCCTCTGGCGACCAGTGCGTGATGGGGGAGGGCGATCTCGTGGACCCGCCT
CCGGATAGCTTCCCGGACGCCGGGGAGGACGACAGCGACGATGACGTCGAGGACATC
GAGGAGCTGGAGCGCCGCATGTGGCGCGACCGTATGAAGCTGAAGCGGCTCAGGGAA
CTGCAGCAGACCCGCGGCAAGGACTCGTTGGCTAGCGGTGCGGACTGGCTGATGGCT
CGTCCAAGCCAAGGCAGTCGCAGGAGCAGGCCCGGCGCAAGAAGATGTCGCGCGCGC
AGGACGGCATCCTCAAGTACATGCTCAAGATGATGGAGGTGTGCCGCGCGCAGGGGTT
TGTGTATGGGATCATTCCGGAGAAGGGCAAGCCAGTGAGTGGTGCCTCCGACAACCTC
CGTGCGTGGTGGAAGGAGAAGGTCCGCTTCGACCGCAACGGACCGGCCGCCATTGCCA
AGTACCAGGCCGACAACGCCGTCCCTGGCGCCGAGAACGAGCTCGCCTCGGGCGCTGC
CAGCCCCCATTCCTTGCAGGAGCTGCAGGACACTACGCTGGGCTCACTGCTCTCAGCGC
TTATGCAGCACTGCGAGCCCCCACAGCGGCGCTACCCGCTCGAGAAGGGCGTTCCTCC
ACCGTGGTGGCCTACCGGCGACGAGGAGTGGTGGCCGGAACTCGGCATTCCCAAGGAC
CAGGGCCCACCCCCGTACAAGAAGCCTCATGACCTTAAGAAGGCCTGGAAGGTGAGCG
TGCTCACCGCTGTCATCAAGCACATGTCACCGGACATAGAGAAGATCCGTCGTGCGCG
GGCACTGGACGTTGTTGCAGGTGTACAATCGGTTGTTTAGCATCAGCTCTGGCTCGGCT
GCAGCGGGGGCGTTCCTCTTCTGGATGAACTCCACGTCGGCGGTCTCCTCCTTCATCGG
CGTCGGCACGAGGAACTTGTCGCTTAGGATAGCGGCACCAAGGTTGAACGCGGTTGGG
TCAGTGACTGCCGTCCTCTGGTTACCGGCCTCGTCCCCCTTGCACTCATCGATGATGTC
CACATCATACTCGCTCGAGCTGGTGTTGAAGGATATGGCACTTGCGATGCCACCAGTA
 
GACGCCGGTGGGCGTGCGCCCGGGTTCAGCTTCTGGTACAGCTCCTCTTCCTGCTTGAC
GACCGCCAGCCAGGTTGAGATCTCCTTGGCAGTCATCTTGTCCTGGAGGCACTTGGACT
GGCGAACCAGTGACTACAGCTACGGATTCCTGGACCGGAATGCCCGCAACAGCCACCA
GTACACCTGCAAGCACAAGGATCCAACCCCTCAGAGCACCGAGAACAAGCCGCCGTCA
GCACCGCCACAGCCACAAGCCTTCCAGCCGGCCTTCAGCCAACCCAACCAGGCACTGA
ACAGTCTGGATTTCAGCCTGCCCATGGACGGGCAGAGGTCCATCGCCGAGCTGATGAA
CATGTACGACAACAACTTCGTGCCGAACAAGAACCCGAGCAGCGACAGCGTCGCCGTC
ATGGAGAGGCCAAACGCGATGCCCCAGCAGAGGATCCAGATGGACGAGGGTTTCTTCG
TACAGGGCAACGGAGCCTTCGACGACGTCAACAACAGCATGATGCAGCAGCAGCAGC
AGCAGGCGCCAGTGCAGCAGCAGCAGCAGTTCTTCATCCGCGACGACACGCCATTCGT
GAGCCAGATGGGCGACATCGCCGCCAGCGCGCCGGAGTTCAGGTTCGGTCCTGGTTTC
AACATGTCTAGCGGCGTCGACTACCCAGGCGCGGCACAGAGGAACGACGGACCAAT
TGGTTCTACTGA
>SEQ ID NO.27 ZmEIL3 KO1 
ATGATGGGAGGCGGGCTGATGATGGATCAGAGCGTGGTGTTCCCTGGCGTCCACAACT
TCGTGGATCTCCTGCAGCAGAACGGCGACAAGAACCTGGGCTTCGCCTCACTCATGCC
GCAGACGTCCTCCGGCGACCAGTGCGTGATGGGGGAGGGTGATCTCGTGGACCCACCG
CCAGACAGCTTCCCGGACGCCGTGGAGGACGACAGCGATGACGACGTTGAGGACATC
GAGGAGCTGGAGCGCCGCATGTGGCGCGACCGCATGAAGCTGAAGCGGCTCAGGGAG
CTGCAGCAGAGCCGCGGCAAGGATCCCATGGCTAGCGGTGGGGGCCTGGCCGACGGCT
CATCCAAGCCAAGGCAGTCGCAGGAGCAGGCCCGGCGCAAGAAGATGTCGCGCGCGC
AGGACGGCATCCTCAAGTACATGCTCAAGATGATGGAGGTGTGCCGCGCGCAGGGGTT
TGTGTACGGGATCATTCCGGAGAAGGGCAAGCCGGTGAGCGGCGCCTCCGACAACCTC
CGTGCCTGGTGGAAGGAGAAGGTCCGCTTCGACCGCAACGGCCCGGCCGCCATCGCCA
AGTATCAGGCCGACAACGCCGTCCCGGGCGCCGAGAATGAGCTCGCCTCGGGCGCTGC
CAGCCCTCATTCCTTGCAGGAGCTGCAGGACACCACCACTCGGCTCGCTGCTCTCAGCA
CTCATGCAGCACTGCGACCCCCCACAGCGGCGCTACCCGCTGGAGAAGGGCGTTCCTC
CACCGTGGTGGCCTACCGGCGACGAGGAGTGGTGGCCGGAGCTTGGCATTCCCAAGGA
CCAGGGCCCACCCCCCCATGACCTTAAGAAGGCCTGGAAGGTGAGCGTGCTCACCGCT
GTCATCAAGCACATGTCACCAGACATAGAGAAGATCCGACGCCTCGTTCGCCAGTCCA
AGTGCCTCCAGGACAAGATGACTGCCAAGGAGATCTCGACCTGGCTGGCGGTCGTCAA
GCAGGAAGAGGAGCTGTACCTGAAGATGCACCCTGGCGCACGCCCGCCGGCATCCACT
GGTGGCATCGCCAGTGCCATATCTTTCAACACCACCTCAAGCGAGTACGACGTGGACA
 
TCGTTGATGAGTGCAAGGGGGATGAGGCTGGCAACCAGAAGACAGCAGTCACTGACC
CAACCTCCTTCAACCTTGGTGCGGCTATCCTAAGTGACAAGTTCCTCATGCCGACGCCG
ATGAAGGAGGAGACCGCTGACGTCGAGTTCATCCAGAAGAGGAACGCCCCCGCTCCTG
CTGAGCCAGAGCTAATGCTAAACAACCGGGTGTACACCTGCAACAACGTCCAGTGCCC
GCACAGTGACTACAGCTATGGATTCCTTGACCGGAATGCCCGCAACAGCCACCAGTAC
ACCTGTAAGTACAACGATCCGATCACTCAGAGCGCCGAGAACAAGCCTCTGCCAGCAC
CGCCGCAACCACAAGCCTTCCAGCCGGCCTTCAGCCAAGCCAATCAGGCAGCGAACAA
TCTGGACTTCAGCCTGCCTATGGACGGGCAGAGGTCCATCGCCGAGCTGATGAACATG
TACGACACCAACTTCATGACGAGCAAGACCATGAGCAGTAGTGACAGCGTCACCATCA
TGGAGAGGCCGAACGCGATGCCCCAGAGGATCCAGATGGACGAGGGCTTCTTCGGAC
AGGGCAACGGAGTCTTCGACGACGTCAATAGCATGATGCAGCAACAACAGCAGCAGG
CACCACCCGTGGTGCAGCAGCAGCAGCAGCAGCAGTTCTTCATCCGCGACGACACGCC
ATTCATGAGCCAGATGGGCGACATCACCAGCACGGCGGAGTTCAGGTTCGGCTCTGGT
TTCAACATGTCTAGCACCGCCGCCGCCGCTGATTACCCAGGCGCGGCGCAGAAGAACG
ACGGGACCAATTGGTTCTACTGA
>SEQ ID NO.28 ZmEIL3 KO2 
ATGATGGGAGGCGGGCTGATGATGGATCAGAGCGTGGTGTTCCCTGGCGTCCACAACT
TCGTGGATCTCCTGCAGCAGAACGGCGACAAGAACCTGGGCTTCGCCTCACTCATGCC
GCAGACGTCCTCCGGCGACCAGTGCGTGATGGGGGAGGGTGATCTCGTGGACCCACCG
CCAGACAGCTTCCCGGACGCCGTGGAGGACGACAGCGATGACGACGTTGAGGACATC
GAGGAGCTGGAGCGCCGCATGTGGCGCGACCGCATGAAGCTGAAGCGGCTCAGGGAG
CTGCAGCAGAGCCGCGGCAAGGATCCCATGGCTAGCGGTGGGGGCCTGGCCGACGGCT
CATCCAAGCCAAGGCAGTCGCAGGAGCAGGCCCGGCGCAAGAAGATGTCGCGCGCGC
AGGACGGCATCCTCAAGTACATGCTCAAGATGATGGAGGTGTGCCGCGCGCAGGGGTT
TGTGTACGGGATCATTCCGGAGAAGGGCAAGCCGGTGAGCGGCGCCTCCGACAACCTC
CGTGCCTGGTGGAAGGAGAAGGTCCGCTTCGACCGCAACGGCCCGGCCGCCATCGCCA
AGTATCAGGCCGACAACGCCGTCCCGGGCGCCGAGAATGAGCTCGCCTCGGGCGCTGC
CAGCCCTCATTCCTTGCAGGAGCTGCAGGACACCACTCGGCTCGCTGCTCTCAGCACTC
ATGCAGCACTGCGACCCCCCACAGCGGCGCTACCCGCTGGAGAAGGGCGTTCCTCCAC
CGTGGTGGCCTACCGGCGACGAGGAGTGGTGGCCGGAGCTTGGCATTCCCAAGGACCA
GGGCCCACCCCCAGAAGCCCATGACCTTAAGAAGGCCTGGAAGGTGAGCGTGCTCAC
CGCTGTCATCAAGCACATGTCACCAGACATAGAGAAGATCCGACGCCTCGTTCGCCAG
TCCAAGTGCCTCCAGGACAAGATGACTGCCAAGGAGATCTCGACCTGGCTGGCGGTCG
 
TCAAGCAGGAAGAGGAGCTGTACCTGAAGATGCACCCTGGCGCACGCCCGCCGGCATC
CACTGGTGGCATCGCCAGTGCCATATCTTTCAACACCACCTCAAGCGAGTACGACGTG
GACATCGTTGATGAGTGCAAGGGGGATGAGGCTGGCAACCAGAAGACAGCAGTCACT
GACCCAACCTCCTTCAACCTTGGTGCGGCTATCCTAAGTGACAAGTTCCTCATGCCGAC
GCCGATGAAGGAGGAGACCGCTGACGTCGAGTTCATCCAGAAGAGGAACGCCCCCGC
TCCTGCTGAGCCAGAGCTAATGCTAAACAACCGGGTGTACACCTGCAACAACGTCCAG
TGCCCGCACAGTGACTACAGCTATGGATTCCTTGACCGGAATGCCCGCAACAGCCACC
AGTACACCTGTAAGTACAACGATCCGATCACTCAGAGCGCCGAGAACAAGCCTCTGCC
AGCACCGCCGCAACCACAAGCCTTCCAGCCGGCCTTCAGCCAAGCCAATCAGGCAGCG
AACAATCTGGACTTCAGCCTGCCTATGGACGGGCAGAGGTCCATCGCCGAGCTGATGA
ACATGTACGACACCAACTTCATGACGAGCAAGACCATGAGCAGTAGTGACAGCGTCAC
CATCATGGAGAGGCCGAACGCGATGCCCCAGAGGATCCAGATGGACGAGGGCTTCTTC
GGACAGGGCAACGGAGTCTTCGACGACGTCAATAGCATGATGCAGCAACAACAGCAG
CAGGCACCACCCGTGGTGCAGCAGCAGCAGCAGCAGCAGTTCTTCATCCGCGACGACA
CGCCATTCATGAGCCAGATGGGCGACATCACCAGCACGGCGGAGTTCAGGTTCGGCTC
TGGTTTCAACATGTCTAGCACCGCCGCCGCCGCTGATTACCCAGGCGCGGCGCAGAAG
AACGACGGGACCAATTGGTTCTACTGA
>SEQ ID NO.29 ZmEIL3 KO3 
ATGATGGAGGCGGGCTGATGATGGATCAGAGCGTGGTGTTCCCTGGCGTCCACAACT
TCGTGGATCTCCTGCAGCAGAACGGCGACAAGAACCTGGGCTTCGCCTCACTCATGCC
GCAGACGTCCTCCGGCGACCAGTGCGTGATGGGGGAGGGTGATCTCGTGGACCCACCG
CCAGACAGCTTCCCGGACGCCGTGGAGGACGACAGCGATGACGACGTTGAGGACATC
GAGGAGCTGGAGCGCCGCATGTGGCGCGACCGCATGAAGCTGAAGCGGCTCAGGGAG
CTGCAGCAGAGCCGCGGCAAGGATCCCATGGCTAGCGGTGGGGGCCTGGCCGACGGCT
CATCCAAGCCAAGGCAGTCGCAGGAGCAGGCCCGGCGCAAGAAGATGTCGCGCGCGC
AGGACGGCATCCTCAAGTACATGCTCAAGATGATGGAGGTGTGCCGCGCGCAGGGGTT
TGTGTACGGGATCATTCCGGAGAAGGGCAAGCCGGTGAGCGGCGCCTCCGACAACCTC
CGTGCCTGGTGGAAGGAGAAGGTCCGCTTCGACCGCAACGGCCCGGCCGCCATCGCCA
AGTATCAGGCCGACAACGCCGTCCCGGGCGCCGAGAATGAGCTCGCCTCGGGCGCTGC
CAGCCCTCATTCCTTGCAGGAGCTGCAGGACTCGGCTCGCTGCTCTCAGCACTCATGCA
GCACTGCGACCCCCCACAGCGGCGCTACCCGCTGGAGAAGGGCGTTCCTCCACCGTGG
TGGCCTACCGGCGACGAGGAGTGGTGGCCGGAGCTTGGCATTCCCAAGGACCAGGGCC
CACCCCCCAAGAAGCCCCATGACCTTAAGAAGGCCTGGAAGGTGAGCGTGCTCACCGC
 
TGTCATCAAGCACATGTCACCAGACATAGAGAAGATCCGACGCCTCGTTCGCCAGTCC
AAGTGCCTCCAGGACAAGATGACTGCCAAGGAGATCTCGACCTGGCTGGCGGTCGTCA
AGCAGGAAGAGGAGCTGTACCTGAAGATGCACCCTGGCGCACGCCCGCCGGCATCCAC
TGGTGGCATCGCCAGTGCCATATCTTTCAACACCACCTCAAGCGAGTACGACGTGGAC
ATCGTTGATGAGTGCAAGGGGGATGAGGCTGGCAACCAGAAGACAGCAGTCACTGAC
CCAACCTCCTTCAACCTTGGTGCGGCTATCCTAAGTGACAAGTTCCTCATGCCGACGCC
GATGAAGGAGGAGACCGCTGACGTCGAGTTCATCCAGAAGAGGAACGCCCCCGCTCCT
GCTGAGCCAGAGCTAATGCTAAACAACCGGGTGTACACCTGCAACAACGTCCAGTGCC
CGCACAGTGACTACAGCTATGGATTCCTTGACCGGAATGCCCGCAACAGCCACCAGTA
CACCTGTAAGTACAACGATCCGATCACTCAGAGCGCCGAGAACAAGCCTCTGCCAGCA
CCGCCGCAACCACAAGCCTTCCAGCCGGCCTTCAGCCAAGCCAATCAGGCAGCGAACA
ATCTGGACTTCAGCCTGCCTATGGACGGGCAGAGGTCCATCGCCGAGCTGATGAACAT
GTACGACACCAACTTCATGACGAGCAAGACCATGAGCAGTAGTGACAGCGTCACCATC
ATGGAGAGGCCGAACGCGATGCCCCAGAGGATCCAGATGGACGAGGGCTTCTTCGGA
CAGGGCAACGGAGTCTTCGACGACGTCAATAGCATGATGCAGCAACAACAGCAGCAG
GCACCACCCGTGGTGCAGCAGCAGCAGCAGCAGCAGTTCTTCATCCGCGACGACACGC
CATTCATGAGCCAGATGGGCGACATCACCAGCACGGCGGAGTTCAGGTTCGGCTCTGG
TTTCAACATGTCTAGCACCGCCGCCGCCGCTGATTACCCAGGCGCGGCGCAGAAGAAC
GACGGGACCAATTGGTTCTACTGA
> SEQ ID NO. 30 ZmRPG promoter sequence 
ACTGGAGCTTAAACTTCTTAATACAGTTGATAATTCGGACTTGGAATACTCCCTTTTCG
CTGTGGTAGTTCATGTTGGAAGTGGGCCAAACCATGGCCACTATATCAGTTTGGTTAAG
AGCCACAATCATTGGTTATTCTTTGATGACGAGAACGTTGAGATGACTGATGAGTCCAT
GGTACAGGCATTCTTTGGCTCACCGCAGGAGTTCAGTGGTAATACTGATAATGGCTAC
ATACTCTTCTATGAAAGCCTTGCTGAAAGAAGTTGATGTCCTTCTCATACCATGTGATT
AGTTATACACTTTCCTTTCCCTTCCATTTCTGCTGGGATATATAGAATATTCAGTGAAA
ACCAGAATAAGCTGAATGAGAGGCCTTCAGCTTCAGTTGGTATGGTACGATCAACGCT
TGGCAATAAAACAAGTTACCTGGAGGGGCTATAGGCTGACTAGTTTGTAGTTCCTACTT
TATCTGTTTTTATGCATATACAGGTTGTATCAATTCCTGAATAATGTTTGTTGAAGACCT
TTATTATCATGTAAAGAGATTCTATCTAGGAAGCCTTTTTTGGATTCGAGTAAAAAAT
TGTTTCTTAAATAAATAGCTCATGCCGATCTTCATTCATAGGATCCTCAGTTCCTCACCT
GCTCATCGGTAGCTGACATAAGTGATAAGTCCTTAACAGTTTCCAGATCACCTCCTTTG
TGTTATTGTGTCCTGCTCATATGGTACATAACATATTTCAAAAATTCGAAGATGGATCT
 
GAGATTATCTCAAAAGATACATGTTGTTTGAATTCTGAATGCTGTGTGCATGCGCGCGT
GTGTGGGGTGGGGAATCTGTCCTTCCTATCCGAATAGGACTCAACCGAATTTGGATGTC
TAGGCCTTATACTAAGGCCCCGTTTGGTTAGAGGGACTAATCTTTAGTTCTTGGTTTTA
GTCCTGTCTAGTCCATATTTTGCCAAACAGACCTAAGAAAAATATGGTCCCGATCTAGT
TGCATGCACATGTGAGCTGCATCTTCATACACACAACACTCAGCACGAGCACTCGTCTG
TTGGTGGTGTAGAAATAAAGTATTCGAAAAATATGGTTTTAAATACTATAGTTTTGAAA
TGACAAGACATATCTATAGTTTTTTACCACAGTATTAAAAACTTTGATGTGGAGCTAA
GTTTCTAAAACTGTGAAAGTACTGCCGCATAGTCTAAACTACAGTTTGAAAACATAATT
TTTGAAGAGTCTACCAAATATTGTATTAAAAAAATACCGCAACATTTCAAATAAATAC
AGCATTCTTAAGAAACAGAGAAAATACTCCACTCCCAAACGGATCCTGAACTATACAG
CCATTTCGTGTATTTCAACAAATACAACTAGTCCACCTGTTATTACTATAGCCACTACTT
CCTGCAGCTGGCCACTATTGCCATACCGCTGTCATGTTGTTACAACAGATAGAAAAAA
CAAGGTTGACGTATTGCTAACTTTCTGCTGCAACAAAATAAGAGAAAAATACATCAGA
TTATCTTGCATTACGTAGGACTTGGATCAAAGGTTAACAAATACAACTGAATGGTTTAG
ATTTCAGTGGTTTTGAGTGTGGTTGTAGTTGAATTGTATGTTCAAATGGTTGTACAGTTT
GAACATTTTTTATTTTTTTCTTATAAATTATGGTTTTGCACATTTCTTCTAAAATTATG
ACATTAACACTATTTGAAAGGATGAGAAACTGAAATTTTTGCAGTTTTGTTTTGCCACT
TAATCTGGGATCCTTATCCGTCAAGAGTTCATCGATATCCTTATTTGGTATGGATCATC
GTTATCCTTATCTGGTGTGCGTCCTCGGCATCCTTATCTGATGCAGATTTTTTTGGCTCT
ACAGAACCGCGTCCGGTTTTGGATAGCCACAACTTGAACGCGGTTTCGGATAGCTACA
ACTCGAACACGATTTCCTCCTTTAAATAGGTATTGTTGCTGCCATCGAGAGTAGAACAC
AACCAAAGCATCTAGTTTCGTCCCTGCTTCCTGCCTACAGCGTCGTGCGT
> SEQ ID NO. 31 ZmEIL1 promoter sequence 
GTGTCTCCTTTTGAGCTTGTCTATGGGCAGGAAGCAGTGTTGCCTGTGGAAATAAGTTT
GAATGCTGTCAGGTTCGCCAGACAAAATGATTTAAATGCTACTGATTATTATAATTCAA
TGATGGATAATATTGATGAGCTGACCGACAAAAGGATGATAGCTTTGGGAGCAATAGA
AAAGGACAAAATCATGGTAGCCAGGGCCTACAACAAGAAGGTCAAAGCAAAATCATT
TCAAGTAGGGGACCTGGTGTGGAAGACTATTCTGCCTCTAAGGAATAAAGACCGGAAG
TTCGGGAAATGGTCGCCAAGCTGGGAGGGTCCTTATAAAGTAAAACAAGTGATGTCTG
GCAACGCCTATTTGCTACAAACATTACACGGCAAGGATTTGCCTAAGGCTTTGAATGG
GCGTTTTCTCAAACAGTACCATCCTAGTATGTGGCAAGATGGCTAAGAGAACCGATGT
AATCTCATCGAGTTATTTACTTTTGGTTTGCTCAGCTCCACCAAAAGGCAGGGGGCAT
ATGTTGAGCACCAAAATGAGGTGCGGACGGTCCGCGCCTGTGGGCCGGACGGTCCGCG
 
CACGCGCAGAGTAGATTAGGGTTCCGAGTTTGTGCTATGTTTGTTAGCTATATCCGCG
GAATTAGCTCGGAATCCGGTCGTGTAAAGGGTCCAGCCCCCTCCTCTATAAAAGAG
AGGCCTACGACCGATTTGTAATCAACAATCGAATCAATACAACTTCTATTCGCATTTA
TCCTAGGAGTAGTTCTAGTCTTGTTTAGGTTTAGCCTCTCAATCCCCAAATTCTCCGCCT
CTCCTCGACTCTACGTCGATTAGAGGAGTCTAGGTCGGTCTGCCCGAGCCTAGACAACC
CCTAGGATCTCTCCTCCCCGACGGGGTCCCTCCCGGGAGCGAGATCCAGGCGCCGCCG
ACGATCTTCCGCCGCCCCTGCGCACGCGGACCGTCCGGCCCCAGGGCGCGGACCGT
CCGGCCGTCAGGCAGGAAACCTTAGCCCCTGCGCCAGGTCGCGGACCGTCCGGCCCCA
GGTCGCGGACCGTCCGCGCCTGACCAGAGAGCACCGCCGCCGGTTCTTCTTGAGTATTT
GGCGCTCCGAAAAAGCGTCAACAACCACTTTATTATGTATTTATGAAAAATATAATTGT
TGTCTTTCCAGCTAAATTTTATCGTTTTCGAATAATTGACGATCTAGATCCCGTATTCCC
TTTTCGTCCAGTCAAAAAAAATATGAAAACAAAATTAATTTAGATGTTTTTTATTGTT
TTCGACGATTTTTGTCCCTGCGGCTTTCAAACCCGTGGCCTCACTAGTAGCACCCACTC
CTCGGACCCCGCCACACCAGGAGAGGCGCCACTGACCGCCGATCGGACCCCACTTGTC
ACAGGCAGACGCCACCAGCCAAAGCGTTTTCACTCCGTGGCGCGGGTGGCGGTAAAAA
AGCGCCATGACGCGGGTGATTACGGTACGGACCAGCAAGACTTTTTTAACTACCGTG
CCGCATCGCAGTGGCAGCTTGTGAGTGGAGGCGGGGATCATTAGTTTAAGAGGGGAGG
TTATAAAGGCCGCGCTGTCGCTGTTGAGGGCCGAGCAGCAGACCACGACCAGCACGCC
CACTTGCCTGCGATTACTTTGCGCCAGAGGGCAGAAGCCTTGGCCCTGCCATAGCTGTT
CTTAGAGCATCTCCAACAATGCCTCAAACTAATGCCTCAAATCGAAATATAGAGCTCT
ACACAGGAAAAACCACTCCAACAGTGCCTTAATTCAACAATTTTGTCAAAAACTAT
AGGGCACTCTCTCAAGTGCCTCAAATATACTACACCGTAGTGGGCTGCCCTATAATCTA
GATTTGGGGCTTTACTGTTGGAGTAGGGTGTTTTTTGGTGCCCTAAATTCTATAAAATT
TACTTATTTTTAAATTATAAGGCATTTTTATAGGTCACGTTGTTGGAGATGCTCTTATCC
CCCCTTCCTCTTTTCTTTCTCCTCCCTCTTGCGGCTTCTCTCTGGACTCGCCCCTCCCTCC
TCTCGTCTCCCAGGTCATGGCAAAGCTGGAGCACCCGTGAAAATAGCAAGTGGGAGAT
AAACAGGAAGAGCTAGGGTGAGTTCTTCCTACCTTCCTGGACCTTCTATGCTTCCCAGT
TCTTGTGTGGCTGGTCCTTCTTTCTTCGCCGATCTTCTTCTCTTGCTCCTTTCGGTGATA
TCTGTTTGTGCTGCTGCAGGGTGGCCGGATGATTATCTGATAGATTAAGTAGAGTAATA
AATTTTCAGGAGATCTTTTCTCTCTCTAGATTTCTTTTCTTAGGATCCTGTACCTTCTTTG
CAGTGGCCAATTCCCGTTTGAGTTCTTTTCTGATCCTCCCTTTCCCTTGCTGATGTCTTG
TCCTTCTGCGTAATAGCTGCGGCTAAATCCATGGCGGTGTTCCGAGGTTGATGCCTTGG
ATGATGTAGCAGCTCCCATCCAAAAACCTGAGTTTCTTGCATCTCTTTTCTGTTATGTTT
CTTCTAAGCTTCGTTTGGATCTCGATACTTCTAGTCAAAGACTGAGCTCGTTCTAGGAA
 
GTGTGTAGCAGTCGGGTATGATTTGTTCCTGCATAATCTTGGAGGTGCAAGTTTTAT
CAGTTTCTTTAGGATTGATATGCGTTTCGTCCCTTGTTTTTCCCCTATCACAGATGA
CCTTGATTCCCGTTGTCAAGCACATGGGCACATGTTCTTGTTACCAACTGTCCATTCGT
GCTTGGTTGGGTAAAACAAATAGATCCACAAAAAAAATATTCTTTTCGAAAAGCTTC
CCTTACCTGCCTTTTGATCTTTTTTAATCTTTCCTCCTTGTGATCAATCAGAGCTTACGT
TCATACGTTTGCACATGCTTTCGTAAAAAAAGGGCAAAATATGCAACTTTACATGCTTG
TTCCAATGAACCGGCATATGAGAAGTATTTGTAGTTATTTGGTGGAACTATTTATGTGC
ATATGTTTTCATGCTTGAATTCCTTGTTCCACAATTTCTCTTTCCATGTTGTGTAAAATG
TTCCTTTATGCCGAAACAATAAGGACTATGCTTTATTTGTTCCCTCTGAATTGTAAAAG
TATCAATCTCAGAAGAAGGATTGTGGGGGAAGGGTCCATGTATAGTTTGATCAGATCA
TGGCCCTGTTCTGTTTCACCAAACACATGCTAGGCTGCCTTTTTCTGCAACATGATTGTC
TCCTATAGATGTGTAGAGCTATGATGCCAAGGCAAAAAGCCCTTGCAAACCGTGTAAT
CGCCAAATTAAGATGCCACAATAAGCTTCACTCGGTCATCTTAATTACTTTTTTTTACT
ATAACACGCAATCGTGTTGCATATGTTTTTCTTTGCACTTAATTACTGACAGTAAAACA
CAGCAGCTGACGTGTTCATCTTCCTCGTTGCTTCCGCGCAGGTGAATACTAACTACAAG
TAGCAGTAGAAGGAAGAGAGTCAGTCTGCCCGTGACTGCGAGTGAATTTGTTGGGTGT
GGTGTTTGTTGAGATCACG
> SEQ ID NO. 32 ZmEIL3 promoter sequence

AAGCATAGAGATTTAACGAATAAGAACAACATCACCCTGATCACAAGATGCGATTATG
GTGGCGTGGTACTTACCCGTGAAGAACCCTGAGAACATGGGCGACGAGGCGCCAAAG
CTTGTGGTCGCAAAACGTGTGTAGATTTACGACCCCTAATGAGTATTTACAAGTACATA
TTTATAGTCTAGGGGACTCTCCAACCCTAGTGTTACAGACGAGCTCTATCTTCCTATTC
CTAAGCTAACTTATTATCACTAATTTTGTGGCTGCTTATCTCGAGATGTTCTTGTGCGGG
GATACGCCCTTCCGCATCAGTCAGGCCGCTCTTTGCGGCCCATCCTGCGTTGCGGCCTG
TCTCGGGCCTGACTAAAATCCAGTGATAACACCAAGTAAAGTTGAATTAGTCAAGTTA
CTTTCTTTCCCAACCAAGGTATAGCATGGATAAACATTCCTATCCATAAACAAAAAATA
CAATAAGAGTTATAAGAAATTACGTGGAATTCTACTAAACCCTATATTATGTGCATATT
CAATGACACCAGCATTATATTTTGTACATACTTTGTATTCAAGGGAGAATGGAATATTA
AAGAACTATACTCATGGTACGTTTGGTTTAGCTTTTTGAACCTGCTTCTACTTTGAAAA
GACATACCTTCTTAGCTGAACCAAATAGGTTCAACTTTTCTGCAACTACTTTAAAATGA
CAAAAATAAGGTTAAAAATAAATAAGTGTTATAATAAATGAAATAAGAAATATTTTTA
ATAAAAACATATAAAATTATTAAAAATGTTATATATATATATATATAAATATATAAATT
GATTGATAGATATAGTTAAACAAAGTGCATATAAGTAATATATAGTTTGTTCATGGTAA

CTCACAATAGTTTGAACCAAACAACTTTTAGTTTTTCACGGCAAACAACTCATTACAA
TTTTTTATAGCAGATAGCGGAAAACAACTTTTCTCATGGTAGACAACAGAACTAAACG
CACCTTAAATCCTTTGTCACAAACGGTGTACTCCTAAAAATGGTGGGCATGTAAGAAA
ACAAGATAGCATGGAAAATATGTATTAAAATTATGCTGTTATGAAAGTAGGAGTAAT
CGAAGTGACCTCCCACGTTTTCCTTTTATTAAGTTCTTGTTTGGCAACTTTATTTTTCTA
AGAGATTTCTAAATATGATTGTTAAACTAGCCCTAAAAGATAGAATATCTTCGCCTCTG
CACCCACGTACAATTAAATTTTATAATATTTTAGCTTATTTCTGGCAAAATAAAGATG
AAGAGTATACTTGAATATACAAAAGTCATATGCAAAGTATAATATTACTTTATCGTCTA
TTTTTTATAAACATCTTGAAACATTTTTACTTTATCTTCGCGCTGCAGCTGCACCTAGAA
GTAGAACACAACTAATATGCTTCTGCATTTACATGTGAGTCATTGCCGGAATTGTTTAG
AATAGTCTTCCAAATCTGCATCTGAATTTGTTTACGGTATGTAATACTATATGATTCTA
AGGTACCCACCTCTGCTACCGTGAGAAAATAACCCAATATAAAATTATTTGCACTAGG
AACCAATCATGGTCGATGAGCTAGATTAGAGGAACGCGCGCCACATCAAAAGCCGAA
GAGCACGGGGCAGCGGGCCCATCTGCCAGGCCGCCACCACCACCGCCAGCACCCACTC
CCTTTTGTCCTAGCTCTTCGCGCGGTACGATCCTCCTCGGACCACGCCACACCGGGAGA
GGAGAGCAGGAGGCCACCGACCGGCGACCGGGCCCCGCGTGTCACACGCAGACGCCA
CCAGCCAAGCTCTCCGCGGGTGGCGGTAAAAGAGCGCCATCACGCGGGCGATTACGGA
GGGGCAAAATCTTTTTTTACCGCCGCGCAGCATCGCAGTGGCAGCTCGTGAGTGGAGG
GAGGCGGGGATCATTAGTTTAAGAGGGCAGATTATAAAAGCCGGGCTGTCGCTGTTGA
GGCCGAGCACAGCAGCAGAGCACCATCAGCACCAGCACGCCCACTTGCCTCCTATTAT
TTGCGCCAGAGGGCAGGGCAGGGCAGGGCAGGGGCCTTGGCGCTACCCCCGTTCTTAT
CCCCACCTCGTGCCTCCCCTCCTCCCCCCTTCCCCTCTCTCGCCCCTCCTCCCTCCGTCTC
GTCTCCCAGGTCATGGCAAAAGCTGGAGCACCGGGAAAAATAACAAGTGGGAGATAA
ACAGGAAGAGCTAGGTGAGTTCTTCCTACCTGCCTGGACCTCCTACCCTTCCCAGTTCT
TGTGTGGCTGGTCCTCCTTTTCTTCGCTGATCTTCTTCTTCCCTTGCTGCTTGTGCTACTG
GAGTGTGGTCGCCTGATTATCTGATGGATATGTAGAGTGATATTTTTTTCCCCAGGAG
ATCTTTTTTCCTCTCTAGATTTTTTTTTTCAGTATCCTGTAGCTTCCCGAGGTGGCCAATT
CCCGTTTGAGTTCCGATCCTTCTTTTCCCTTGCTGATGTTCTGTCCTCCAGCGTAATAGC
TGCGGCTAAATCCACGACAGAGTTCCAAGGTTGATGCCTTCGATGATTTTGCAGCTCCC
ATCCAAAAACCTGAGTTTCTTACATCTCTTTCCTCTTTTTTTCCCCTTCTACAAGTTTCG
GTTGGATCTCAATACCACAATTAGTCAAAGGCTCAGCTCGTTCTACGGTTCTAGGAAGT
GTCTAACAGCCGGGTGTGATGGAAACATTCTGTTCCTGCATCATCTTCGGAGGCGCAA
AATTTGACCAGTTTTCGTTAGGTTTGATACGTGTTTAGTCCATTTAGTCCCTTGTTTTCT
TCCCTTTCATGTATGACCTTGATTCCCCCTTTCAAGCACATGGGTGCATGTTCTTTGTTA

CCAACTGTCAGTTCGTGCTTGGCTGGTTGAAAAAAAAATGACTACTTATTTTTTTTCCT
GAAAAGCTTTCCTTCCCTGCCCTTTGATCTTTTTTAGCTTTCCCCCTTGTGATTCAACC
AGAGCTTGTGAACATGCTTTCGTAAAAAAAGGGGAAAGATGCAACTTTACCTGCTTG
TTCCGTGAACCGGCATATGAGTAGTTTTTTATGGAACTATTTATATATTCTCATAAGTT
TTCATGCTTTAATTCCTTCTTCCACAAGTATGTGTGATGCATATTTCTCACCCATGCTGT
GTAATCTGTTTCTTTTATGTCGAAACAATAAGGGCCATGCTATATTTGGCCATGTATAG
TTTGATCAGATCAAGGCCCTGTTCTGTTTCACCAGACACATGCCAGGTTGCTAACTTTG
GCCTCCCGGCTGTTGCTTCAGTAGGCAATCTTGCCTTTTTCTGCTACACGATTGTCTCCT
GTAGCTGTGTAGATCTATGATGCCAAGGCAAAAGGCCCTCATGTAATCACCAAATTAA
GATGCCACAGTAGCGTTCACTGGACCATCTTTAATTACCTTTTTTACTAACACGCAATC
GTGTTGCACATGTTTACCCTTGCCCTGCACTTATTGTCTGTGCACTGAATTGCTGACAGT
AAACACAGCAGCTGACGTGTTCGTCGTGTTGCTTCCGCGCAGGTGAATACTTGGAGAA
AAAGGTCCCCCACACCCCCCACGCAGGTGAATACTACAAGTAGCAGTACAAGGAAGA
GAGCCAATCTGCCCGTGACTGCCAGTGAGTTGCTCGGGTGTGGTGGTTAGTTGAGGTC
ACG

### Example 3

SEQ ID NO. 33 Zm00001d000184 
TCCCTTCTTCATCTCTTCTCCTCACAAGGCTTCCTCCCACCACCTCCAGTCCTCCACCGG
ACCACCACCTCCAGTCCTCC
ACCGGACCACCACCTCCACCACTCCTCTCTCAGAACACGCAAACCGCTCGAACCCT
AGCTGGTGGCGGAGGCGGAGGTGGCTCCGGGATGACGCGGCGGTGCTCGCACTGCA
GCCACAACGGGCACAACTCGCGGACGTGCCCCAACCGCGGGGTCAAGATCTTCGGGGT
GCACCTCACCGATGGCTCGGCCATCCGCAAGAGCGCGAGCATGGGGAACCTCTCCCTC
CTCTCCGCGGGATCCACCAGCGGCGGCGTCCCCCGCCGACGGCCCGACCTCGCCG
ACGGCGGCGGGGGCTACGCCTCCGACGACTTCGTCCAGGGGTCGTCCTCCGCCAGCCG
CGATCGAAAGAAAGGTCAGCTACCGCCCGATCTCCACCCCTCGATCATCCACCCGCTA
CCGCCCGATGCAGAACCCTAGATAATCCATCCTTCCAGCTCCTCCAAGCTTTTCCACCC
CTCTTAATCTTTTTAGATGCACCTAGTGCTCGTTGATTAGGTGCCTGTAAGTTTTAGGAA
ACGGGTACGACTCCAGAGAGAGAACATTAATGGTGGTACAACTACCACACTGCGAGTA
CGACTATTCTATGGATATTGTACTAGTTGACTCGATACGTATGGTACTCGAAACTCGAG
TTCAACCCCTAACTGGTACAATGACCACCTATGGAGGCCCTGTACTGTTTTAGTTAGGC
CATGCGGATGCTTTAAGAAGCTCATCTATAGAAACTTTTAGAATATGGGTTATGCATTG
 
TTCCAATCAGCGGCATCAGTTGATTTTGATGAAATTTGTCACGGGTTTGCGACGCTGAA
TAGCTGTGGAGGACTGGAGGTATAGAGAGATGCGTAGTCAGAGGTATAGGGCTGCGTT
ATCAGGAAGGATCTGCCGAGCAATTGAAACTGCTGCGCTAACTGCTGACGCGTAGTCT
TATGTATGCCATACATGCGCCAATGGCCAAGGTCTGGTGTAAATGTGCTACTGCACTGG
GATGTAATTACTATTACGCAGTGCCAAGTAACGGCTGTCCATACTTGATAAGGAGACA
CGTTTTTCACATGTTATGGGGAAATAAAAATAAAATGCTTGATAAATGACTACCAATG
TTCTGCAAGTTTATCTCTATTGTTATTATTGCTGCTGATTCTTGTGAGATTTTTATGCG
AACAATGATAGCTAGATCTTGGTTTACACTTGGTGGTGGGTGGTAGCTGTTTTAGTTTG
AGTCAACTGGCACCTTCTTCAGATTATGCCCTTTTTAGCTCTCCTCTGATTTCGTGCTG
TTTGGCTATTCTTCTATTTGTCAATGGCACCCTTCCTATATGATTTGTTCAACAGCAAAG
TGGCGGTGCTAACTTTACCATGCTCTGTTAATATGCTGCTGTTGATTCATTTCAGTTCA
CTGTCTTCTTGTAGCTACACATATTGCTGCCTGCCTTCATGGCTGCATTTTGGGCTTTCT
ATGCCATGTTGCTGATTAAGGTTGCTCTGACAGGTGTTCCTTGGACTGAAGAAGAACAC
CGGAGGTTTTGCTGGGATTACAAAAGCTCGGAAAGGGGATTGGCGAGGAATTTCTC
GTAATTTTGTGGTCTCAAGAACACCTACTCAAGTAGCAAGTCATGCTCAAAAGTATTTT
ATACGCCAATCAAATATGAGCAGAAGGAAGAGAAGGTCTAGCCTTTTCGACATGGTTC
CTGATGAGGTAACTTGCTTGGTCTTAGTCTTCATTTCTACACAAATATTGCTTGCTATGG
CCTTGTTTTATACCTATGCATGTGTTGTAACTACATTATTGCTTCTTTTTGTAGTAATATT
TATCATATTACTTATACTAGTGCAACTTTTTGGATCTAGTTCTGCAGCTTGTATGTTTTC
CTGGCAGTCACTGTTTGCTGTTTAAACAGAGTGTAAATGGTAAAAGGCTAACTGTGCC
ATTTAGGCCGTAACGTTCCATTAAATGGTTGGACCGTTGAAACCATAAAAAATTGTCTG
AACAGCTTAAACAGAATAAAGTTAAACATGAGTTAATGGGCTAAATTGCCGTTTAACT
CACCGTTTAGTCAATAGGTAGATTATAACAATCACAGTGGCACCAAATTAATTGACATT
GTAATTTAACTCACCGTTTAGTTAATAGGAGGATTATAACTATCACATTGTCCTGTTTA
GTAGAGTTCGACTTCTTGATTCGTTTAGAGTGATTTAGAAGTGATTTTGTTGGGGATAA
AACGCAGATCGGTCAGGGTGGGATTTTTTAGCCGTTTGGCTGTCTAGGAGCGATTCTCA
CAGAGAATATGGAGCTGTGAGCGCTTCCTTGTGGCTTGTGCTTTGCATATAATTTGTGA
ACTGTGTTCTGTTTGTTTTACAGTCCATGGACCTTCCGCCCCTTCCTGGAAGTCAAGAA
CCAGAGACCTCAATGTTAAATCAACCGCCACTGCCTCCTGCTGTGGAGGAGGAGGTGG
AATCGATGGAGTCAGATACTTCTGCTGTCGCAGAGAGTTCTGGAGCTTCTGCTCTCATG
CCCGAGAGTTTACAGCCTACCTATCCGATGATTGTTCCAGCTTATTTCTCGCCGTTCTTG
CAATTCTCAGTTCCTTTCTGGCCAAATCAGGAAGATGGAGGCGATCTTCCCCAAGAAA
CACACGAGATTGTCAAGCCTGTTGCAGTTCATTCCCAGAATCCAATTAATGTTGATGAA
CTCGTGGGCATGTCAAAGCTAAGCATATGGGAGCATGGTCAGGAGACAGTGTCTACTT
 
CTCTGTCGCTAAATCTGCTAGGGGGTCAAAATAGGCAGTCGGCTTTCCATGCAAACCCT
CAAACAAGAGCTCAAGCCTGATCATCCCCTACCAGCACAGAAACTGCATTTAGATGG
AATCCTGGGCAAAATAAGCTTTGCTCTTTTTCTTCTTCTAAATTTAAGATCACTGAGTC
ATAAGGTCACTGGTTCGAAGTAGCTTCTCCGTATTTGCGGGAAAGCCTGCCCTGGTTT
CTCTCCTTCATCAGACCCCACTCATATGGGAGCCTTCAATATTGTCTTTTTATATATTT
AAGATTGACCGTGCTCGGTAGCTTGTATCATGTGCTGTAAGTTATGCTATGTATGAATG
TACCTGTAGTTTGTATGGCACGCATGGTATGCCACTCTTATGTTAGCAAAGTCATTAAT
GTTAGTGCTGATCTTTATGGTTGTAATTTTATAAACTTCTAAAAAAAAGTATAAGCCAG
TTCTTTTCAACTG
SEQ ID NO. 34 Zm00001d029875 
GCACCGGCTCCGGTCCTCCTCCCATCACCATCACCACCGTCGTTCCTCTCACGAGTCCC
GACACACGCAAACCGCACGAACCCTGGCTGGCGGGATGACGCGGCGGTGCTCGC
ACTGCAGCCACAACGGGCACAACTCGCGGACGTGCCCAACCGCGGGTCAAGATCTT
CGGGGTGCGCCTCACCGATGGCTCGGCCATCCGCAAGAGCGCAAGCATGGGAACCTC
TCCCTCCTCTCCGCGGGGTCAACCAGCGGCGGCGTCGCCCGCCGACGGCCCGACC
TCGCCGACGGCGGCGGGGGCTACGCCTCCGACGACTTCGTCCAGGGGTCGTCCTCCGC
CAGCCGCGAGCGTAAGAAAGGTCAGTTACCGCCCGATCTCTGCCCCCTCCTCTCGAATC
GATCATCCACCCGCTACCACTCGATGCAGAACCCTAGATAATAGATCCTTCCAGCTCTA
ACCAGCTCCAAGTTTTTCCATTCATTTCTCTCCTGCCTACTCGGCTTACTCTTTTAGAT
GCACCTAGTGGCTAGTGCTCACTGATTAGGTACCTGTAAGGTTGGGAAACGGGTACTG
CACCGGCTGAGAGAAAATCATTAATGGTAATACCACACTGTGCTGAGTACTACTATTG
TATCGATATTGTACTAGTTGAGTTGATACATATGGTACTCGAATTCAGCCCCTGACTGG
TGCAAGGACCACCTGCCCTATGTCGTTTGACTGGGTCATGGGGATAATTTAACAAACTC
AGCTCTAGAAACTTTTAGAAAATGGGTTATGGATTGCTCCAAGCAGTGGTGTCAGTTG
ATTTTGATGAAATTTGTCACTGATTCGTTATGCTGAATGGCTGTAGAGGCATAGAGGGC
TGCGTAATCAAAGGTACAGGGCTGCGTAATCAGGAAGGATATGCCGAGCAATTGAAAC
CGCTGCGCTAACTGCTGAAACGTTGTCTTATGTATGCCATACATGTGCTAATGGTCACG
GTCTGGTGTAATGTGCTACTGCACTGGGATGTAATTACTATTACACAGTGCCATATTCC
GGCTGCCCATACTTGATAAGGAAACAAGTTTTTCATATGTTATGGGAAAAATCAAA
TGCTTGATACATGAATACCAATGTTCTGCAAGTTTATCTTTATTGTTAATTATTGCTGC
TGATTCTTGTGAGATTTTACACGAACAGTGATATCTAGTTCTTGGGTTACACTTGGTG
GTGGGTGGTAGGTGTTTAATGTAAGTCAAGTGGCAAGAAAAGGGCAAACACAGTGCC
GGAGGCTCCCACATGAGTGGGGTCTGCGGAAGGGATAAACCGAGGCAATCCTGTAGG
 
GTCTAAGAAAAGAGTAAAACTGAGTCAAACCTTCCCCCATAAATACAGAGAGGTTGTT
TCAAACTCGGACTGGGTGACTCATGAGACAGCTCTCACCACTACACTAGGCCTGCCCTT
CAATGTAAGTCAAGTGGCACCTTCTGCATATTATGCCCATCTCTGTTCTGATTTGTGCT
GTTTGGCTAGTCCTCTGTTTGTCGATGACACCCTACCTATATGATTTGTTTAACAGCAA
GGTGGTGATGCTAACTTTTACCATAGTCTGTTAATATGCTGCTGTCCATTCCTTTCTGTT
CACTGGTTTCTTGTTGCTTCACATATTGCTGCCTACCTGCATGGCTGCATTGCTGATTGA
GGTTGCTCTTACAGGTGTTCCTTGGACTGAAGAAGAACACCGGAGGTTTTGCTGGGAT
TACAAAAGCTTGGGAAAGGTGATTGGCGAGGGATTTCTCGTAATTTCGTGGTCTCAAG
AACACCTACTCAAGTAGCAAGTCATGCTCAAAAATATTTATACGTCAATCAAATATG
AGCAGAAGGAAGAGAAGGTCTAGCCTTTTTGACATGGTGCCTGATGAGGTAACTTGCT
TGATTTACCTTGATTGCTTGCTTTTGCCTTGTTTTGTACTCATACATGTGTTGTAACTAC
ACTATTGCTTTTTTGTAATAAATATCATATTACTTATACTTGTGCAACATTTTGGATCT
AGTTCAGTCGCTTGTATGTGTTACTGACACTCATTGTTCGCTGTTTAAACATTGTGTAAA
TGGTAAAAGGCCAGCTGTGTCATTTAGGCCTTAAACGTTCCATCAAATGGTTGGACTGT
TTAAACTGTACGGTCTAAATGGCTTAAACATGATTAAATGGGTTAAATTGCTGTTTAAC
TCACTGTTTATTCAACAGGCAGATTACAACTATCGCATTGGCACCATACTAATTAACAT
TGTAAGAGTGGAATTTAGTTCCTTTCCTCAATTTTTTTGCTAGGATACATATTTTTTAC
ATATGTAAATGTGTATACTTTCTGGTATATGTATGTTTGACTTTTGCATACATAAAATAT
GTATATTTTAGTGGTACTATACAAAAGCGTTTAGATCGTTTAACTCAGTTTAAACACCG
TCTAAACAGTGAACACTGGGTGACCAACTGTTTACTGTTTAGCATTTAGATAACACTGG
TGACGGTTATGCGCAGTGTCCTATCCTTGATCTCATTAGGTAGTTCTTACGGCCTGTTTG
GTAGAGCTTTATTTGTTGATTCTTAATTCTAGCAATTTTGATGGGAAGTAGGTCAGAA
GTGATTTAGAGTGATTCTGGTGGGATTAAATGAAGTGGTGGAGAGGAATTTTCTGTA
GCCATTTGGCTGTCCAGGAGTGATTCTCACAAGAATCTGGAGCTGGGAGCAATTACTA
GTGCTTTGCATATAATTTGAACTGAGCTGTGCTCTGTTTGTTTTACAGTCCATGGACCTT
CCACCCCTTCCTGGAAGTCAAGAGCCAGAGACCTCAGTGTTAAATCAACCACCACTGC
CTCCCCCTGTGGAGGAGGAGGAGGAGGTGGAATCGATGGAGTCAGATACTTCTGCTGT
TGCGGAGAGTTCTGCAGCTTCAGCTCTTATGCCCGAGAGTTTACAGCCTACCTATCCGA
TGATTGTTCCAGCTTATTTCTCACCGTTCTTGCAATTCTCAGTTCCTTTCTGGCCAAATC
AGGAAGATGGAGGTGATCTGCCTCAAGAAACGCACGAGATTGTCAAGCCTGTTGCAGT
TCATTCCAAGAATCCAATTAATGTTGATGAACTTGTGAGCATGTCAAAGCTAAGCATA
GGGGAGCCTGGTCAGGAAACGGTGTCTACTTCTCTGTCGTTAAATCTGCTGGTGGGTCA
AAATAGGCAGTCGGCCTTCCATGCAAATCCTCAAACGAGGGCTCAAGCTTGATCCCGC
TTCCGCACAGAAACTGCATTAAGAAGGATTCCCTCTCTTTTTTTTTTTCTATATGTAAGAT
 
TGACCGTGCTCTGTAGCTTGTATCATGTTGTAAGTTATGCTATGTATGAATGTACCTGTT
GTTTGTCTGGCACGCATGACATGCCACTCATGATAACAAACTCATAATGTTAGTGCTGA
TCTTTATGGTTGTAATGTTGTAAACTTTTCTGAGAGAAAAAAACGTACTAGCCAGTTGT
TTTC
SEQ ID NO. 35 Coding sequence of Zm00001d000184 in K22 
ATGACGCGGCGGTGCTCGCACTGCAGCCACAACGGGCACAACTCGCGGACGTGCCCCA
ACCGCGGGTCAAGATCTTCGGGGTGCACCTCACCGATGGCTCGGCCATCCGCAAGAG
TGCGAGCATGGGGAACCTCTCCCTCCTCTCCGCGGATCCACCAGCGGCGGCGCTTCCC
CCGCCGACGGGCCCGACCTCGCCGACGGCGGCGGGGGCTACGCCTCCGACGACTTCGT
CCAGGGGTCGTCCTCCGCCAGCCGCGATCGCAAGAAAGGTGTTCCTTGGACTGAAGAA
GAACACCGGAGGTTTTTGCTGGGATTACAAAAGCTTGGGAAAGGGGATTGGCGAGGA
ATTTCTCGTAATTTTGTGGTCTCAAGAACACCTACTCAAGTAGCAAGTCATGCTCAAAA
ATATTTATACGCCAATCAAATATGAGCAGAAGGAAGAGAAGGTCTAGCCTTTTCGAC
ATGGTTCCTGATGAGTCCATGGACCTTCCACCCCTTCCTGGAAGTCAAGAACCAGAGAT
CTCACAGTTAAATCAACCGCCACTGCCGCCTCCTGTGGAGGAGGAGGTGGAATCGATG
GAGTCAGATACTTCTGCTGTCGCAGAGAGTTCTGGAGCTTCTGCTCTCATGCCCGAGAG
TTTACAGCCTACCTATCCGATGATTGTTCCAGCTTATTTCTCGCCGTTCTTGCAATTCTC
AGTTCCTTTCTGGCCAAATCAGGAAGATGGAGGCGATCTTCCCCAAGAAACACACGAG
ATTGTCAAGCCTGTTGCAGTTCATTCCCAGAATCCAATTAATGTTGATGAACTCGTGGG
CATGTCAAAGCTAAGCATATGGGAGCATGGTCAGGAGACAGTGTCTACTTCTCTGTCC
CTAAATCTGCTGGGGGGTCAAAATAGGCAGTCGGCTTTCCATGCAAATCCTCAAACAA
GAGCTCAAGCCTGA
SEQ ID NO. 36 Coding sequence of Zm00001d029875 (ZmMYBR43) in K22 
ATGACGCGGCGGTGCTCGCACTGCAGCCACAACGGGCACAACTCGCGGACGTGCCCCA
ACCGCGGGGTCAAGATCTTCGGGGTGCGCCTCACCGATGGCTCGGCCATCCGCAAGAG
CGCAAGCATGGGGAACCTCTCCCTCCTCTCCGCGGGGTCAACCAGCGGCGGCGCGTCG
CCCGCCGACGGGCCCGACCTCGCCGACGGCGGCGGGGGCTACGCCTCCGACGACTTCG
TCCAGGGGTCGTCCTCCGCCAGCCGCGAGCGTAAGAAAGGTGTTCCTTGGACTGAAGA
AGAACACCGGAGGTTTTTGCTGGGATTACAAAAGCTTGGGAAAGGTGATTGGCGAGGG
ATTTCTCGTAATTTCGTGGTCTCAAGAACACCTACTCAAGTAGCAAGTCATGCTCAAAA
ATATTTTATACGTCAATCAAATATGAGCAGAAGGAAGAGAAGGTCTAGCCTTTTTGAC
ATGGTGCCTGATGAGTCCATGGACCTTCCACCCCTTCCTGGAAGTCAAGAGCCAGAGA
 
CCTCAGTGTTAAATCAACCACCACTGCCTCCCCCTGTGGAGGAGGAGGAGGAGGTGGA
ATCGATGGAGTCAGATACTTCTGCTGTTGCGGAGAGTTCTGCAGCTTCAGCTCTTATGC
CCGAGAGTTTACAGCCTACCTATCCGATGATTGTTCCAGCTTATTTCTCACCGTTCTTGC
AATTCTCAGTTCCTTTCTGGCCAAATCAGGAAGATGGAGGTGATCTGCCTCAAGAAAC
GCACGAGATTGTCAAGCCTGTTGCAGTTCATTCCAAGAATCCAATTAATGTTGATGAAC
TTGTGAGCATGTCAAAGCTAAGCATAGGGGAGCCTGGTCAGGAAACGGTGTCTACTTC
TCTGTCGTTAAATCTGCTGGTGGGTCAAAATAGGCAGTCGGCCTTCCATGCAAATCCTC
AAACGAGGGCTCAAGCTTGA
SEQ ID NO. 37 Protein sequence of Zm00001d000184 (ZmMYBST1) 
MTRRCSHCSHNGHNSRTCPNRGVKIFGVHLTDGSAIRKSASMGNLSLLSAGSTSGGASPAD
GPDLADGGGGYASDDFVQGSSSASRDRKKGVPWTEEEHRRFLLGLQKLGKGDWRGISRN
FVVSRTPTQVASHAQKYFIRQSNMSRRKRRSSLFDMVPDESMDLPPLPGSQEPETSMLNQP
PLPPAVEEEVESMESDTSAVAESSGASALMPESLQPTYPMIVPAYFSPFLQFSVPFWPNQED
GGDLPQETHEIVKPVAVHSQNPINVDELVGMSKLSIWEHGQETVSTSLSLNLLGGQNRQSA
FHANPQTRAQA
SEQ ID NO. 38 Protein sequence of Zm00001d029875 (ZmMYBR43) 
MTRRCSHCSHNGHNSRTCPNRGVKIFGVRLTDGSAIRKSASMGNLSLLSAGSTSGGASPAD
GPDLADGGGGYASDDFVQGSSSASRERKKGVPWTEEEHRRFLLGLQKLGKGDWRGISRNF
VVSRTPTQVASHAQKYFIRQSNMSRRKRRSSLFDMVPDESMDLPPLPGSQEPETSVLNQPP
LPPPVEEEEEVESMESDTSAVAESSAASALMPESLQPTYPMIVPAYFSPFLQFSVPFWPNQE
DGGDLPQETHEIVKPVAVHSKNPINVDELVSMSKLSIGEPGQETVSTSLSLNLLVGQNRQSA
FHANPQTRAQA
SEQ ID NO. 39 ST1 & R43-KO target 1
   GAACCTCTCCCTCCTCTCCG
SEQ ID NO. 40 ST1 & R43-KO target 2
   CCTCCGACGACTTCGTCCAG
SEQ ID NO.41 gRNA1 
GAACCUCUCCCUCCUCUCCGguuuuagagcuagaaauagcaaguuaaaauaaggcuaguccguuaucaacuugaa
aaaguggcaccgagucggugcuuuuuuu
SEQ ID NO.42 gRNA2 
CCUCCGACGACUUCGUCCAGguuuuagagcuagaaauagcaaguuaaaauaaggcuaguccguuaucaacuugaa
aaaguggcaccgagucggugcuuuuuuu
SEQ ID NO. 43 DNA sequence after knockout of Zm00001d000184 (ZmMYBST1) of ST1 & R43-KO1 
TCCCTTCTTCATCTCTTCTCCTCACAAGGCTTCCTCCCACCACCTCCAGTCCTCCACCGG
ACCACCACCTCCAGTCCTCCACCGGACCACCACCTCCACCACTCCTCTCTCAGAACACG
CGCAAACCGCTCGAACCCTAGCTGGTGGCGGAGGCGGAGGTGGCTCCGGGATGACG
CGGCGGTGCTCGCACTGCAGCCACAACGGGCACAACTCGCGGACGTGCCCCAACCGCG
GGGTCAAGATCTTCGGGGTGCACCTCACCGATGGCTCGGCCATCCGCAAGAGCGCGAG
CATGGGGAACCTCTCCCTCCTCTTCCGCGGATCCACCAGCGGCGGCGCTTCCCCCGCC
GACGGGCCCGACCTCGCCGACGATCGCAAGAAAGGTCAGCTACCGCCCGACCCCGATC
TCTACCCCTCGTCCCTCGATCATCCGCCCGCTACCGTCCGATGCAGAACCCTAGATAAT
CGATCCTTCCAGCTCTAACCAGTTCCTCCAAGCTTTTCCATCCCTCTTAATCTTTTTAGA
TGCACCCACTGCTCGTTGATTAGGTGCCTGTAAGTTTTAGGAAACGGGTACGACTCCAG
AGAGAGAACATTAATGGTGGTACAACTACCACACTGCGAGTACGACTATTCTATGGAT
ATTGTACTAGTTGACTCGATACGTATGGTACTCGAAACTCGAGTTCAACCCCTAACTGG
TACAATGACCACCTATGGAGGCCCTGTACTGTTTTAGTTAGGCCATGCGGATGCTTTAA
GAAGCTCATCTATAGAAACTTTTAGAATATGGGTTATGCATTGTTCCAATCAGCGGCAT
CAGTTGATTTTGATGAAATTTGTCACGGGTTTGCGACGCTGAATAGCTGTGGAGGACTG
GAGGTATAGAGAGATGCGTAGTCAGAGGTATAGGGCTGCGTTATCAGGAAGGATCTGC
CGAGCAATTGAAACTGCTGCGCTAACTGCTGACGCGTAGTCTTATGTATGCCATACATG
CGCCAATGGCCAAGGTCTGGTGTAAATGTGCTACTGCACTGGGATGTAATTACTATTAC
GCAGTGCCAAGTAACGGCTGTCCATACTTGATAAGGAGACACGTTTTTCACATGTTAT
GGGGAAATAAAAATAAAATGCTTGATAAATGACTACCAATGTTCTGCAAGTTTTATCT
CTATTGTTATTATTGCTGCTGATTCTTGTGAGATTTTTATGCGAACAATGATAGCTAGA
TCTTGGTTTACACTTGGTGGTGGGTGGTAGCTGTTTTAGTTTGAGTCAACTGGCACCTTC
TTCAGATTATGCCCTTTTTTAGCTCTCCTCTGATTTCGTGCTGTTTGGCTATTCTTCTATT
TGTCAATGGCACCCTTCCTATATGATTTGTTCAACAGCAAAGTGGCGGTGCTAACTTTT
ACCATGCTCTGTTAATATGCTGCTGTTGATTCATTTCAGTTCACTGTCTTCTTGTAGCTA
CACATATTGCTGCCTGCCTTCATGGCTGCATTTTGGGCTTTCTATGCCATGTTGCTGATT
AAGGTTGCTCTGACAGGTGTTCCTTGGACTGAAGAAGAACACCGGAGGTTTTTGCTGG
 
GATTACAAAAGCTCGGGAAAGGGGATTGGCGAGGAATTTCTCGTAATTTTGTGGTCTC
AAGAACACCTACTCAAGTAGCAAGTCATGCTCAAAAGTATTTATACGCCAATCAAAT
ATGAGCAGAAGGAAGAGAAGGTCTAGCCTTTTCGACATGGTTCCTGATGAGGTAACTT
GCTTGGTCTTAGTCTTCATTTCTACACAAATATTGCTTGCTATGGCCTTGTTTTATACCT
ATGCATGTGTTGTAACTACATTATTGCTTCTTTTTGTAGTAATATTTATCATATTACTTA
TACTAGTGCAACTTTTTGGATCTAGTTCTGCAGCTTGTATGTTTTCCTGGCAGTCACTGT
TTGCTGTTTAAACAGAGTGTAAATGGTAAAAGGCTAACTGTGCCATTTAGGCCGTAAC
GTTCCATTAAATGGTTGGACCGTTGAAACCATAAAAAATTGTCTGAACAGCTTAAACA
GAATAAAGTTAAACATGAGTTAATGGGCTAAATTGCCGTTTAACTCACCGTTTAGTCAA
TAGGTAGATTATAACAATCACAGTGGCACCAAATTAATTGACATTGTAATTTAACTCAC
CGTTTAGTTAATAGGAGGATTATAACTATCACATTGTCCTGTTTAGTAGAGTTCGACTT
CTTGATTCGTTTAGAGTGATTTAGAAGTGATTTGTTGGGATAAAACGCAGATCGGTC
AGGGTGGGATTTTTTAGCCGTTTGGCTGTCTAGGAGCGATTCTCACAGAGAATATGGA
GCTGTGAGCGCTTCCTTGTGGCTTGTGCTTTGCATATAATTTGTGAACTGTGTTCTGTTT
GTTTTACAGTCCATGGACCTTCCGCCCCTTCCTGGAAGTCAAGAACCAGAGACCTCAAT
GTTAAATCAACCGCCACTGCCTCCTGCTGTGGAGGAGGAGGTGGAATCGATGGAGTCA
GATACTTCTGCTGTCGCAGAGAGTTCTGGAGCTTCTGCTCTCATGCCCGAGAGTTTACA
GCCTACCTATCCGATGATTGTTCCAGCTTATTTCTCGCCGTTCTTGCAATTCTCAGTTCC
TTTCTGGCCAAATCAGGAAGATGGAGGCGATCTTCCCCAAGAAACACACGAGATTGTC
AAGCCTGTTGCAGTTCATTCCCAGAATCCAATTAATGTTGATGAACTCGTGGGCATGTC
AAAGCTAAGCATATGGGAGCATGGTCAGGAGACAGTGTCTACTTCTCTGTCGCTAAAT
CTGCTAGGGGGTCAAAATAGGCAGTCGGCTTTCCATGCAAACCCTCAAACAAGAGCTC
AAGCCTGATCATCCCCTACCAGCACAGAAACTGCATTTTAGATGGAATCCTGGGCAAA
ATAAGCTTTGCTCTTTTTTCTTCTTCTAAATTTAAGATCACTGAGTCATAAGGTCACTGG
TTCGAAGTAGCTTCTCCGTATTTGCGGGGAAAGCCTGCCCTGGTTTCTCTCCTTCATCA
GACCCCACTCATATGGGAGCCTTCAATATTGTCTTTTTTATATATTTAAGATTGACCGTG
CTCGGTAGCTTGTATCATGTGCTGTAAGTTATGCTATGTATGAATGTACCTGTAGTTTGT
ATGGCACGCATGGTATGCCACTCTTATGTTAGCAAAGTCATTAATGTTAGTGCTGATCT
TTATGGTTGTAATTTTATAAACTTCTAAAAAAAAGTATAAGCCAGTTCTTTTCAACTG
SEQ ID NO. 44 DNA sequence after knockout of Zm00001d029875 (ZmMYBR43) of ST1 & R43-KO1 
GCACCGGCTCCGGTCCTCCTCCCATCACCATCACCACCGTCGTTCCTCTCACGAGTCCC
GACACACGCAAACCGCACGAACCCTGGCTGGCGGCGGATGACGCGGCGGTGCTCGC
 
ACTGCAGCCACAACGGGCACAACTCGCGGACGTGCCCCAACCGCGGGGTCAAGATCTT
CGGGGTGCGCCTCACCGATGGCTCGGCCATCCGCAAGAGCGCAAGCATGGGGAACCTC
TCCCTCCTCAGGGGTCGTCCTCCGCCAGCCGCGAGCGTAAGAAAGGTCAGTTACCGCC
CGATCTCTGCCCCCTCCTCTCGAATCGATCATCCACCCGCTACCACTCGATGCAGAACC
CTAGATAATAGATCCTTCCAGCTCTAACCAGCTCCAAGTTTTCCATTCATTTCTCTCCT
GCCTACTCGGCTTACTCTTTTTAGATGCACCTAGTGGCTAGTGCTCACTGATTAGGTAC
CTGTAAGGTTGGGAAACGGGTACTGCACCGGCTGAGAGAAAATCATTAATGGTAATAC
CACACTGTGCTGAGTACTACTATTGTATCGATATTGTACTAGTTGAGTTGATACATATG
GTACTCGAATTCAGCCCCTGACTGGTGCAAGGACCACCTGCCCTATGTCGTTTGACTGG
GTCATGGGGATAATTTAACAAACTCAGCTCTAGAAACTTTTAGAAAATGGGTTATGGA
TTGCTCCAAGCAGTGGTGTCAGTTGATTTTGATGAAATTTGTCACTGATTCGTTATGCT
GAATGGCTGTAGAGGCATAGAGGGCTGCGTAATCAAAGGTACAGGGCTGCGTAATCA
GGAAGGATATGCCGAGCAATTGAAACCGCTGCGCTAACTGCTGAAACGTTGTCTTATG
TATGCCATACATGTGCTAATGGTCACGGTCTGGTGTAATGTGCTACTGCACTGGGATGT
AATTACTATTACACAGTGCCATATTCCGGCTGCCCATACTTGATAAGGAAACAAGTTTT
TTCATATGTTATGGGGAAAAATCAAATGCTTGATACATGAATACCAATGTTCTGCAAGT
TTTATCTTTATTGTTAATTATTGCTGCTGATTCTTGTGAGATTTTACACGAACAGTGAT
ATCTAGTTCTTGGGTTACACTTGGTGGTGGGTGGTAGGTGTTTTAATGTAAGTCAAGTG
GCAAGAAAAGGGCAAACACAGTGCCGGAGGCTCCCACATGAGTGGGGTCTGCGGAAG
GGATAAACCGAGGCAATCCTGTAGGGTCTAAGAAAAGAGTAAAACTGAGTCAAACCTT
CCCCCATAAATACAGAGAGGTTGTTTCAAACTCGGACTGGGTGACTCATGAGACAGCT
CTCACCACTACACTAGGCCTGCCCTTCAATGTAAGTCAAGTGGCACCTTCTGCATATTA
TGCCCATCTCTGTTCTGATTTTGTGCTGTTTGGCTAGTCCTCTGTTTGTCGATGACACCC
TACCTATATGATTTGTTTAACAGCAAGGTGGTGATGCTAACTTTTACCATAGTCTGTTA
ATATGCTGCTGTCCATTCCTTTCTGTTCACTGGTTTCTTGTTGCTTCACATATTGCTGCCT
ACCTGCATGGCTGCATTGCTGATTGAGGTTGCTCTTACAGGTGTTCCTTGGACTGAAGA
AGAACACCGGAGGTTTTTGCTGGGATTACAAAAGCTTGGGAAAGGTGATTGGCGAGGG
ATTTCTCGTAATTTCGTGGTCTCAAGAACACCTACTCAAGTAGCAAGTCATGCTCAAAA
ATATTTTATACGTCAATCAAATATGAGCAGAAGGAAGAGAAGGTCTAGCCTTTTTGAC
ATGGTGCCTGATGAGGTAACTTGCTTGATTTACCTTGATTGCTTGCTTTTGCCTTGTTTT
GTACTCATACATGTGTTGTAACTACACTATTGCTTTTTTTGTAATAAATATCATATTACT
TATACTTGTGCAACATTTTGGATCTAGTTCAGTCGCTTGTATGTGTTACTGACACTCATT
GTTCGCTGTTTAAACATTGTGTAAATGGTAAAAGGCCAGCTGTGTCATTTAGGCCTTAA
ACGTTCCATCAAATGGTTGGACTGTTTAAACTGTACGGTCTAAATGGCTTAAACATGAT
 
TAAATGGGTTAAATTGCTGTTTAACTCACTGTTATTCAACAGGCAGATTACAACTATC
GCATTGGCACCATACTAATTAACATTGTAAGAGTGGAATTTAGTTCCTTTCCTCAATTT
TTTTTGCTAGGATACATATTTTTACATATGTAAATGTGTATACTTTCTGGTATATGTAT
GTTTGACTTTTGCATACATAAAATATGTATATTTTAGTGGTACTATACAAAAGCGTTTA
GATCGTTTAACTCAGTTTAAACACCGTCTAAACAGTGAACACTGGGTGACCAACTGTTT
ACTGTTTAGCATTTAGATAACACTGGTGACGGTTATGCGCAGTGTCCTATCCTTGATCT
CATTAGGTAGTTCTTACGGCCTGTTTGGTAGAGCTTTATTTGTTGATTCTTAATTCTAGC
AATTTTGATGGGGAAGTAGGTCAGAAGTGATTTAGAGTGATTCTGGTGGGGATTAAAT
GAAGTGGTGGAGAGGAATTTTCTGTAGCCATTTGGCTGTCCAGGAGTGATTCTCACAA
GAATCTGGAGCTGGGAGCAATTACTAGTGCTTTGCATATAATTTGAACTGAGCTGTGCT
CTGTTTGTTTTACAGTCCATGGACCTTCCACCCCTTCCTGGAAGTCAAGAGCCAGAGAC
CTCAGTGTTAAATCAACCACCACTGCCTCCCCCTGTGGAGGAGGAGGAGGTGGAA
TCGATGGAGTCAGATACTTCTGCTGTTGCGGAGAGTTCTGCAGCTTCAGCTCTTATGCC
CGAGAGTTTACAGCCTACCTATCCGATGATTGTTCCAGCTTATTTCTCACCGTTCTTGCA
ATTCTCAGTTCCTTTCTGGCCAAATCAGGAAGATGGAGGTGATCTGCCTCAAGAAACG
CACGAGATTGTCAAGCCTGTTGCAGTTCATTCCAAGAATCCAATTAATGTTGATGAACT
TGTGAGCATGTCAAAGCTAAGCATAGGGGAGCCTGGTCAGGAAACGGTGTCTACTTCT
CTGTCGTTAAATCTGCTGGTGGGTCAAAATAGGCAGTCGGCCTTCCATGCAAATCCTCA
AACGAGGGCTCAAGCTTGATCCCGCTTCCGCACAGAAACTGCATTAAGAAGGATTCCC
TCTCTTTTTTTTTCTATATGTAAGATTGACCGTGCTCTGTAGCTTGTATCATGTTGTAA
GTTATGCTATGTATGAATGTACCTGTTGTTTGTCTGGCACGCATGACATGCCACTCATG
ATAACAAACTCATAATGTTAGTGCTGATCTTTATGGTTGTAATGTTGTAAACTTTTCTG
AGAGAAAAAAACGTACTAGCCAGTTGTTTTC
SEQ ID NO. 45 DNA sequence after knockout of Zm00001d000184 (ZmMYBST1) of ST1 & R43-KO2 
TCCCTTCTTCATCTCTTCTCCTCACAAGGCTTCCTCCCACCACCTCCAGTCCTCCACCGG
ACCACCACCTCCAGTCCTCCACCGGACCACCACCTCCACCACTCCTCTCTCAGAACACG
CGCAAACCGCTCGAACCCTAGCTGGTGGCGGAGGCGGAGGTGGCTCCGGGATGACG
CGGCGGTGCTCGCACTGCAGCCACAACGGGCACAACTCGCGGACGTGCCCCAACCGCG
GGGTCAAGATCTTCGGGGTGCACCTCACCGATGGCTCGGCCATCCGCAAGAGCGCGAG
CATGGGGAACCTCTCCCTCCTCTCCGCGGATCCACCAGCGGCGGCGCTTCCCCCGCCG
ACGGGCCCGACCTCGCCGACGGCGGCGGGGGCTACGCCTCCGACGACTTCGTCAGCCG
CGATCGCAAGAAAGGTCAGCTACCGCCCGACCCCGATCTCTACCCCTCGTCCCTCGATC
 
ATCCGCCCGCTACCGTCCGATGCAGAACCCTAGATAATCGATCCTTCCAGCTCTAACCA
GTTCCTCCAAGCTTTTCCATCCCTCTTAATCTTTTAGATGCACCCACTGCTCGTTGATT
AGGTGCCTGTAAGTTTAGGAAACGGGTACGACTCCAGAGAGAACATTAATGGTGG
TACAACTACCACACTGCGAGTACGACTATTCTATGGATATTGTACTAGTTGACTCGATA
CGTATGGTACTCGAAACTCGAGTTCAACCCCTAACTGGTACAATGACCACCTATGGAG
GCCCTGTACTGTTTTAGTTAGGCCATGCGGATGCTTTAAGAAGCTCATCTATAGAAACT
TTTAGAATATGGGTTATGCATTGTTCCAATCAGCGGCATCAGTTGATTTTGATGAAATT
TGTCACGGGTTTGCGACGCTGAATAGCTGTGGAGGACTGGAGGTATAGAGAGATGCGT
AGTCAGAGGTATAGGGCTGCGTTATCAGGAAGGATCTGCCGAGCAATTGAAACTGCTG
CGCTAACTGCTGACGCGTAGTCTTATGTATGCCATACATGCGCCAATGGCCAAGGTCTG
GTGTAAATGTGCTACTGCACTGGGATGTAATTACTATTACGCAGTGCCAAGTAACGGCT
GTCCATACTTGATAAGGAGACACGTTTTTCACATGTTATGGGGAAATAAAAATAAAA
TGCTTGATAAATGACTACCAATGTTCTGCAAGTTTATCTCTATTGTTATTATTGCTGCT
GATTCTTGTGAGATTTTTATGCGAACAATGATAGCTAGATCTTGGTTTACACTTGGTG
GTGGGTGGTAGCTGTTTTAGTTTGAGTCAACTGGCACCTTCTTCAGATTATGCCCTTTTT
TAGCTCTCCTCTGATTTCGTGCTGTTTGGCTATTCTTCTATTTGTCAATGGCACCCTTCCT
ATATGATTTGTTCAACAGCAAAGTGGCGGTGCTAACTTTTACCATGCTCTGTTAATATG
CTGCTGTTGATTCATTTCAGTTCACTGTCTTCTTGTAGCTACACATATTGCTGCCTGCCT
TCATGGCTGCATTTTGGGCTTTCTATGCCATGTTGCTGATTAAGGTTGCTCTGACAGGT
GTTCCTTGGACTGAAGAAGAACACCGGAGGTTTTGCTGGGATTACAAAAGCTCGGGA
AAGGGGATTGGCGAGGAATTTCTCGTAATTTTGTGGTCTCAAGAACACCTACTCAAGT
AGCAAGTCATGCTCAAAAGTATTTTATACGCCAATCAAATATGAGCAGAAGGAAGAGA
AGGTCTAGCCTTTTCGACATGGTTCCTGATGAGGTAACTTGCTTGGTCTTAGTCTTCATT
TCTACACAAATATTGCTTGCTATGGCCTTGTTTATACCTATGCATGTGTTGTAACTACA
TTATTGCTTCTTTTTGTAGTAATATTTATCATATTACTTATACTAGTGCAACTTTTTGGAT
CTAGTTCTGCAGCTTGTATGTTTTCCTGGCAGTCACTGTTTGCTGTTTAAACAGAGTGTA
AATGGTAAAAGGCTAACTGTGCCATTTAGGCCGTAACGTTCCATTAAATGGTTGGACC
GTTGAAACCATAAAAAATTGTCTGAACAGCTTAAACAGAATAAAGTTAAACATGAGTT
AATGGGCTAAATTGCCGTTTAACTCACCGTTTAGTCAATAGGTAGATTATAACAATCAC
AGTGGCACCAAATTAATTGACATTGTAATTTAACTCACCGTTTAGTTAATAGGAGGATT
ATAACTATCACATTGTCCTGTTTAGTAGAGTTCGACTTCTTGATTCGTTTAGAGTGATTT
AGAAGTGATTTTGTTGGGGATAAAACGCAGATCGGTCAGGGTGGGATTTTTTAGCCGT
TTGGCTGTCTAGGAGCGATTCTCACAGAGAATATGGAGCTGTGAGCGCTTCCTTGTGGC
TTGTGCTTTGCATATAATTTGTGAACTGTGTTCTGTTTGTTTTACAGTCCATGGACCTTC
 
CGCCCCTTCCTGGAAGTCAAGAACCAGAGACCTCAATGTTAAATCAACCGCCACTGCC
TCCTGCTGTGGAGGAGGAGGTGGAATCGATGGAGTCAGATACTTCTGCTGTCGCAGAG
AGTTCTGGAGCTTCTGCTCTCATGCCCGAGAGTTTACAGCCTACCTATCCGATGATTGT
TCCAGCTTATTTCTCGCCGTTCTTGCAATTCTCAGTTCCTTTCTGGCCAAATCAGGAAGA
TGGAGGCGATCTTCCCCAAGAAACACACGAGATTGTCAAGCCTGTTGCAGTTCATTCCC
AGAATCCAATTAATGTTGATGAACTCGTGGGCATGTCAAAGCTAAGCATATGGGAGCA
TGGTCAGGAGACAGTGTCTACTTCTCTGTCGCTAAATCTGCTAGGGGGTCAAAATAGG
CAGTCGGCTTTCCATGCAAACCCTCAAACAAGAGCTCAAGCCTGATCATCCCCTACCA
GCACAGAAACTGCATTTTAGATGGAATCCTGGGCAAAATAAGCTTTGCTCTTTTTTCTT
CTTCTAAATTTAAGATCACTGAGTCATAAGGTCACTGGTTCGAAGTAGCTTCTCCGTAT
TTGCGGGGAAAGCCTGCCCTGGTTTCTCTCCTTCATCAGACCCCACTCATATGGGAGCC
TTCAATATTGTCTTTTTTATATATTTAAGATTGACCGTGCTCGGTAGCTTGTATCATGTG
CTGTAAGTTATGCTATGTATGAATGTACCTGTAGTTTGTATGGCACGCATGGTATGCCA
CTCTTATGTTAGCAAAGTCATTAATGTTAGTGCTGATCTTTATGGTTGTAATTTTATAAA
CTTCTAAAAAAAAGTATAAGCCAGTTCTTTTCAACTG
SEQ ID NO. 46 DNA sequence after knockout of Zm00001d029875 (ZmMYBR43) of ST1 & R43-KO2 
GCACCGGCTCCGGTCCTCCTCCCATCACCATCACCACCGTCGTTCCTCTCACGAGTCCC
GACACACGCAAACCGCACGAACCCTGGCTGGCGGGATGACGCGGCGGTGCTCGC
ACTGCAGCCACAACGGGCACAACTCGCGGACGTGCCCCAACCGCGGGTCAAGATCTT
CGGGGTGCGCCTCACCGATGGCTCGGCCATCCGCAAGAGCGCAAGCATGGGGAACCTC
TCCCTCCTCTCCGCGGGGTCAACCAGCGGCGGCGTCGCCCGCCGACGGCCCGACC
TCGCCGACGGCGGCGGGGGCTACGCCTCCGACGACTTCGTCACAGGGGTCGTCCTCCG
CCAGCCGCGAGCGTAAGAAAGGTCAGTTACCGCCCGATCTCTGCCCCCTCCTCTCGAAT
CGATCATCCACCCGCTACCACTCGATGCAGAACCCTAGATAATAGATCCTTCCAGCTCT
AACCAGCTCCAAGTTTTTCCATTCATTTCTCTCCTGCCTACTCGGCTTACTCTTTTTAGA
TGCACCTAGTGGCTAGTGCTCACTGATTAGGTACCTGTAAGGTTGGGAAACGGGTACT
GCACCGGCTGAGAGAAAATCATTAATGGTAATACCACACTGTGCTGAGTACTACTATT
GTATCGATATTGTACTAGTTGAGTTGATACATATGGTACTCGAATTCAGCCCCTGACTG
GTGCAAGGACCACCTGCCCTATGTCGTTTGACTGGGTCATGGGGATAATTAACAAACT
CAGCTCTAGAAACTTTTAGAAAATGGGTTATGGATTGCTCCAAGCAGTGGTGTCAGTTG
ATTTTGATGAAATTTGTCACTGATTCGTTATGCTGAATGGCTGTAGAGGCATAGAGGGC
TGCGTAATCAAAGGTACAGGGCTGCGTAATCAGGAAGGATATGCCGAGCAATTGAAAC
 
CGCTGCGCTAACTGCTGAAACGTTGTCTTATGTATGCCATACATGTGCTAATGGTCACG
GTCTGGTGTAATGTGCTACTGCACTGGGATGTAATTACTATTACACAGTGCCATATTCC
GGCTGCCCATACTTGATAAGGAAACAAGTTTTTCATATGTTATGGGAAAAATCAAA
TGCTTGATACATGAATACCAATGTTCTGCAAGTTTATCTTTATTGTTAATTATTGCTGC
TGATTCTTGTGAGATTTTACACGAACAGTGATATCTAGTTCTTGGGTTACACTTGGTG
GTGGGTGGTAGGTGTTTTAATGTAAGTCAAGTGGCAAGAAAAGGGCAAACACAGTGCC
GGAGGCTCCCACATGAGTGGGGTCTGCGGAAGGATAAACCGAGGCAATCCTGTAGG
GTCTAAGAAAAGAGTAAAACTGAGTCAAACCTTCCCCCATAAATACAGAGAGGTTGTT
TCAAACTCGGACTGGGTGACTCATGAGACAGCTCTCACCACTACACTAGGCCTGCCCTT
CAATGTAAGTCAAGTGGCACCTTCTGCATATTATGCCCATCTCTGTTCTGATTTTGTGCT
GTTTGGCTAGTCCTCTGTTTGTCGATGACACCCTACCTATATGATTTGTTTAACAGCAA
GGTGGTGATGCTAACTTTTACCATAGTCTGTTAATATGCTGCTGTCCATTCCTTTCTGTT
CACTGGTTTCTTGTTGCTTCACATATTGCTGCCTACCTGCATGGCTGCATTGCTGATTGA
GGTTGCTCTTACAGGTGTTCCTTGGACTGAAGAAGAACACCGGAGGTTTTGCTGGGAT
TACAAAAGCTTGGGAAAGGTGATTGGCGAGGGATTTCTCGTAATTTCGTGGTCTCAAG
AACACCTACTCAAGTAGCAAGTCATGCTCAAAAATATTTTATACGTCAATCAAATATG
AGCAGAAGGAAGAGAAGGTCTAGCCTTTTTGACATGGTGCCTGATGAGGTAACTTGCT
TGATTTACCTTGATTGCTTGCTTTTGCCTTGTTTTGTACTCATACATGTGTTGTAACTAC
ACTATTGCTTTTTTTGTAATAAATATCATATTACTTATACTTGTGCAACATTTTGGATCT
AGTTCAGTCGCTTGTATGTGTTACTGACACTCATTGTTCGCTGTTTAAACATTGTGTAAA
TGGTAAAAGGCCAGCTGTGTCATTTAGGCCTTAAACGTTCCATCAAATGGTTGGACTGT
TTAAACTGTACGGTCTAAATGGCTTAAACATGATTAAATGGGTTAAATTGCTGTTTAAC
TCACTGTTTATTCAACAGGCAGATTACAACTATCGCATTGGCACCATACTAATTAACAT
TGTAAGAGTGGAATTTAGTTCCTTTCCTCAATTTTTTTGCTAGGATACATATTTTTTAC
ATATGTAAATGTGTATACTTTCTGGTATATGTATGTTTGACTTTTGCATACATAAAATAT
GTATATTTTAGTGGTACTATACAAAAGCGTTTAGATCGTTTAACTCAGTTTAAACACCG
TCTAAACAGTGAACACTGGGTGACCAACTGTTTACTGTTTAGCATTTAGATAACACTGG
TGACGGTTATGCGCAGTGTCCTATCCTTGATCTCATTAGGTAGTTCTTACGGCCTGTTTG
GTAGAGCTTTATTTGTTGATTCTTAATTCTAGCAATTTTGATGGGGAAGTAGGTCAGAA
GTGATTTAGAGTGATTCTGGTGGGGATTAAATGAAGTGGTGGAGAGGAATTTTCTGTA
GCCATTTGGCTGTCCAGGAGTGATTCTCACAAGAATCTGGAGCTGGGAGCAATTACTA
GTGCTTTGCATATAATTTGAACTGAGCTGTGCTCTGTTTGTTTTACAGTCCATGGACCTT
CCACCCCTTCCTGGAAGTCAAGAGCCAGAGACCTCAGTGTTAAATCAACCACCACTGC
CTCCCCCTGTGGAGGAGGAGGAGGAGGTGGAATCGATGGAGTCAGATACTTCTGCTGT
 
TGCGGAGAGTTCTGCAGCTTCAGCTCTTATGCCCGAGAGTTTACAGCCTACCTATCCGA
TGATTGTTCCAGCTTATTTCTCACCGTTCTTGCAATTCTCAGTTCCTTTCTGGCCAAATC
AGGAAGATGGAGGTGATCTGCCTCAAGAAACGCACGAGATTGTCAAGCCTGTTGCAGT
TCATTCCAAGAATCCAATTAATGTTGATGAACTTGTGAGCATGTCAAAGCTAAGCATA
GGGGAGCCTGGTCAGGAAACGGTGTCTACTTCTCTGTCGTTAAATCTGCTGGTGGGTCA
AAATAGGCAGTCGGCCTTCCATGCAAATCCTCAAACGAGGGCTCAAGCTTGATCCCGC
TTCCGCACAGAAACTGCATTAAGAAGGATTCCCTCTCTTTTTTTTTCTATATGTAAGAT
TGACCGTGCTCTGTAGCTTGTATCATGTTGTAAGTTATGCTATGTATGAATGTACCTGTT
GTTTGTCTGGCACGCATGACATGCCACTCATGATAACAAACTCATAATGTTAGTGCTGA
TCTTTATGGTTGTAATGTTGTAAACTTTTCTGAGAGAAAAAAACGTACTAGCCAGTTGT
TTTC
SEQ ID NO. 47 DNA sequence after knockout of Zm00001d000184 (ZmMYBST1) of ST1 & R43-KO3 
TCCCTTCTTCATCTCTTCTCCTCACAAGGCTTCCTCCCACCACCTCCAGTCCTCCACCGG
ACCACCACCTCCAGTCCTCCACCGGACCACCACCTCCACCACTCCTCTCTCAGAACACG
CGCAAACCGCTCGAACCCTAGCTGGTGGCGGAGGCGGAGGTGGCTCCGGGATGACG
CGGCGGTGCTCGCACTGCAGCCACAACGGGCACAACTCGCGGACGTGCCCCAACCGCG
GGGTCAAGATCTTCGGGGTGCACCTCACCGATGGCTCGGCCATCCGCAAGAGCGCGAG
CATGGGGAACCTCTCCCTCCTCTACAGGGGTCGTCCTCCGCCAGCCGCGATCGCAAGA
AAGGTCAGCTACCGCCCGACCCCGATCTCTACCCCTCGTCCCTCGATCATCCGCCCGCT
ACCGTCCGATGCAGAACCCTAGATAATCGATCCTTCCAGCTCTAACCAGTTCCTCCAAG
CTTTTCCATCCCTCTTAATCTTTTTAGATGCACCCACTGCTCGTTGATTAGGTGCCTGTA
AGTTTAGGAAACGGGTACGACTCCAGAGAGAACATTAATGGTGGTACAACTACCA
CACTGCGAGTACGACTATTCTATGGATATTGTACTAGTTGACTCGATACGTATGGTACT
CGAAACTCGAGTTCAACCCCTAACTGGTACAATGACCACCTATGGAGGCCCTGTACTG
TTTTAGTTAGGCCATGCGGATGCTTTAAGAAGCTCATCTATAGAAACTTTTAGAATATG
GGTTATGCATTGTTCCAATCAGCGGCATCAGTTGATTTTGATGAAATTTGTCACGGGTT
TGCGACGCTGAATAGCTGTGGAGGACTGGAGGTATAGAGAGATGCGTAGTCAGAGGT
ATAGGGCTGCGTTATCAGGAAGGATCTGCCGAGCAATTGAAACTGCTGCGCTAACTGC
TGACGCGTAGTCTTATGTATGCCATACATGCGCCAATGGCCAAGGTCTGGTGTAAATGT
GCTACTGCACTGGGATGTAATTACTATTACGCAGTGCCAAGTAACGGCTGTCCATACTT
GATAAGGAGACACGTTTTTCACATGTTATGGGGAAATAAAAATAAAATGCTTGATAA
ATGACTACCAATGTTCTGCAAGTTTATCTCTATTGTTATTATTGCTGCTGATTCTTGTG
 
AGATTTTTATGCGAACAATGATAGCTAGATCTTGGTTTACACTTGGTGGTGGGTGGTA
GCTGTTTTAGTTTGAGTCAACTGGCACCTTCTTCAGATTATGCCCTTTTTTAGCTCTCCT
CTGATTTCGTGCTGTTTGGCTATTCTTCTATTTGTCAATGGCACCCTTCCTATATGATTT
GTTCAACAGCAAAGTGGCGGTGCTAACTTTTACCATGCTCTGTTAATATGCTGCTGTTG
ATTCATTTCAGTTCACTGTCTTCTTGTAGCTACACATATTGCTGCCTGCCTTCATGGCTG
CATTTTGGGCTTTCTATGCCATGTTGCTGATTAAGGTTGCTCTGACAGGTGTTCCTTGGA
CTGAAGAAGAACACCGGAGGTTTTTGCTGGGATTACAAAAGCTCGGAAAGGGGATTG
GCGAGGAATTTCTCGTAATTTTGTGGTCTCAAGAACACCTACTCAAGTAGCAAGTCATG
CTCAAAAGTATTTTATACGCCAATCAAATATGAGCAGAAGGAAGAGAAGGTCTAGCCT
TTTCGACATGGTTCCTGATGAGGTAACTTGCTTGGTCTTAGTCTTCATTTCTACACAAAT
ATTGCTTGCTATGGCCTTGTTTTATACCTATGCATGTGTTGTAACTACATTATTGCTTCT
TTTTGTAGTAATATTTATCATATTACTTATACTAGTGCAACTTTTTGGATCTAGTTCTGC
AGCTTGTATGTTTTCCTGGCAGTCACTGTTTGCTGTTTAAACAGAGTGTAAATGGTAAA
AGGCTAACTGTGCCATTTAGGCCGTAACGTTCCATTAAATGGTTGGACCGTTGAAACCA
TAAAAAATTGTCTGAACAGCTTAAACAGAATAAAGTTAAACATGAGTTAATGGGCTAA
ATTGCCGTTTAACTCACCGTTTAGTCAATAGGTAGATTATAACAATCACAGTGGCACCA
AATTAATTGACATTGTAATTTAACTCACCGTTTAGTTAATAGGAGGATTATAACTATCA
CATTGTCCTGTTTAGTAGAGTTCGACTTCTTGATTCGTTTAGAGTGATTTAGAAGTGATT
TTGTTGGGGATAAAACGCAGATCGGTCAGGGTGGGATTTTTTAGCCGTTTGGCTGTCTA
GGAGCGATTCTCACAGAGAATATGGAGCTGTGAGCGCTTCCTTGTGGCTTGTGCTTTGC
ATATAATTTGTGAACTGTGTTCTGTTTGTTTTACAGTCCATGGACCTTCCGCCCCTTCCT
GGAAGTCAAGAACCAGAGACCTCAATGTTAAATCAACCGCCACTGCCTCCTGCTGTGG
AGGAGGAGGTGGAATCGATGGAGTCAGATACTTCTGCTGTCGCAGAGAGTTCTGGAGC
TTCTGCTCTCATGCCCGAGAGTTTACAGCCTACCTATCCGATGATTGTTCCAGCTTATTT
CTCGCCGTTCTTGCAATTCTCAGTTCCTTTCTGGCCAAATCAGGAAGATGGAGGCGATC
TTCCCCAAGAAACACACGAGATTGTCAAGCCTGTTGCAGTTCATTCCCAGAATCCAATT
AATGTTGATGAACTCGTGGGCATGTCAAAGCTAAGCATATGGGAGCATGGTCAGGAGA
CAGTGTCTACTTCTCTGTCGCTAAATCTGCTAGGGGGTCAAAATAGGCAGTCGGCTTTC
CATGCAAACCCTCAAACAAGAGCTCAAGCCTGATCATCCCCTACCAGCACAGAAACTG
CATTTTAGATGGAATCCTGGGCAAAATAAGCTTTGCTCTTTTTTCTTCTTCTAAATTTAA
GATCACTGAGTCATAAGGTCACTGGTTCGAAGTAGCTTCTCCGTATTTGCGGGGAAAG
CCTGCCCTGGTTTCTCTCCTTCATCAGACCCCACTCATATGGGAGCCTTCAATATTGTCT
TTTTTATATATTTAAGATTGACCGTGCTCGGTAGCTTGTATCATGTGCTGTAAGTTATGC
TATGTATGAATGTACCTGTAGTTTGTATGGCACGCATGGTATGCCACTCTTATGTTAGC
 
AAAGTCATTAATGTTAGTGCTGATCTTTATGGTTGTAATTTTATAAACTTCTAAAAAAA
AGTATAAGCCAGTTCTTTTCAACTG
SEQ ID NO. 48 DNA sequence after knockout of Zm00001d029875 (ZmMYBR43) of ST1 & R43-KO3

GCACCGGCTCCGGTCCTCCTCCCATCACCATCACCACCGTCGTTCCTCTCACGAGTCCC
GACACACGCAAACCGCACGAACCCTGGCTGGCGGCGGATGACGCGGCGGTGCTCGC
ACTGCAGCCACAACGGGCACAACTCGCGGACGTGCCCCAACCGCGGGGTCAAGATCTT
CGGGGTGCACCTCACCGATGGCTCGGCCATCCGCAAGAGCGCGAGCATGGGGAACCTC
TCCCTCCTCTACAGGGGTCGTCCTCCGCCAGCCGCGATCGCAAGAAAGGTCAGCTACC
GCCCGACCCCGATCTCTACCCCTCGTCCCTCGATCATCCGCCCGCTACCACTCGATGCA
GAACCCTAGATAATAGATCCTTCCAGCTCTAACCAGCTCCAAGTTTTCCATTCATTTCT
CTCCTGCCTACTCGGCTTACTCTTTTAGATGCACCTAGTGGCTAGTGCTCACTGATTAG
GTACCTGTAAGGTTGGGAAACGGGTACTGCACCGGCTGAGAGAAAATCATTAATGGTA
ATACCACACTGTGCTGAGTACTACTATTGTATCGATATTGTACTAGTTGAGTTGATACA
TATGGTACTCGAATTCAGCCCCTGACTGGTGCAAGGACCACCTGCCCTATGTCGTTTGA
CTGGGTCATGGGGATAATTTAACAAACTCAGCTCTAGAAACTTTAGAAAATGGGTTA
TGGATTGCTCCAAGCAGTGGTGTCAGTTGATTTTGATGAAATTTGTCACTGATTCGTTA
TGCTGAATGGCTGTAGAGGCATAGAGGGCTGCGTAATCAAAGGTACAGGGCTGCGTAA
TCAGGAAGGATATGCCGAGCAATTGAAACCGCTGCGCTAACTGCTGAAACGTTGTCTT
ATGTATGCCATACATGTGCTAATGGTCACGGTCTGGTGTAATGTGCTACTGCACTGGGA
TGTAATTACTATTACACAGTGCCATATTCCGGCTGCCCATACTTGATAAGGAAACAAGT
TTTTTCATATGTTATGGGGAAAAATCAAATGCTTGATACATGAATACCAATGTTCTGCA
AGTTTTATCTTTATTGTTAATTATTGCTGCTGATTCTTGTGAGATTTTACACGAACAGT
GATATCTAGTTCTTGGGTTACACTTGGTGGTGGGTGGTAGGTGTTTTAATGTAAGTCAA
GTGGCAAGAAAAGGGCAAACACAGTGCCGGAGGCTCCCACATGAGTGGGGTCTGCGG
AAGGGATAAACCGAGGCAATCCTGTAGGGTCTAAGAAAAGAGTAAAACTGAGTCAAA
CCTTCCCCCATAAATACAGAGAGGTTGTTTCAAACTCGGACTGGGTGACTCATGAGAC
AGCTCTCACCACTACACTAGGCCTGCCCTTCAATGTAAGTCAAGTGGCACCTTCTGCAT
ATTATGCCCATCTCTGTTCTGATTTTGTGCTGTTTGGCTAGTCCTCTGTTTGTCGATGAC
ACCCTACCTATATGATTTGTTTAACAGCAAGGTGGTGATGCTAACTTTTACCATAGTCT
GTTAATATGCTGCTGTCCATTCCTTTCTGTTCACTGGTTTCTTGTTGCTTCACATATTGCT
GCCTACCTGCATGGCTGCATTGCTGATTGAGGTTGCTCTTACAGGTGTTCCTTGGACTG
AAGAAGAACACCGGAGGTTTTTGCTGGGATTACAAAAGCTTGGGAAAGGTGATTGGCG

AGGGATTTCTCGTAATTTCGTGGTCTCAAGAACACCTACTCAAGTAGCAAGTCATGCTC
AAAAATATTTATACGTCAATCAAATATGAGCAGAAGGAAGAGAAGGTCTAGCCTTTT
TGACATGGTGCCTGATGAGGTAACTTGCTTGATTTACCTTGATTGCTTGCTTTTGCCTTG
TTTTGTACTCATACATGTGTTGTAACTACACTATTGCTTTTTTGTAATAAATATCATAT
TACTTATACTTGTGCAACATTTTGGATCTAGTTCAGTCGCTTGTATGTGTTACTGACACT
CATTGTTCGCTGTTTAAACATTGTGTAAATGGTAAAAGGCCAGCTGTGTCATTTAGGCC
TTAAACGTTCCATCAAATGGTTGGACTGTTTAAACTGTACGGTCTAAATGGCTTAAACA
TGATTAAATGGGTTAAATTGCTGTTTAACTCACTGTTTATTCAACAGGCAGATTACAAC
TATCGCATTGGCACCATACTAATTAACATTGTAAGAGTGGAATTTAGTTCCTTTCCTCA
ATTTTTTTTGCTAGGATACATATTTTTACATATGTAAATGTGTATACTTTCTGGTATAT
GTATGTTTGACTTTTGCATACATAAAATATGTATATTTTAGTGGTACTATACAAAAGCG
TTTAGATCGTTTAACTCAGTTTAAACACCGTCTAAACAGTGAACACTGGGTGACCAACT
GTTTACTGTTTAGCATTTAGATAACACTGGTGACGGTTATGCGCAGTGTCCTATCCTTG
ATCTCATTAGGTAGTTCTTACGGCCTGTTTGGTAGAGCTTTATTTGTTGATTCTTAATTC
TAGCAATTTTGATGGGGAAGTAGGTCAGAAGTGATTTAGAGTGATTCTGGTGGGGATT
AAATGAAGTGGTGGAGAGGAATTTTCTGTAGCCATTTGGCTGTCCAGGAGTGATTCTC
ACAAGAATCTGGAGCTGGGAGCAATTACTAGTGCTTTGCATATAATTTGAACTGAGCT
GTGCTCTGTTTGTTTTACAGTCCATGGACCTTCCACCCCTTCCTGGAAGTCAAGAGCCA
GAGACCTCAGTGTTAAATCAACCACCACTGCCTCCCCCTGTGGAGGAGGAGGAGGAGG
TGGAATCGATGGAGTCAGATACTTCTGCTGTTGCGGAGAGTTCTGCAGCTTCAGCTCTT
ATGCCCGAGAGTTTACAGCCTACCTATCCGATGATTGTTCCAGCTTATTTCTCACCGTTC
TTGCAATTCTCAGTTCCTTTCTGGCCAAATCAGGAAGATGGAGGTGATCTGCCTCAAGA
AACGCACGAGATTGTCAAGCCTGTTGCAGTTCATTCCAAGAATCCAATTAATGTTGATG
AACTTGTGAGCATGTCAAAGCTAAGCATAGGGGAGCCTGGTCAGGAAACGGTGTCTAC
TTCTCTGTCGTTAAATCTGCTGGTGGGTCAAAATAGGCAGTCGGCCTTCCATGCAAATC
CTCAAACGAGGGCTCAAGCTTGATCCCGCTTCCGCACAGAAACTGCATTAAGAAGGAT
TCCCTCTCTTTTTTTTTCTATATGTAAGATTGACCGTGCTCTGTAGCTTGTATCATGTTG
TAAGTTATGCTATGTATGAATGTACCTGTTGTTTGTCTGGCACGCATGACATGCCACTC
ATGATAACAAACTCATAATGTTAGTGCTGATCTTTATGGTTGTAATGTTGTAAACTTTT
CTGAGAGAAAAAAACGTACTAGCCAGTTGTTTTC

### Example 4

SEQ ID NO. 49 DNA sequence of NIL^{DAN340} gctacccgttggtcatcattcctaccccactactgtctatggttcaattataagtgaataaaaaaagctataatcttatcaatccccgtacattaatcac 
aatcgaagaatcctacagaaaatgcacaagcttcctggtcaggtcaatcacgtacaactccacagcggaaggccgcaaagacttggtctgagg
aggaggagtcacccggggagaaggcgcggcagcgcaaaaggttgaatcatagatctgctcgagccccttatcatgcacggacacgtgcagac
aggatagccgcagtacgcacatgcatgggatggggagctggagatcatgtcatgctgctgcctgcgtggtgcatgcattgttcatcagcctgcg
cagggcatgtgacgcagccatctcgatcggaagcgtcgtgcgccgacaccacttcgtgttcgtgactgcagactctgcgccggtttccccggcc
accggggtcactcttcctgatcgaggagggccgccgtgatccatttgcagctcgatcgcagccgccttcgctcccatgccaggttcagttcacct
gggcgatcaggtaatattttttttctatgtctgaatctgcagacaataataataatttaaacaacaaccaactagtacaattcacaacggacaatctgt
aggtttttttgtatcgttaaacaacgtaaagaaagtgttgaataattagacaaattccaatctaaattccaaatataaatcatgaccaaatcagaagaa
ctgaaataacaagccaaatcagatgatgcgtactgattagacttactgatcgatggcacgcgccggaatcagcagggtcgacgatgtcgaagta
gcgaaatcagcagggtcgacgagatcagcagatcacgagcagtcgcgtgaagacgcttcccaaaaaccttattcgccctctcccggtgcagg
atctagaagacgaagggttccggagacctgctctcctgatcgcagatgcacctctgcggtcgggacgaagggaactaaaaggcggctcagct
atgaagagaggcgaaagcgaactgggatctgggatgatttggaggctggctgccgggctccttttatagggccgagaccgcgacccccgtgc
ttatccgcccacgaaaatctcgcaatcagttgagattttatagggatcggttaggataagcgtaacagccaagaaaccaaaaaatcaaacggcaa
aaggtagccgcgcccccgcaaggcgaggggccggatttcggcggaccattcacgcgcatgtcgtgcgcccgcgcccttcgccccggcccg
gcccggcccggccaggccaggcgagcgagcgcgcgcgcgcgtgtggctctccacactctctcctctcatccatgacttggtgagtgagtgtg
gcttccatatttaagctagctctactccacaagagctagcaatatggtgctattggttccacctatccctttgccatccacttatatgggcttttgagatt
ttcctggaattattagaaattcttaattgggccaagcccataaatcctaacaatcccccaccagatctcaaatgcccatctgcagttttcgccactgtt
cactactgtttaatatactagtttttcagcagagactgttaagttgaacttccgcctagaactccaagttacaccaacccacaacttggacaatggact
atgccttgaattgcaagttttgcgtgaatgggtttcacttgaagtcatgaccagtacttggctaccagtagtccccttctcgggtggagcatatacgt
cgtactccaaggtctcttcatgagtttactagagatcacccaaatctcatagactgcaacgttagacagtctaactcatataggtgtgttctttcaaag
aatgttctgcaggacaacatcttcgctaatacaagccaacagaacacattaaggcacaaagccaacctgccttacagcatttgagagtattgcatc
tttacttagagagggtcaaaagttactctcctcagttcaccaatggcttgttcttcccaggacctaattcacgggatctccgatcacatagactgggtt
tccaccatggcaactttattcgggtctcatacccatctctctcgatgcgatttctatcacattacgtggtagtcccttggtaaaaggatctgccagattt
ttatctgttgaaatataagtcacacttataactccggagtttctcaactttctgacagacttcaatcgtcttttgacatgtcttgatgactttccattatcctt
agaactcgtcactttagcaatcactgtctgattgtcacagttcataaggatagctggtattggtttctcaaccaccggcaagtccatcaagagttcac
gcaaccattctgcctcaacggttgctgtgtcaggtgcagctagctcggcttccatggttgacctcgtcaaaatggtctgcttgcatgacctccatga
taccgcacctccaccaatagtaaagacataaccactggtggcataaagctcgtctgcatcagatatccagttcgaatcactatatccttcaagtact
gcatgctgaccagaatagtgaattccataactcattgtaccttgcaggtagcgcataacccgctcaagtgcatgccaatgatcagtcccggggttt
gacatgaacctactcaatttgctcacagcaaacgagatatcgggccttgtttcaccagcaagatacatgagtgaaccgacaatctgagagtatctc
aattggtctaaaccaattctcttgttctttcgcagtgtcacactgggatcataaggagttggagaaggtttgcactcagagaagccaaatcgcttcaa
aaccttttcaacatagtgagattgcgagagagtaatcccaccatctgccttaatcagcttgatgtttagaatcacatcagcttctcccagatctttcata
tcaaaactctttgatagaaaagacttgacttcattgatcacatcaatgtttgtgccaaatatcaatatatcatcaacatataagcacaatataactccttc
gcccccaccacagcgataatatacacacctgtctgcctcattaatggcaaagcctgcagacgttagagtagtgtcaaacttctcatgccactgcttt
ggtgcttgcttcagaccatacaaagatttcaataacttgcacaccttgctttcttgaccctttactacaaatccatcaggctgttccatatagatttcctc
gtccagctctccattaagaaaagctgtctttacatccatctgatgaacaaggagaccatacgaggcagccaaagaaagtagtactcgaatagtgg
 
tcattctagcaacaggtgagtaagtatcaaagaagtcttctccttctttctgagtatagcctttagccacaagcctagccttgtacttttcaattgtacca
tcaggcttgagcttctttttaaacacccacttacaacccacgggtttgcatccatagggtcgatcagtgacttcccacgtaccatttgaaagaataga
gtccatctcattatgaactgcttctttccaatcatctgcatctggagatgcaaatgcttctgcaatggtagtaggagtatcgtccacaaggtacacaat
gaaatcattaccaaaggattttccaaccctttgtctcttgctccttttaggagcatcattgtcatcctcctctaggacaatttcatgtggctgttcaaaact
ctcaataggtgtattatgttcaggaattatctcagaagagtatctagaattgctatgaatgtctttcattggaaatatatgctcaaagaaagtagcatca
cgtgattccataatagtatcaacatacacatcaggaacttcagatttaactactaaaaatctatatgctatgctacacgaaacatatccaagaaagac
acaatccactgtccttggaccaagcttgcgctttttattaattggtacattgactttcgccatgcacccccaagtgcgcaagtatgaaagtgatggttt
tctcccaacccacttctcataaggggtttctcttctttgcccataggaattctattcagaacatgacatgaagtcaggactgcctccccccaccatgc
cttagataaaccacaagtgtctaacatggcattcaccaggtcagtcaacgtacggtttttcctttcagcaatcccgtttgactcgggtgaataggga
ggagtcctctcatgaataatgccatgttctgcacagaaatcatcaaagactttgggaaagaactcgccaccacgatctgatctaagacgtttgatct
ttctctctagttggttttcaacctcagccttatagatttaaagtagtctaaagcctcatctttagtttttagcaagtatacatagcaaaatctagacgcat
catcaatcaatgtcatgaagtatctcttaccaccttttgtcaacacaccattcatctcacaaagatcagaatgtatgagttctagtggtgccaggtgtct
ctcctcagcagccttatgaggctttcgaggttgcttcgactgcacacaactatggcacttagaacctttgactatggtgatattcggaattaaactcat
ggttgcaagccgagacatagagccaaaattaatatgacacaaacgagaatgccaaatactcgcaaggtcatcaacattagcacaaatatggttc
acagacttattattgaaatctaacaaagaaaagcggaacaagcctccgcaatcatagcctttaccaataaattgtccagacttggacacaactaatt
tattggactctaaaactaccttgaacccatctctacatagaagggttccgctaacgagattcttgtgtatagaagggacatgatgcacgttcttcagc
tgcacgatctttcccgaagtaaacttcagatccaccgtgccagtgccatgaacagaagcatgtgacccattccccattagcacggaagaatcccg
ggcgccctgataagaagagaacaagttgatgtcagaacacacatgaacattagcaccagtatcaagccaccagctaggtgattgaaatactgag
aagatgaaaggtaaattaccataccctttgtcttcctcattgctagcgaccactgtgttgacattgcccttttttgccacggcgatccgcttgatcggga
caatccttggcaaaatgacccgcctcgccacatgcgaaacatgtcaattcagccttgttcttcttcttcttgaagttggtagttttgttgggcttgttag
attttggctttcctttgcccttgttgtggttcttctgaaccatgttggcgctggagtggccctcgcctcctttagatcccgtgtccttagcccgagctttct
cctcaacatccagagacgctatcagattttcaactgatatctcctgtctcttatgtttcagagctgtggcgaagttcctccatgtagaaggcaactttg
caataatgcatccggccacaaatcggtcaggaaggactatcttaaggtggtcgagctccttggctatacactgtatttcatgagcttgctctacaat
agagcgattatcaaccatcttataatcatgaaagctctccatgatatacaggtcactgccagcatctgatgcaccatacttagtagtaagtgcatccc
acaactctttcccgtctgtgtactgcatattcgcatcaaccagacgatcaacaagggcgctaagaacggctcccgtgaacatagtattggcatggt
cgtactctttctcctgttcaggagtcagtggaccctcaggtctgcctttactaacatggaagacattcatagcagtaagccagagcgtggccttgac
ttgccatctcttaaagtgcataccattaaacttttctggcttcagcgcgtcggcaaaagcagccatagaaaaactaggaaattgtctacaataaggtt
tttggattgttgaataattagacaaattccaatctaaattccgaatataaatcatgaccaaatcagaagaactgaaataacaagccaaatcagatgat
gcgtactgattagacttactgatcgatggcacgcgccggaatcagcagggtcgacgatgtcgaagtagcgaaatcagcagggtcgacgagatc
agcagatcacgagcagtcgcgtgaagacgcttcccaaaaaccttattcgccctctcccggtgcaggatctagaagacgaagggttccggagac
ctgctctcctgatcgcagatgcacctctgcggtcgggacgaagggaactaaaaggcggctcagctatgaagagaggcgaaagcgaactggg
atctaggatgatttggaggctggctgccgggctcctttatagggccgagactgcgacccccgtgcttatccgcccacgaaaatctcgcaatcag
ttgagattttatagggatcggttaggataagcgtaacagccaagaaaccaaaaaatcaaacggcaaaaggtagccgcgcccccgcaaggcga
ggggccggatttcggcggaccattcacgcgcatgtcgtgcgcccgcgcccttcgccccggccccggccaggccaggcgagcgagcgcgcg
 
cgcgcgtgtggctctccacactctctcctctcatccatgacttggtgagtgagtgtggcttccatatttaagctagctctactccacaagagctagca
atatggtgctattggttccacctatccctttgccatccacttatatgggcttttgaaattttcctggaattattagaaattcttaattgggccaagcccata
aatcctaacagaaagtacatactgcaaatcagggcatctgaaaggtacgggaaaaaaacaaattcgcacaagcttccttccttctccttcctggt
caatcacgtaccactccctgcggctgcggccgcagcgcaagacaaaattaaacggggacgctgcgtcgagcgagcgcactgcagcttcgta
gtctatctccatcacgtcgttctggcatccctcccatctgcaccacgtttttttcttctctctccggatgattgcggcgaccagatcgatggaggggc
ggccgccgccggcaccgcgcgcgcgggaaagcgagaccacgaggccagacgacgcataaggttgaaccatagtaacatagatctgccca
agcccttatcaagttgagatgcacggacacctgcagacaggatagccgcaatacgcacatgcatgtggcctgggggacatgttgctcttatctat
cggcaagctggagctggagatgctagctgctgctgctgcgtgcatgcgttgttcttctttagccgtgtcaggcatggacctgactggaaaccaac
cgaattcctgagtttcttctctttttttccccccttcgcgtacctgcgctgcgctgcgcagggcatgtgacgcagccacatctcgaacttaacgagagc
gtcgtgcgccgacaccacttcgtgttcgtggctgtagac
SEQ ID NO. 50 DNA sequence of NIL^{K22} 
Gctacccgttggtcatcattcctaccccactactgtctatggttcaattataagtgaataaaaaagctataatcttatcaatccccgtacattaatcac
aatcgaagaatcctacagaaaatgcacaagcttcctggtcaggtcaatcacgtacaactccacagcggaaggccgcaaagacttggtctgagg
aggaggagtcacccggggagaaggcgcggcagcgcaaaaggttgaatcatagatctgctcgagcccttatcatgcacggacacgtgcagac
aggatagccgcagtacgcacatgcatgggatggggagctggagatcatgtcatgctgctgcctgcgtggtgcatgcattgttcatcagcctgcg
cagggcatgtgacgcagccatctcgatcggaagcgtcgtgcgccgacaccggttcgtgttcgtgactgcatcagacttctctgcgccggtttccg
cggccaccggggtcactcttcctgatcgaggagggccgccgtgatccatttgcagctcgatcgcagccgccttcgctcaaaccgccatgccag
gttcagttcacctgggcgaccaggtaatatatatatttttctatgtctgaatctgcagacaataataataatttgaacaacaaccaactagtacaattc
acacgggacaatctgtaggggtttttttttgtatagttaaacaacgtaaagaaagtacatactgcaaatcagggcatctgaaaggtacggaagtg
aagaatcctacagaaaaaaaaaaaacaaattcgcacaagcttccttccttcctggtcaatcacgtaccactcactccctgcggctgcggccgcag
cgcaagacaaaacggggacgctgcgttgagcgagcgcactgcagcttcgtagtctatctccatcacgttctggcatcatccctcccatctgcacc
acgtttttttttctctctctccggatgactgcggcgaccagatcgatggaggggcggccgccggcaccgcgcgcgcgggaaagcgagaccagg
agggccagacgacgcataaggttgaaccatacatagatctgcccgagcccttatcaagttgagatgcacggacacctgcagacagggatatat
atagccgcgcgcaatacgcacatgcatcatgcatgggggacaggttgctcttatgatctatcggcaagctggagatgctagctgctgctgcgtcc
atgcgttgttcttctttagccgtcaggcatggacctgactggaaccaaccgaattcctgagtttcttctctttttttcccccctcgcgtacctgcgctgcg
ctgcgcagggcatgtgacgcagccacatctcgaacttaacgagagcgtcgtgcgccgacaccagttcgtgttcgtggctgtagac
SEQ ID NO. 51 Target sequence for gene editing of NILK22
   tcactcttcctgatcgagga
SEQ ID NO. 52 Target sequence for gene editing of NILK22
   gactggaaccaaccgaatt
SEQ ID NO. 53 RNA molecule recognizing the target sequence for gene editing of NILK22 
tcactcttcctgatcgaggagttttagagctagaaatagcaagttaaaataaggctagtccgttatcaacttgaaaaagtggcaccgagtcggtgct
tttttt
SEQ ID NO. 54 RNA molecule recognizing the target sequence for gene editing of NILK22 
aattcggttggtttccagtcgttttagagctagaaatagcaagttaaaataaggctagtccgttatcaacttgaaaaagtggcaccgagtcggtgctt
ttttt
SEQ ID NO. 55 Mutant gene sequence of NILK22 
gctacccgttggtcatcattcctaccccactactgtctatggttcaattataagtgaataaaaaagctataatcttatcaatccccgtacattaatcac
aatcgaagaatcctacagaaaatgcacaagcttcctggtcaggtcaatcacgtacaactccacagcggaaggccgcaaagacttggtctgagg
aggaggagtcacccggggagaaggcgcggcagcgcaaaaggttgaatcatagatctgctcgagcccttatcatgcacggacacgtgcagac
aggatagccgcagtacgcacatgcatgggatgggggagctggagatcatgtcatgctgctgcctgcgtggtgcatgcattgttcatcagcctgcg
cagggcatgtgacgcagccatctcgatcggaagcgtcgtgcgccgacaccacttcgtgttcgtgactgcagactctgcgccggtttccccggcc
accggggtcactcttcctgatcgaaaccaaccgaattcctgagtttcttctctttttttccccccttcgcgtacctgcgctgcgctgcgcagggcatgt
gacgcagccacatctcgaacttaacgagagcgtcgtgcgccgacaccacttcgtgttcgtggctgtagac
SEQ ID NO. 56 Mutant gene sequence of NILK22 
Gctacccgttggtcatcattcctaccccactactgtctatggttcaattataagtgaataaaaaagctataatcttatcaatccccgtacattaatcac
aatcgaagaatcctacagaaaatgcacaagcttcctggtcaggtcaatcacgtacaactccacagcggaaggccgcaaagacttggtctgagg
aggaggagtcacccggggagaaggcgcggcagcgcaaaaggttgaatcatagatctgctcgagcccttatcatgcacggacacgtgcagac
aggatagccgcagtacgcacatgcatgggatgggggagctggagatcatgtcatgctgctgcctgcgtggtgcatgcattgttcatcagcctgcg
cagggcatgtgacgcagccatctcgatcggaagcgtcgtgcgccgacaccggttcgtgttcgtgactgcatcagacttctctgcgccggtttccg
cggccaccggggtcactcttcctgatcggaaaccaaccgaattcctgagtttcttctctttttttcccccctcgcgtacctgcgctgcgctgcgcagg
gcatgtgacgcagccacatctcgaacttaacgagagcgtcgtgcgccgacaccagttcgtgttcgtggctgtagac
SEQ ID NO. 57 Sequence corresponding to Indel234 in SEQ ID NO. 50 
GATATATATAGCCGCGCAATACGCACATGCATCATGCATGGGGACAGGTTGCTCT
TATGATCTATCGGCAAGCTGGAGATGCTAGCTGCTGCGTCCATGCGTTGTTCTTCT
TTAGCCGTCAGGCATGGACCTGACTGGAACCAACCGAATTCCTGAGTTTCTTCTCTTTT
TTTCCCCCTCGCGTACCTGCGCTGCGCTGCGCAGGGCATGTGACGCAGCCACATCTCGA
ACTTAACGAGAG
SEQ ID NO. 58, Indel234 replacement sequence

CATGGACCTGACTGGAAACCAACCGAATTCCTGAGTTTCTTCTCTTTTTCCCCCTTCGC
GTACGTACCTGCGCTGCGCAGGGCATGTGAGGCTACCCGCAGTGATATACGGTAAAGA
AGACGCCACCAATATAAGGCTAGGTATAGAGTAAAATTTACTGCAGTGGAGTACTCT
ATAGGTAGCTGTAAACTTATGGGGGCAAACAAAACGCTATTTACAGCGTCGATGCTT
TTTTACTCTATCAGTCCACACGTCCATGCAGCAGGTACGGTGCCGAGCGCCATCAGCCG
GTGTGGCAGTAGGGGACAAAAAAAGATATATAATATATGTAGTGATTGATATAGAGTT
GATGTATGGAGTAAATATGACTGCAGTAGATTATGATATAGGGTAGAGAATCTTGATG
ATGAGGATAGAATATTTCTTTTAGAGTAGAAATTTAGAGTAGAATGACTGCAGATAGC
CTGACGCAGCCCAT

## Claims

1. A micropeptide, wherein the micropeptide is a micropeptide having an amino acid sequence set forth in any one of SEQ ID NO. 4 or SEQ ID NO. 11-SEQ ID NO. 18 or an amino acid sequence having not less than 90% sequence identity to the sequences described above.

2. A nucleotide sequence encoding the micropeptide according to claim 1.

3. A vector or construct expressing the micropeptide according to claim 1, comprising an operably linked promoter and a nucleotide sequence encoding the micropeptide, wherein preferably, the nucleotide sequence encoding the micropeptide is SEQ ID NO. 3.

4. A method for synthesizing the micropeptide according to claim 1, wherein the method is a chemical method or a biological method; the chemical method comprises a step of liquid-phase synthesis or solid-phase synthesis, and the biological method comprises the following steps: 1) constructing a vector comprising an operably linked promoter and a nucleotide sequence encoding the micropeptide; 2) transfecting a host cell and screening for positive clones; and 3) culturing the host cell and isolating a micropeptide product.

5. A formulation, comprising the micropeptide according to claim 1 or a nucleotide encoding the micropeptide at a concentration of 1 µM-50 µM, wherein optionally, the formulation further comprises: water, a bacterial solution, a cell culture medium, a plant culture medium, normal saline, a cosmetic excipient, or a pharmaceutically acceptable excipient.

6. An anti-aging method, comprising preparing an anti-aging product, or a product associated with the treatment of an aging-related disease, or an antioxidant product by using the micropeptide according to claim 1 or a nucleotide encoding the micropeptide, wherein preferably, the product comprises a formulation, a probe, a reagent, a kit, a health care product, a cosmetic product, a drug, or a pharmaceutical combination.

7. A method for delaying aging, prolonging lifespan, or reducing generation of reactive oxygen species in an animal or human subject, comprising providing the micropeptide according to claim 1 or a nucleotide encoding the micropeptide to the animal or human subject, preferably, by means of topical, oral, or intravenous injection.

8. A method for regulating the plant kernel water content or the plant kernel dehydration rate or delaying fruit ripening, comprising overexpressing a nucleotide sequence encoding the micropeptide in a plant; or administering to a plant a reagent comprising the micropeptide according to claim 1 or a nucleotide encoding the micropeptide, wherein preferably, the reagent further comprises water, a bacterial solution, a cell culture medium, a plant culture medium, or normal saline; preferably, the administration comprises spraying *in vitro* or adding to the soil; preferably, the plant is a plant of the family *Poaceae*.

9. A method for cultivating a maize material having the trait of low kernel water content or fast dehydration rate, comprising the following steps:
1) providing a maize material to be improved; and
2) performing genetic modification or hybridization on the material to be improved in step 1), wherein
step 2) is selected from at least one of the following steps:
i) reducing the expression level of the micropeptide according to claim 1 in the material to be improved;
ii) increasing the expression levels of the ZmEIL1 gene set forth in SEQ ID NO. 19 and the ZmEIL3 gene set forth in SEQ ID NO. 20 in the material to be improved;
iii) increasing the expression levels of ZmMYBST1 and ZmMYBR43 transcription factors having the amino acid sequences set forth in SEQ ID NO. 35 and SEQ ID NO. 36, respectively, in the material to be improved, wherein preferably, the nucleotide sequences coding sequences of the transcription factors are set forth in SEQ ID NO. 33 and SEQ ID NO. 34, respectively; and
iv) selecting a maize material exhibiting the trait of low kernel water content or fast dehydration rate based on at least one of the following molecular markers and hybridizing the maize material with the material to be improved in step 1), wherein the molecular markers comprise:
Indel6181: a 6181-bp insertion between positions 722-779 based on the sequence set forth in SEQ ID NO. 50;
Indel50: a 50-bp deletion between positions 729-778 based on the sequence set forth in SEQ ID NO. 50;
Indel234: a 234-bp insertion between positions 1131-1377 based on the sequence set forth in SEQ ID NO. 50; or
an SNP mutation at position 67, or position 70, or position 473, or position 513, or position 597, or position 599, or position 605 based on the sequence set forth in SEQ ID NO. 50.

10. The method according to claim 9, wherein in step i), the coding sequence of the micropeptide is knocked out by means of gene editing, or the expression of the coding sequence of the micropeptide is reduced through RNA interference.

11. The method according to claim 9, wherein a nucleotide sequence encoding the micropeptide is SEQ ID NO. 3.

12. The method according to claim 9, wherein increasing the expression in step ii) or iii) comprises gene overexpression or high-activity promoter expression.

13. The method according to claim 9, further comprising step 3), selecting or selecting from the progeny a maize material exhibiting the trait of low kernel water content or fast dehydration rate, wherein preferably, in step 3), the selection is performed by detecting the molecular marker Indel234, Indel50, or Indel6181; preferably, the material with a fast dehydration rate is a haplotype that does not comprise Indel234, Indel50, or Indel6181.

14. A method for cultivating a maize material having the trait of high kernel water content or slow dehydration rate, comprising the following steps:
1) providing a maize material to be improved; and
2) performing genetic modification or hybridization on the material in step 1, wherein
step 2) is selected from at least one of the following steps:
i) increasing the expression level of the micropeptide according to claim 1 in the material to be improved;
ii) reducing the expression levels of the ZmEIL1 gene set forth in SEQ ID NO. 19 and the ZmEIL3 gene set forth in SEQ ID NO. 20;
iii) reducing the expression levels of ZmMYBST1 and ZmMYBR43 transcription factors having the amino acid sequences set forth in SEQ ID NO. 35 and SEQ ID NO. 36, respectively; wherein preferably, the nucleotide sequences coding sequences of the transcription factors are set forth in SEQ ID NO. 33 and SEQ ID NO. 34, respectively;
iv) deleting any fragment between positions 1049-1417 based on SEQ ID NO. 50 in the material to be improved; and
v) selecting a maize material exhibiting the trait of high kernel water content or slow dehydration rate based on at least one of the following molecular markers and hybridizing the maize material with the material to be improved in step 1), wherein the molecular markers comprise:
Indel6181: a 6181-bp insertion at positions 722-779 based on the sequence set forth in SEQ ID NO. 50;
Indel50: a 50-bp deletion at positions 729-778 based on the sequence set forth in SEQ ID NO. 50;
Indel234: a 234-bp insertion at positions 1131-1377 based on the sequence set forth in SEQ ID NO. 50; or
an SNP mutation at position 67, or position 70, or position 473, or position 513, or position 597, or position 599, or position 605 based on the sequence set forth in SEQ ID NO. 50.

15. The method according to claim 14, wherein increasing the expression in step i) comprises introducing an expression cassette, wherein the expression cassette comprises a promoter, the ZmRPG gene set forth in SEQ ID NO. 1 or 2 or a nucleotide sequence encoding the micropeptide set forth in SEQ ID NO. 3, and a terminator; preferably, the promoter is a high-activity promoter, such as a maize ubiquitin promoter.

16. The method according to claim 14, wherein reducing the expression in step ii) or iii) comprises reducing by means of gene editing or RNA interference.

17. The method according to claim 14, wherein the deletion in step iv) is performed using a gene editing method.
The method according to claim 14, further comprising step 3), selecting or selecting from the progeny a maize material exhibiting the trait of high kernel water content or slow dehydration rate, wherein preferably, the selection is performed by detecting the molecular marker; preferably, the material with a slow dehydration rate is a haplotype comprising Indel234 and Indel50, or a haplotype that does not comprise Indel234 and Indel50 but comprises Indel6181.
